# EUROPEAN PATENT SPECIFICATION

(11) **EP 4 211 228 B1**
(45) Date of publication and mention of the grant of the patent: **05.11.2025**
(21) Application number: 21787116.9
(22) Date of filing: 08.09.2021
(51) Int. Cl.: C12N 5/0783, C07K 14/705, G01N 33/50

(54) **T CELL PHENOTYPES ASSOCIATED WITH RESPONSE TO ADOPTIVE CELL THERAPY**
T-ZELL-PHÄNOTYPEN IM ZUSAMMENHANG MIT DER REAKTION AUF EINE ADOPTIVE ZELLTHERAPIE
PHÉNOTYPES DE LYMPHOCYTES T ASSOCIÉS À UNE RÉPONSE À UNE THÉRAPIE CELLULAIRE ADOPTIVE

(30) Priority: 08.09.2020 US 202063075536 P
(43) Date of publication of application: 19.07.2023
(73) Proprietor: The United States of America, as represented by the Secretary, Department of Health and Human Services, Maryland 20892-7788 (US)
(72) Inventor: LOWERY, III, Frank J., Clarksburg, Maryland 20871 (US); KRISHNA, Sri, North Bethesda, Maryland 20852 (US); ROBBINS, Paul F., Chevy Chase, Maryland 20815 (US); ROSENBERG, Steven A., Potomac, Maryland 20854 (US); ALTAN-BONNET, Gregoire Y., Bethesda, Maryland 20817 (US)
(74) Representative: Maiwald GmbH
(86) International application number: PCT/US2021/049387
(87) International publication number: WO 2022/055946

(56) References cited:
- WO-A1-2018/067991
- MATTHEW J LOZA ET AL: "T-cell specific defect in expression of the NTPDase CD39 as a biomarker for lupus", CELLULAR IMMUNOLOGY, ACADEMIC PRESS, SAN DIEGO, CA, US, vol. 271, no. 1, 10 June 2011 (2011-06-10), pages 110 - 117, XP028284095, ISSN: 0008-8749, [retrieved on 20110619], DOI: 10.1016/J.CELLIMM.2011.06.010
- FLORIAN MAIR ET AL: "A Targeted Multi-omic Analysis Approach Measures Protein Expression and Low-Abundance Transcripts on the Single-Cell Level", CELL REPORTS, vol. 31, no. 1, 1 April 2020 (2020-04-01), US, pages 107499, XP055724607, ISSN: 2211-1247, DOI: 10.1016/j.celrep.2020.03.063
- FERNANDO P. CANALE ET AL: "CD39 Expression Defines Cell Exhaustion in Tumor-Infiltrating CD8 + T Cells", CANCER RESEARCH, vol. 78, no. 1, 1 January 2018 (2018-01-01), US, pages 115 - 128, XP055662774, ISSN: 0008-5472, DOI: 10.1158/0008-5472.CAN-16-2684
- GALLERANO DANIELA ET AL: "+ T-cell functions", INTERNATIONAL JOURNAL OF CANCER, vol. 147, no. 9, 8 July 2020 (2020-07-08), US, pages 2597 - 2610, XP055856088, ISSN: 0020-7136, Retrieved from the Internet <URL:https://onlinelibrary.wiley.com/doi/full-xml/10.1002/ijc.33131> DOI: 10.1002/ijc.33131
- MITA YUKIYOSHI ET AL: "Crucial role of CD69 in anti-tumor immunity through regulating the exhaustion of tumor-infiltrating T cells", INTERNATIONAL IMMUNOLOGY, vol. 30, no. 12, 14 November 2018 (2018-11-14), GB, pages 559 - 567, XP055856092, ISSN: 0953-8178, Retrieved from the Internet <URL:https://watermark.silverchair.com/dxy050.pdf?token=AQECAHi208BE49Ooan9kkhW_Ercy7Dm3ZL_9Cf3qfKAc485ysgAAAt8wggLbBgkqhkiG9w0BBwagggLMMIICyAIBADCCAsEGCSqGSIb3DQEHATAeBglghkgBZQMEAS4wEQQMMSxPdUBQok55jUnCAgEQgIICkkLcOZgMJqKkM1YpI4cbu-26ir7gyjbroTau-zy-01OYXYB1xZeU6DScOkZRCsTmc9JqgMZoOrcA9UYAJib9N1TMZ17tg> DOI: 10.1093/intimm/dxy050
- KIMURA MOTOKO Y ET AL: "A new therapeutic target: the CD69-Myl9 system in immune responses", SEMINARS IN IMMUNOPATHOLOGY, SPRINGER BERLIN HEIDELBERG, BERLIN/HEIDELBERG, vol. 41, no. 3, 5 April 2019 (2019-04-05), pages 349 - 358, XP036782988, ISSN: 1863-2297, [retrieved on 20190405], DOI: 10.1007/S00281-019-00734-7
- KRISHNA SRI ET AL: "Stem-like CD8 T cells mediate response of adoptive cell immunotherapy against human cancer", SCIENCE (AMERICAN ASSOCIATION FOR THE ADVANCEMENT OF SCIENCE), 11 December 2020 (2020-12-11), United States, pages 1328 - 1334, XP055856073, Retrieved from the Internet <URL:https://www.science.org/doi/10.1126/science.abb9847> [retrieved on 20211028], DOI: 10.1126/science.abb9847
- CREELAN BENJAMIN C ET AL: "Tumor-infiltrating lymphocyte treatment for anti-PD-1-resistant metastatic lung cancer: a phase 1 trial", NATURE MEDICINE, NATURE PUBLISHING GROUP US, NEW YORK, vol. 27, no. 8, 1 August 2021 (2021-08-01), pages 1410 - 1418, XP037549198, ISSN: 1078-8956, [retrieved on 20210812], DOI: 10.1038/S41591-021-01462-Y
- HELD WERNER ET AL: "Not All Tumor-Infiltrating CD8+ T Cells Are Created Equal", CANCER CELL, CELL PRESS, US, vol. 39, no. 2, 8 February 2021 (2021-02-08), pages 145 - 147, XP086493273, ISSN: 1535-6108, [retrieved on 20210208], DOI: 10.1016/J.CCELL.2021.01.015

## Description

### CROSS REFERENCE TO RELATED APPLICATION

This patent application claims the benefit of U.S. Provisional Patent Application No. 63/075,536, filed September 8, 2020.

### STATEMENT REGARDING FEDERALLY SPONSORED RESEARCH OR DEVELOPMENT

This invention was made with Government support under project numbers ZIA BC 010984 and ZIA BC 011726 by the National Institutes of Health, National Cancer Institute. The Government has certain rights in the invention.

### INCORPORATION-BY-REFERENCE OF MATERIAL SUBMITTED ELECTRONICALLY

Incorporated by reference in its entirety herein is a computer-readable nucleotide/amino acid sequence listing submitted concurrently herewith and identified as follows: One 134,069 Byte ASCII (Text) file named "756715_ST25.txt," dated September 7, 2021.

### BACKGROUND OF THE INVENTION

Adoptive cell therapy (ACT) using T cells can produce positive clinical responses in some patients. Nevertheless, several obstacles to the successful use of ACT for the treatment of cancer and other conditions remain. For example, the impact of T cell phenotype on clinical success of ACT in humans has not been elucidated. Accordingly, there is a need for improved methods of preparing cell populations for ACT.

Loza et al., Cell Immunol 271(2011), 110-117 describes a T-cell specific defect in expression of the NTPDase CD39 as a biomarker for lupus. Mair et al., Cell Rep 31 (2020), 1-32 describes a targeted multi-omic analysis approach measuring protein expression and low-abundance transcripts on the single-cell level. Canale et al., Cancer Res 78(2017), 115-128 describes that CD39 expression defines cell exhaustion in tumor-infiltrating CD8+ T cells. Gallerano et al., Int J Cancer 147 (2020), 2597-2610 describes that genetically driven CD39 expression shapes human tumor-infiltrating CD8+ T-cell functions. WO 2018/067991 A1 relates to immune checkpoint targets. Mita et al., Int. Immunol. 30 (2018), 559-567 describes a crucial role of CD69 in anti-tumor immunity through regulating the exhaustion of tumor-infiltrating T cells. Kimura et al., Sem Immunopathol 41 (2019), 349-358 describes the CD69-Myl9 system as a new therapeutic target in immune responses.

### BRIEF SUMMARY OF THE INVENTION

The invention is defined in the claims. An embodiment of the invention provides a method of obtaining a cell population enriched for T cells with a phenotype comprising markers CD3+, CD8+, CD39-, and CD69-, the method comprising: (a) obtaining a bulk population of T cells from a tumor sample of a patient; (b) specifically selecting T cells with a phenotype comprising markers CD3⁺, CD8+, CD39⁻, and CD69⁻ from the bulk population; (c) separating the cells selected in (b) from cells which lack the phenotype and (d) isolating tumor-reactive T cells from the separated T cells which have the phenotype to obtain a cell population enriched for T cells with the phenotype.

Another embodiment of the invention provides a method of obtaining a cell population enriched for T cells with a phenotype comprising markers CD3+, CD8+, CD39-, and CD69-, the method comprising: (a) obtaining a bulk population of T cells from a tumor sample of a patient; (b) isolating tumor-reactive T cells from the tumor sample;(c) specifically selecting T cells with a phenotype comprising markers CD3⁺, CD8+, CD39⁻, and CD69⁻ from the isolated tumor-reactive T cells; and (d) separating the cells selected in (c) from cells which lack the phenotype to obtain a cell population enriched for T cells with the phenotype.

Further embodiments of the invention provide an isolated or purified cell population obtained according to any of the inventive methods and related pharmaceutical compositions comprising the same.

The references to the methods of treatment by therapy or surgery or in vivo diagnosis method in this description are to be interpreted as references to compounds, pharmaceutical compositions and medicaments of the present invention for use in those methods.

Another embodiment of the invention provides a method of treating or preventing a condition in a mammal, the method comprising obtaining a cell population enriched for T cells with the phenotype according to any of the inventive methods and administering the cell population or a pharmaceutical composition comprising the cell population to the mammal in an amount effective to treat or prevent the condition in the mammal, wherein the condition is cancer.

Still another embodiment of the invention provides a method of selecting a therapy for a cancer patient, the method comprising: (a) obtaining a bulk population of T cells from a tumor sample of a patient; (b) measuring the proportion of T cells in the bulk population which (i) have or (ii) lack a phenotype, wherein the phenotype comprises markers CD3⁺, CD8+, CD39⁻, and CD69⁻; and (c) comparing the proportion of T cells which (i) have or (ii) lack the phenotype to a control; and (d) selecting the patient for a therapy which is not immunotherapy when (i) the proportion of T cells which have the phenotype is lower than the control or (ii) the proportion of T cells which lack the phenotype is equal to or higher than the control; or (e) selecting the patient for an immunotherapy when (i) the proportion of T cells which have the phenotype is equal to or higher than the control or (ii) the proportion of T cells which lack the phenotype is lower than the control.

Another embodiment of the invention provides a method of treating cancer in a patient, the method comprising: receiving an identification of a therapy selected for a cancer patient, wherein the therapy has been selected by any of the inventive methods; and (I) treating the patient by administering a therapy which is not immunotherapy to the patient in an amount effective to treat cancer in the patient when (i) the proportion of T cells which have the phenotype is lower than the control or (ii) the proportion of T cells which lack the phenotype is equal to or higher than the control; or (II) treating the patient by administering an immunotherapy to the patient in an amount effective to treat cancer in the patient when (i) the proportion of T cells which have the phenotype is equal to or higher than the control or (ii) the proportion of T cells which lack the phenotype is lower than the control.

Still another embodiment of the invention provides a method for predicting the clinical response to immunotherapy in a cancer patient, the method comprising: (a) obtaining a bulk population of T cells from a tumor sample from the cancer patient; (b) measuring the proportion of T cells in the bulk population which (i) have or (ii) lack a phenotype, wherein the phenotype comprises markers CD3⁺, CD8+, CD39⁻, and CD69⁻; and (c) comparing the proportion of T cells which (i) have or (ii) lack the phenotype to a control; and (d) identifying the patient as likely to have a negative clinical response to the immunotherapy when (i) the proportion of T cells which have the phenotype is lower than the control or (ii) the proportion of T cells which lack the phenotype is equal to or higher than the control; or (e) identifying the patient as likely to have a positive clinical response to the immunotherapy when (i) the proportion of T cells which have the phenotype is equal to or higher than the control or (ii) the proportion of T cells which lack the phenotype is lower than the control.

### BRIEF DESCRIPTION OF THE SEVERAL VIEWS OF THE DRAWING(S)

Figure 1A is a schematic illustrating a melanoma cohort patient ACT infusion products (I.P.) and schema used for the study described in the Examples.
Figure 1B shows t-SNE (t-distributed stochastic neighbor embedding) plots of live CD45⁺CD3⁺ cell clusters of I.P. from all patient I.P. (left), clusters from CR I.P. only (middle), and clusters from NR I.P. only (right). Cluster 1 represents CD39⁻CD69⁻DN population.
Figure 1C is a plot showing the percentage of CR and NR I.P. cells in each cluster **P* = 0.0264 from two-sided Wilcoxon rank-sum test adjusted by Bonferroni correction for all clusters.
Figures 1D-1E are scatter plots showing the results of flow-based independent validation of CR I.P. and NR I.P. samples (N = 38) showing the percentages of CD39⁻ CD69⁻ (DN) cells of total CD8⁺(1E) and total CD3⁺(1F).
Figure 1F is a scatter plot showing the total number of infused cells within CR I.P. and NR I.P. (N = 38).
Figure 1G is a scatter plot showing the total number of CD8⁺CD39⁻CD69⁻ (DN) cells in CR I.P. and NR I.P. (N = 38). * *P* < 0.05 ***P* < 0.01 by two-sided Wilcoxon rank-sum test.
Figure 1H shows progression-free survival (PFS) of all I.P. samples (N = 54) separated by median total cell numbers infused (left) and median CD8⁺CD39⁻CD69⁻ (DN) cell numbers infused (right).
Figure 1I shows melanoma-specific survival (MSS) of all I.P. samples (N = 54) separated by median cell number infused (left) and median CD39⁻CD69⁻ cell number infused (right). "Low" indicates patients with I.P. cells less than median of the sub-group analyzed and "High" indicates patients with I.P in Figures 1H-1I. Cells greater than median of the sub-group were analyzed. P-values by the Log-rank Mantel-Cox test is shown in Figures 1H-1I.
Figure 2A is a t-SNE plot of all CD8⁺ TILs from CR and NR I.P. (5 CRs, 5 NRs).
Figure 2B is a box plot representing the percentage of S.Cluster.A (top) and S.Cluster.B (bottom) within CR and NR I.P. S.Cluster.A encompasses clusters C0, C2, C5, C6, and C7; S.Cluster.B comprises clusters C1, C3, C4, and C8. **P* < 0.05 by two-sided Wilcoxon rank-sum test are shown.
Figure 2C is a bar graph showing the results of an analysis in which each patient I.P. was scored by DN and DP gene signature scores and their mean scGSEA scores are plotted on the y-axis. *****P* < 0.0001 by two-sided Wilcoxon rank-sum test comparing the mean DN and DP signature scores between CRs and NRs.
Figure 2D shows scatter plots showing the results of a flow cytometric analysis of inhibitory and memory markers within each subset (DN [CD39⁻CD69⁻], SP [CD39⁻ CD69⁺, CD39⁺CD69⁻] and DP [CD39⁺CD69⁺] of patient I.P. in the validation set (n=38) expressed as % of each subset (parent gate). **P* < 0.05 ***P* < 0.01 ****P* < 0.001 *****P* < 0.0001 comparing DN and other subsets by two-sided Wilcoxon rank-sum test corrected by Bonferroni multiple comparisons. Flow cytometric analysis shows low expression of T-cell exhaustion markers in CD39⁻CD9⁻ subsets.
Figure 2E is a graph showing the results of a flow cytometry analysis of intracellular TCF7 expression for DN and DP subsets. Histogram shows representative patient I.P. sample (top) and box plot shows the quantitation for 18 I.P. (bottom). ****P* < 0.001 by two-sided Wilcoxon rank-sum test. Flow cytometric analysis shows higher expression of T-cell stemness factor TCF7 in CD39⁻CD69⁻ subset.
Figure 2F shows flow cytometry plots showing the phenotypes of DN, SP, and DP states before and after anti-CD3/anti- CD28 stimulation for 48 hours of a representative patient sample (left), and dot plots quantifying phenotypes of daughter cells after stimulation of FACS-sorted DN parent (circles) or FACS-sorted DP parent (squares) ***P < 0.001 by two-sided Wilcoxon rank-sum test, N = 6 I.P.
Figure 2G shows graphs. TILs from pre ICB therapy were scored by the top DN and DP gene signature scores and mean scGSEA scores are plotted on y-axis. TILs from responding lesions are represented by A, and cells from progressing lesions are represented by B. Cell numbers and *****P* < 0.0001 by two-sided Wilcoxon rank-sum test are shown.
Figure 3A shows representative CR I.P. showing detection of neoantigen-specific tetramers (top) and CD39/CD69 phenotypes of bulk CD8+ TILs and CD8+ tetramer+ TILs (bottom). Numbers within quadrants, listed under tetramer category represent percent of the sub-population within each CD8⁺tetramer⁺gate.
Figure 3B shows NR I.P. with comparable number of neoantigens (top) and CD39/CD69 phenotypes of bulk CD8⁺ TILs and CD8⁺ tetramer⁺ TILs (bottom). Numbers within quadrants, listed under tetramer category represent percent of the sub-population within each CD8⁺tetramer⁺gate.
Figure 3C shows DN, SP, and DP phenotypes within all 26 neoantigen-specific T cell populations expressed as a percent of tetramer⁺ cells of each individual tetramer from 11 patients. CRs and NRs are combined for this data analysis (CRs + NRs). *P < 0.05, *****P* < 0.0001 by two-sided Wilcoxon rank-sum test followed by Bonferroni correction for multiple comparisons.
Figure 3D is a graph showing DN, SP, and DP phenotypes of 26 neoantigen-specific TILs sub-divided by response status (CRs versus NRs). ***P* < 0.01, ****P* < 0.001 by two-sided Wilcoxon rank-sum test between CRs and NR tetramer⁺ cells foreach subset.
Figure 3E is a graph showing CD39-negative neoantigen-specific tetramer⁺ TILs (CD39⁻ as a single marker) sub- divided by response status (CRs versus NRs). *****P <* 0.0001 by two-sided Wilcoxon rank-sum test.
Figure 3F is a t-SNE plot of CD8⁺ TILs from 3713-CR I.P. showing projection of stem-like DN and differentiated DP gene signatures. Dotted lines indicate the stem-like and differentiated clusters analyzed.
Figure 3G is a graph showing Pt. 3713 NeoTCR⁺ cells from stem-like C0 cluster and differentiated C1 cluster displayed as a percent of total NeoTCR⁺ cells for each neoantigen specificity.
Figure 3H is a graph showing each NeoTCR⁺ clonotype scored by their mean fitness score (value of DN minus DP scGSEA scores). Positive values indicate clonotype enrichment in stem-like phenotypes and negative scores indicate clonotype enrichment in differentiated state.
Figure 3I is a graph showing post-ACT persistence of the immunodominant SRPXₘᵤₜNeoTCR clonotypes from Pt. 3713-CR I.P. in peripheral blood normalized to their initial frequency in the infusion product (day 0).
Figure 3J is a t-SNE plot of CD8⁺ TILs from Pt. 4000-NR I.P. showing projection of stem-like DN and differentiated DP gene signatures (left), projection of neoantigen-specific TCRs shaded by antigen specificity (right). Dotted lines indicate the stem-like and differentiated clusters analyzed.
Figure 3K is a graph showing each NeoTCR⁺ clonotype (HIVEP2ₘᵤₜ, AMPHₘᵤₜ) scored by their mean fitness scores as described for Figure 3H.
Figure 3L is a graph showing post-ACT persistence of the HIVEP2ₘᵤₜ and AMPHₘᵤₜNeoTCR clonotypes from Pt. 4000-NR in peripheral blood normalized to their frequency in the infusion product. Patient follow-up was stopped at 150 days due to disease progression.
Figure 4A is a schema for using endogenous human TCR to track NY-ESO-1 TCR-transduced (ESO.TCR⁺) I.P. clones in post-treatment peripheral blood of a complete responder to NY-ESO-1 TCR therapy (ESO.CR).
Figure 4B is a graph showing post-treatment peripheral blood persistence of top 20 ESO.TCR+ I.P. clones using endogenous human TCR according to enrichment in DN (circles) and DP (squares) phenotypes in TCR infusion product. Clones with (Frequency in DN / Frequency in DP) > 1 are defined as enriched in DN state; clones with (Frequency in DN / Frequency in DP) < 1 are defined as enriched in DP state.
Figure 4C is a violin plot comparing clones undetectable at day 7 post-ACT (Non-Persistent) with long-term persistent clones at day 1846 post-ACT by the ratio of their clonotypic frequency in the CD39⁻ CD69⁻ stem-like state to their clonotypic frequency within the CD39⁺CD69⁺ differentiated state in the I.P. A ratio > 1 represents enrichment in the DN state while a ratio < 1 represents enrichment in the DP state. ***P* < 0.01 by two-sided Wilcoxon rank-sum test.
Figure 4D is a schema of adoptive transfer of sorted DN and DP pmel-transgenic T cells into mice bearing established B 16 melanoma tumors.
Figure 4E is a graph showing tumor growth curves of mice bearing B16 tumors treated with pmel DN or DP T cells in two doses. N = 6 mice per group. **P* < 0.05 ***P <* 0.01 for tumor growth kinetics calculated by Wilcoxon rank-sum test. A represents untreated. B represents treatment with CD39⁺CD69⁺ (3e5, 5e5 dose). C represents treatment with CD39⁻CD69⁻ (3e5 dose). D represents treatment with CD39⁻CD69⁻ (5e5 dose).
Figure 4F is a graph showing survival curves of mice bearing B16 tumors treated with pmel DN or DP T cells in two doses. N = 6 mice per group. *P < 0.05 ***P* < 0.01 for tumor growth kinetics calculated by Wilcoxon rank-sum test. The dotted line represents untreated. A (squares) represents treatment with CD39⁺CD69⁺ (3e5, 5e5 dose). B represents treatment with CD39⁻CD69⁻ (3e5 dose). C represents treatment with CD39⁻CD69⁻ (5e5 dose).
Figure 4G is an illustration of the role of stem-like T cells in immunotherapy and ACT success and paradoxical nature of tumor mutation-reactive T cells in stem-like and terminally differentiated states.
Figure 5A shows manual gating of CyTOF data for t-SNE projection of CyTOF data (left: live-cell gate; right: CD45⁺DNA(2n) Singlet gate).

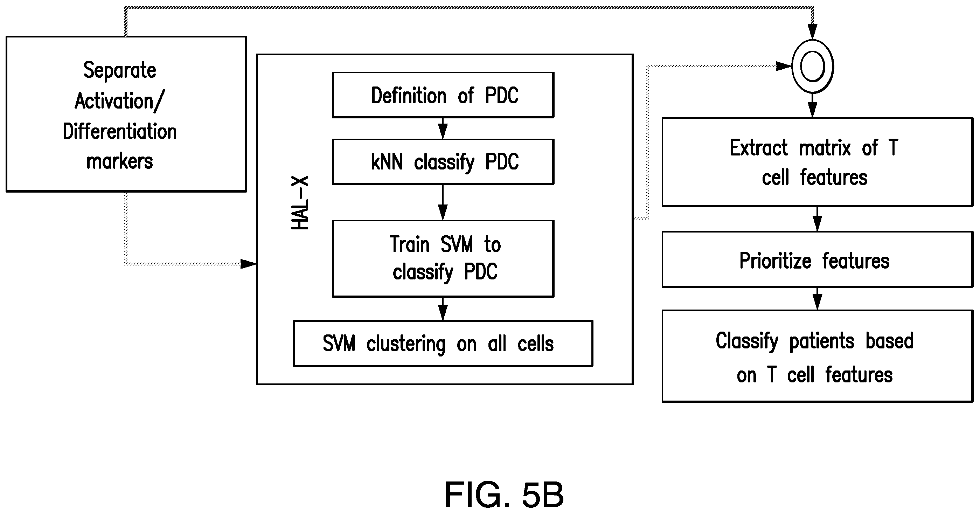

Figure 5B is a schematic illustrating a data processing pipeline for CyTOF data: activation/exhaustion markers (CTLA-4, PD-1, CD28, LAG3, OX40, CD25, FAS, 4-1BB, HLA-DR, CD39, CD69, ICOS) were distinguished from differentiation markers (e.g. CD2, CD3, CD4, CD8 etc.) to process the data. A hierarchical agglomerative learning algorithm (HAL-X) built upon a k-nearest neighbor (kNN) classification trained on expression of differentiation markers only "pure differentiation cluster" (PDC) was used to classify cells into 22 clusters. The expression levels of activation/exhaustion markers were then measured on these 22 clusters to generate a matrix of features. Features with high discriminatory power are chosen to build a classifier of patients based on their clinical response (CR: complete response; NR: progressive disease).
Figure 5C shows a Receiver Operating characteristic (ROC) for the classification of patients based on activation/exhaustion markers: Area Under the Curve (AUC) is larger than 89% for the 17 patients under analysis. Five iterations ("split") are presented here corresponding to the 80%/20% split of patients' samples into random train and test subsets; "average" represents the average ROC across the five splits; "baseline" represents the ROC for a random classification.
Figure 6A shows CD39 and CD69 expression dot plots gated on CD8+ T cells from one representative CR and NR I.P. (left panel) and quantification of 16 I.P. (right panel) in the discovery set. Top row represents CyTOF data of the two patients and bottom row represents flow cytometry analysis of the same patients. Numbers indicate p-values by two-sided Wilcoxon rank-sum test.
Figure 6B shows linear regression between flow cytometry and CyTOF analysis of 16 I.P. samples analyzed for CD39⁻ CD69⁻ subset.
Figure 6C shows expression profiles of various subsets in 38 validation I.P. compared between responders and non-responders. Numbers indicate p-values by two-sided Wilcoxon rank-sum test analyzed individually without multiple corrections.

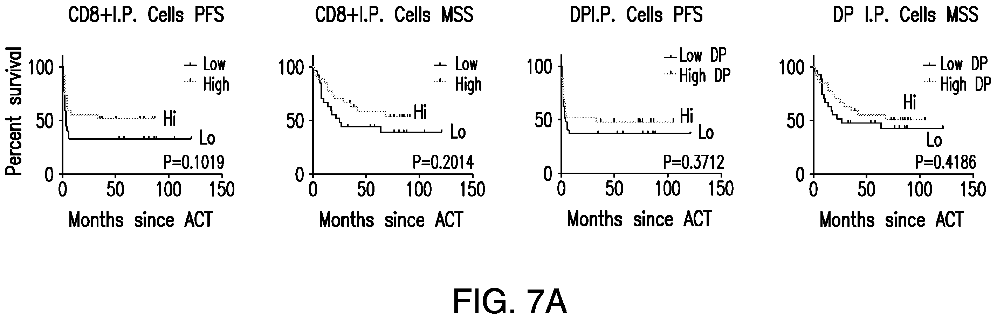

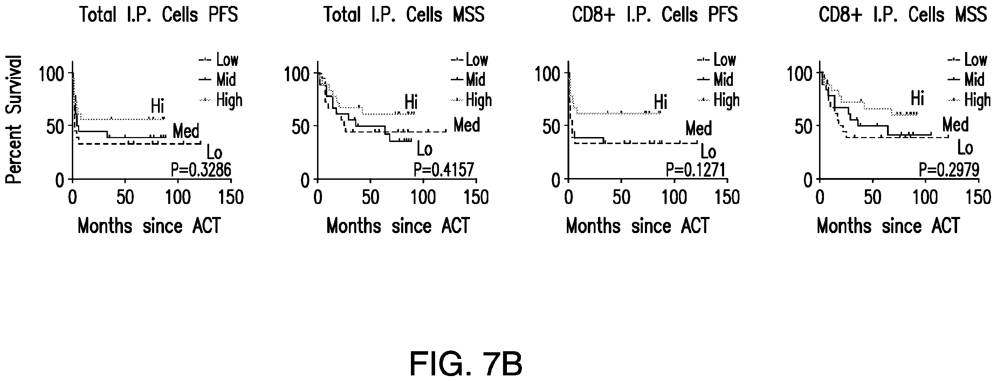

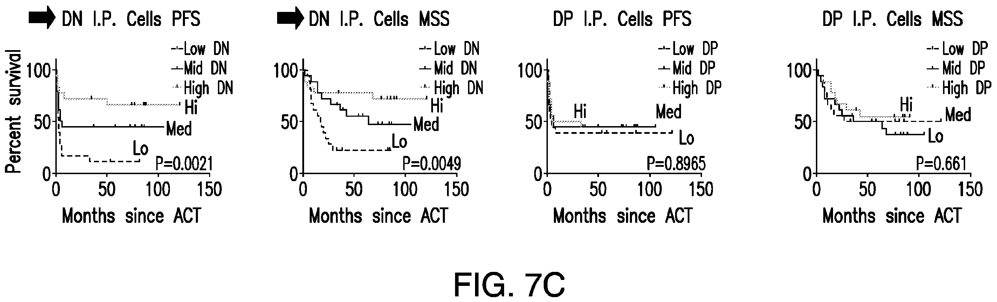

Figure 7A-7C shows graphs showing progression-free survival and melanoma-specific survival according to cell numbers of TIL subpopulations received in infusion products. 7A: PFS and MSS of I.P. cells infused divided at median by CD8⁺ cell numbers and CD39⁺CD69⁺ (DP) cell numbers received into high and low. 7B: lack of dose dependence of patient response on total cell numbers and CD8⁺ cell numbers in I.P. shown by tertiles indicating Low (dashed), Middle and High tertiles of cells. 7C: dose dependence of patient response on CD39⁻CD69⁻ (DN) cell numbers and lack of dose dependence on CD39⁺CD69⁺ (DP) cell numbers in I.P. shown by tertiles indicating Low (dashed), Middle and High tertiles of cells. Arrows indicate the statistically significant tertile analyses from DN cell infused. Numbers in all plots indicate p-values by Mantel-Cox log-rank test.
Figure 8A shows the CD39/CD69 transcriptome analysis on 9335 single cells (circles) from 4 I.P. (2 CRs, 2 NRs) defined 4420 CD39⁻CD69⁻ cells (cells with bottom two quartiles of CD39 and CD69 co-expression) and CD39⁺CD69⁺ cells (cells co-expressing top two quartiles of CD39 and CD69).
Figure 8B shows a volcano plot of differentially expressed genes between DN and DP subsets from Fig. 8A showing statistically significant genes of interest. Full list of genes are listed in Table 3.
Figure 8C shows an overlap of the number of genes found between DN vs DP DEG shown in Fig. 8B and S.Cluster.A-specific genes defined by unsupervised clustering of responder clusters shown in Fig. 2B.
Figure 9A shows a representative flow cytometry plot for inhibitory and memory markers from CD39⁻CD69⁻ (DN), CD39⁺CD69⁺ (DP), and CD39⁻CD69⁺, CD39⁺CD69⁻ (SP) states from CD8⁺ TILs from a patient I.P. (3733-CR). Numbers indicate % of parent gate (DN, SP, and DP states).
Figure 9B shows a summary of paired analysis of the indicated cell surface markers (n = 38 I.P.). ****P* < 0.001 *****P* < 0.0001 by paired t-tests.
Figure 10 is a graph showing secreted cytokines from DN and DP subsets after CD3/CD28 stimulation. DN and DP subsets were isolated from patient I.P. (N = 5) and stimulated with anti-CD3/CD28 beads for 48h followed by multi-cytokine analysis of cytokines secreted in the supernatant of daughter cells. ** *P<* 0.05 ** *P* < 0.01 by paired Wilcoxon test.
Figure 11A-11B are graphs showing mean phenotypic fitness score of TILs from ACT I.P. (N=10) and pre-ICB- treatment CD8⁻TIIs from Sade-Feldman sample cohort. Mean fitness score is defined as the difference between the normalized DN and DP scGSEA scores (DN minus DP) from each cell from patient TIL. Figure 11A shows ACT I.P. from CRs, NRs shown in Fig. 2C. Figure 11B shows pre-treatment TILs from Sade-Feldman cohort shown in Fig. 2G. **** *P* < 0.0001 by two-sided Wilcoxon test.
Figure 12A shows the HLA- restriction definition for neoantigens screened in this study.
Figure 12B shows the neoantigen specificity defined by titration of TILs or TCRs from I.P. against mutant (squares) and wild-type (circles) peptides by IFNγ ELISpots for patients screened in this study. Additional neoantigens were obtained from previously published studies. There is no wild-type for CDKN2A f.s. The last two panels represent HIVEP2 and AMPH NeoTCR from Patient 4000-NR, transduced into healthy PBL and titrated against mutant and wild-type peptides using 4-1BB upregulation of CD8⁺mTCRβ⁺ subset to demonstrate NeoTCR specificity (used in Fig. 3J-3L).
Figure 13 shows the neoantigen T cell identification by tetramers from patient I.P. All 26 neoantigens from 11 patient I.P. shown in Figure 3A-C detected by dual colored tetramers (materials and methods). Numbers indicate neoantigen-specific TIL as a % of CD8+ T cells within each patient I.P. Note that Pt. 3713 had other neoantigens that were non tetramerizable but with verified NeoTCRs.
Figure 14A shows 3713 NeoTCR⁺ cells displayed as % of stem-like C0 cluster and differentiated C1 cluster shown for each NeoAg.
Figure 14B shows post-ACT persistence of other sub-dominant NeoTCR clonotypes from Pt. 3713-CR in peripheral blood normalized to their frequency in the I.P., related to SRPX-NeoTCR data shown in Fig. 3I.
Figure 15A shows 4000 NeoTCR+ cells displayed as a % of stem-like C1/C5 clusters and differentiated C0 cluster shown for each NeoTCR specificity.
Figure 15B shows Pt. 4000-NR NeoTCR+ cells from stem-like C1/C5 cluster and differentiated C0 cluster displayed as a % of total NeoTCR+ cells for each neoantigen specificity.
Figure 16A is a schema for *in vitro* proliferation experiment. CD39⁻CD69⁻ and CD39⁺CD69⁺ CD8 T cells were FACS-isolated from 3733-CR I.P. and co-cultured overnight with autologous 3733-mel tumor cell line. Tumor-reactive CD8⁺4-1BB⁺ cells from DN and DP subsets (DN 4-1BB⁺, DP 4-1BB⁺) were FACS-isolated and subjected to rapid expansion (REP, materials and methods) repeatedly using 10⁵ input T cells with irradiated feeders to mimic ACT TIL infusion products. Tumor reactivity and cell numbers was assessed at completion of REP culture experiment.
Figure 16B shows absolute cell numbers obtained at end of each REP (left) and total tumor-reactive theoretical cell yield assuming all cells as subsequent inputs (right).
Figure 16C shows tumor reactivity of each REP'd subset was assessed by CD8⁺ 4-1BB upregulation after co-culture with autologous 3733-mel tumor line from each population.
Figure 16D shows a flow cytometry plot from one biological replicate shown in Figure S14C.
Figure 17 is a schematic illustrating various embodiments of the inventive methods.
Figure 18A is a schematic illustrating a strategy for identifying accurate markers of neoantigen specific tumor reactive stem-like T-cells that differentiate bystander stem-like T-cells in the stem-like CD39⁻CD69⁻ (DN) cluster of Fig. 3F.
Figure 18B shows a volcano plot of differentially expressed genes between bystander DN T cells and neoantigen-specific T cell subsets from Fig. 3F showing statistically significant genes of interest. Full list of genes are listed in Table 4.

### DETAILED DESCRIPTION OF THE INVENTION

It has been discovered that a CD39⁻CD69⁻ T cell population administered to cancer patients may be associated with complete cancer regression and/or T cell persistence and that a CD39⁺CD69⁺ T cell population administered to patients may be associated with poor persistence. Although T cells administered to both responders and non-responders to ACT may include anti-tumor neoantigen-reactive CD39⁺CD69⁺ T cells, ACT responders were found to have been administered a pool of CD39⁻CD69⁻ neoantigen-specific T cells that was largely lacking in the T cells administered to ACT non-responders. Tumor-reactive CD39⁻CD69⁻ T cells may provide one or more of increased self-renewal, expansion, persistence, and anti-tumor response in the body of a patient as compared to CD39⁺CD69⁺ T cells.

An embodiment of the invention provides a method of obtaining a cell population enriched for T cells with a phenotype comprising markers CD3⁺, CD8⁺, CD39⁻, and CD69⁻. The cell population may be administered to a patient, for example, for the treatment or prevention of cancer.

The method comprises obtaining a bulk population of T cells from a tumor sample of a patient. The tumor sample may be, for example, tissue from primary tumors or tissue from the site of metastatic tumors. As such, the tumor sample may be obtained by any suitable means, including, without limitation, aspiration, biopsy, or resection.

The method further comprises specifically selecting T cells with a phenotype comprising markers CD3⁺, CD8+, CD39⁻, and CD69⁻ from the bulk population. Selecting the T cells which have the phenotype may comprise sorting the T cells into separate single T cell samples and separately detecting the expression and/or non-expression of one or more markers of the phenotype by one or more single T cells. In an embodiment of the invention, specifically selecting T cells with the phenotype comprises carrying out single cell transcriptome analysis.

Detecting the expression and/or non-expression of one or more markers by the one or more single T cells may be carried out using, for example, the CHROMIUM Single Cell Gene Expression Solution system (10x Genomics, Pleasanton, CA) ("CHROMIUM system"). The CHROMIUM system performs deep profiling of complex cell populations with highthroughput digital gene expression on a cell-by-cell basis. The CHROMIUM system barcodes the cDNA of individual cells for 5' transcriptional or T cell receptor (TCR) analysis. For example, samples may start with an input of 10,000 cells and yield data for about 3000 cells/sample, with an average of about 500 genes/cell.

In an embodiment of the invention, specifically selecting T cells with the phenotype comprises carrying out Cellular Indexing of Transcriptomes and Epitopes by Sequencing (CITE-Seq) analysis. CITE-Seq is described at, for example, Stoeckius et al., Nat. Methods, 14(9): 865-868 (2017). Briefly, CITE-seq combines antibody-based detection of protein markers together with transcriptome profiling for many single cells in parallel. Oligonucleotide-labeled antibodies are used to integrate cellular protein and transcriptome measurements into an efficient, single-cell readout.

Because of the high dimensionality of the data yielded by the single cell transcriptome analysis (e.g., about 3000 cells/sample and about 500 genes/cell), dimensionality reduction may be carried out for analysis of the marker expression data. Accordingly, in an embodiment of the invention, specifically selecting T cells with a phenotype comprises carrying out t-Distributed Stochastic Neighbor Embedding (t-SNE) analysis. t-SNE visualizes high-dimensional data by giving each data point a location in a two or three-dimensional map. t-SNE is described at, for example, Van der Maaten and Hinton, J. Machine Learning Res., 9: 2579-2605 (2008). Briefly, t-SNE is carried out in two steps. In step 1, a probability distribution is created in the high-dimensional space that dictates the relationships between various neighboring points. In step 2, a low dimensional space is recreated that follows that probability distribution as best as possible. The "t" in t-SNE comes from the t-distribution, which is the distribution used in Step 2. The "S" and "N" ("stochastic" and "neighbor") come from the use of a probability distribution across neighboring points.

The phenotype may include (i) positive expression of one or more markers, (ii) negative expression of one or more markers, or (iii) positive expression of one or more markers in combination with negative expression of one or more markers. As used herein, the term "positive" (which may be abbreviated as "⁺"), with reference to expression of the indicated marker, means that the T cell upregulates expression of the indicated marker as compared to other T cells in the tumor sample of the cancer patient. Upregulated expression may encompass, for example, a quantitative increase in expression of the indicated marker by an average logarithmic fold change (to the base 2) of about 0.2, about 0.5, about 1, about 2, about 3, about 4, about 5, about 6, about 7, about 8, about 9, about 10, about 11, about 12, about 13, about 14, about 15, about 16, about 17, about 18, about 19, about 20, about 21, about 22, about 23, about 24, about 25, about 26, about 27, about 28, about 29, about 30, about 31, about 32, about 33, about 34, about 35, or a range of any two of the foregoing values, or more. The term "negative" (which may be abbreviated as "-"), as used herein with reference to expression of the indicated marker, means that the T cell downregulates expression of the indicated marker as compared to other T cells in the tumor sample of the cancer patient. Downregulated expression may encompass, for example, a quantitative decrease in expression of the indicated marker by an average logarithmic fold change (to the base 2) of about -0.2, about -0.5, about -1, about -2, about -3, about -4, about -5, about -6, about -7, about -8, about -9, about -10, about -11, about -12, about -13, about -14, about -15, about -16, about -17, about -18, about -19, about -20, about -21, about -22, about -23, about - 24, about -25, about -26, about -27, about -28, about -29, about -30, about -31, about -32, about -33, about -34, about -35, or a range of any two of the foregoing values, or more. Although downregulated expression may encompass an absence of expression of the indicated marker, downregulation also encompasses the presence of the expression of the indicated marker, albeit at a lower level as compared to other T cells in the tumor sample of the cancer patient.

Specifically selecting T cells with the phenotype may comprise detecting the presence or absence of, or measuring the quantity of, the product(s) of expression of the markers in the phenotypes described herein. In this regard, specifically selecting T cells which have the phenotype may comprise detecting the presence of protein(s) encoded by positively expressed marker(s) of the phenotype. Alternatively or additionally, specifically selecting T cells which have the phenotype may comprise detecting the absence of protein(s) encoded by marker(s) that are negative for expression in the phenotype. Alternatively or additionally, specifically selecting T cells which have the phenotype may comprise measuring the quantity of protein(s) encoded by marker(s) that are negative for expression in the phenotype. Alternatively or additionally, specifically selecting T cells which have the phenotype may comprise measuring the quantity of protein(s) encoded by marker(s) that are positive for expression in the phenotype. Alternatively or additionally, specifically selecting T cells which have the phenotype may comprise detecting the presence of RNA encoded by positively expressed marker(s) of the phenotype. Alternatively or additionally, specifically selecting T cells which have the phenotype may comprise detecting the absence of RNA encoded by marker(s) that are negative for expression in the phenotype. Alternatively or additionally, specifically selecting T cells which have the phenotype may comprise measuring the quantity of RNA encoded by positively expressed marker(s) of the phenotype. Alternatively or additionally, specifically selecting T cells which have the phenotype may comprise measuring the quantity of RNA encoded by negatively expressed marker(s) of the phenotype. In an embodiment of the invention, specifically selecting T cells which have the phenotype comprises detecting the presence and/or absence of cell surface expression of the one or more markers in the phenotype. In an embodiment of the invention, specifically selecting T cells which have the phenotype comprises measuring the quantity of cell surface expression of the one or more markers in the phenotype.

In an embodiment of the invention, the phenotype further comprises one or more of marker(s): AHNAK⁺, ALS92183.1⁺, ANXA1⁺, ANXA4⁺, AQP3⁺, ATM⁺, BIN1⁺, C10orf54⁺, C11orf21⁺, C16orf54⁺, CCDC109B⁺, CD27⁺, CD52⁺, CD55⁺, CD8B⁺, CDC25B⁺, CDC42SE1⁺, CLEC2B⁺, CLIC3⁺, CLUAP1⁺, CRBN⁺, CTD-3184A7.4⁺, DDI2⁺, DND1⁺, EMP3⁺, EPB41⁺, ERN1⁺, FAIM3⁺, FAM65B⁺, FBXL8⁺, FGFBP2⁺, GADD45B⁺, GIMAP4⁺, GIMAP7⁺, GPR155⁺, GZMM⁺, HSD17B11⁺, IL10RA⁺, IL7R⁺, ISG20⁺, KANSL1-AS1⁺, KLF2⁺, KLHL24⁺, LDLRAP1⁺, LEF1⁺, LGALS3⁺, LINC00861⁺, LITAF⁺, LYAR⁺, MAPKAPK5-AS1⁺, MED15⁺, MIAT⁺, MIR142⁺, MXI1⁺, MYC⁺, NEAT1⁺, NOSIP⁺, ODF2L⁺, P2RY8⁺, PDE6G⁺, PIK3IP1⁺, PLEC⁻, PLP2⁺, PPP2RSC⁺, PXN⁺, R3HDM4⁺, RAMP1⁺, RASA3⁺, RASGRP2⁺, RCBTB2⁺, RNASET2⁺, RP11-395B7.4⁺, RP11-539L10.2⁺, RP11-640M9.1⁺, S100A10⁺, S100A4⁺, S100A6⁺, S1PR1⁺, S1PR4⁺, SAMD3⁺, SELL⁺, SH3BP5⁺, SIGIRR⁺, SLAMF6⁺, SLCO3A1⁺, SORL1⁺, STK38⁺, SYNJ2⁺, TCF7⁺, TIMP1⁺, TRADD⁺, TSC22D3⁺, TSPAN32⁺, TXNIP⁺, UBXN11⁺, VCL⁺, VNN2⁺, YPEL3⁺, ZFP36L2⁺, ZNF276⁺, and ZNF683⁺. In an embodiment of the invention, the phenotype further comprises any 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, or more of the markers listed in this paragraph. In an embodiment of the invention, the phenotype further comprises all of the markers listed in this paragraph.

In an embodiment of the invention, the phenotype lacks one or more of marker(s): ACOT7⁺, ADAM19⁺, AGPAT9⁺, AGTRAP⁺, AIF1⁺, ASPM⁺, ATAD2⁺, AURKA⁺, AURKB⁺, BIRC3⁺, BIRC5⁺, BRCA1⁺, C15orf48⁺, CASC5⁺, CCL3⁺, CCNA2⁺, CCNB1⁺, CCNB2⁺, CCND2⁺, CD38⁺, CD40LG⁺, CD69⁻, CD8B⁺, CDC20⁺, CDCA3⁺, CDCA8⁺, CDK1⁺, CDKN3⁺, CDT1⁺, CENPA⁺, CENPE⁺, CENPF⁺, CENPM⁺, CENPW⁺, CEP55⁺, CISH⁺, CKS1B⁺, CKS2⁺, CLSPN⁺, CRTAM⁺, CSF2⁺, DLGAP5⁺, DUSP5⁺, DUSP6⁺, DUT⁺, EGR1⁺, ENTPD1⁺, FEN1⁺, GINS2⁺, GTSE1⁺, H2AFX⁺, HIST1H4C⁺, HLA⁺DQA2⁺, HMMR⁺, IFI27⁺, IFNG⁺, IL2RA⁺, IL5⁺, KIAA0101⁺, KIF 11⁺, KIF23⁺, KPNA2⁺, KRT7⁺, MAD2L1⁺, MCM7⁺, MKI67⁺, MX1⁺, MYBL2⁺, NCAPG⁺, NDC80⁺, NDFIP2⁺, NUDT1⁺, NUSAP1⁺, ORC6⁺, PBK⁺, PCNA⁺, PLK1⁺, RPL39L⁺, RRM2⁺, SGOL2⁺, SHCBP1⁺, SMC2⁺, SPC25⁺, STMN1⁺, TESC⁺, TK1⁺, TNF⁺, TNFRSFI8⁺, TNFSF10⁺, TOP2A⁺, TPM4⁺, TPX2⁺, TUBA1B⁺, TUBA1C⁺, TUBB⁺, TYMS⁺, UBE2C⁺, UBE2S⁺, UBE2T⁺, XCL1⁺, and ZWINT⁺. In an embodiment of the invention, the phenotype lacks any 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, or more of the markers listed in this paragraph. In an embodiment of the invention, the phenotype lacks all of the markers listed in this paragraph.

In an embodiment of the invention, the phenotype further comprises one or more of marker(s): AHI1⁺, ALOX5AP⁺, ANXA5⁺, CD68⁺, CD74⁺, CD99⁺, CDC27⁺, CDCA7⁺, CISH⁺, COTL1⁺, CTSH⁺, CTSW⁺, DDX60⁺, GTSF1⁺, HIST1H2AG⁺, HLA-DPA1⁺, HLA-DRB1⁺, HLA-DRB5⁺, HMGN3⁺, IGFBP3⁺, IL32⁺, INTS4⁺, ITGAE⁺, ITGB1⁺, KLRC3⁺, LGALS1⁺, LIME1⁺, PDCD1⁺, PPM1M⁺, PRSS57⁺, RAB34⁺, RBPMS⁺, S100A11⁺, S100A4⁺, SNAP47⁺, TNFRSF10A⁺, VSIR⁺, ZBP1⁺, and ZNF683⁺. In an embodiment of the invention, the phenotype further comprises any 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, or more of the markers listed in this paragraph. In an embodiment of the invention, the phenotype further comprises all of the markers listed in this paragraph. The phenotype described in this paragraph may be characteristic of neoantigen-specific T cells and distinguish the neoantigen-specific T cells from bystander T cells. The phrase "bystander T cells," as used herein, means T cells which have antigenic specificity for irrelevant antigens (not neoantigens).

In an embodiment of the invention, the phenotype lacks one or more of marker(s): AQP3⁺, ASXL2⁺, CD6⁺, CLDND1⁺, EEF1A1⁺, EPB41⁺, ERN1⁺, FCMR⁺, GIMAP4⁺, GIMAP7⁺, GNLY⁺, GZMK⁺, IL27RA⁺, LINC01943⁺, MRPL57⁺, MT-CO1⁺, MT-CO2⁺, RGS10⁺, RPL13A⁺, RPL 18⁺, RPL18A⁺, RPL37⁺, RPL41⁺, RPLP0⁺, SFMBT2⁺, TPT1⁺, and TRGV10⁺. In an embodiment of the invention, the phenotype lacks any 2, 3, 4, 5, 6, 7, 8, 9, 10, 11, 12, 13, 14, 15, 16, 17, 18, 19, 20, 21, 22, 23, 24, 25, 26, or more of the markers listed in this paragraph. In an embodiment of the invention, the phenotype lacks all of the markers listed in this paragraph. The presence of one or more of the markers in this paragraph may be characteristic of bystander T cells, and the presence of one or more of the markers in this paragraph may distinguish the bystander T cells from neoantigen-specific T cells. Conversely, a phenotype which lacks one or more of the markers described in this paragraph may be characteristic of neoantigen-specific T cells and distinguish the neoantigen-specific T cells from bystander T cells.

The phenotype may further comprise CD4⁺. Alternatively, the phenotype further comprises CD4⁻.

The method may further comprise separating the selected cells which have the phenotype from cells which lack the phenotype to obtain a cell population enriched for T cells with the phenotype. In this regard, the selected cells may be physically separated from unselected cells, i.e., the cells that do not have the phenotype. The selected cells may be separated from unselected cells by any suitable method such as, for example, sorting.

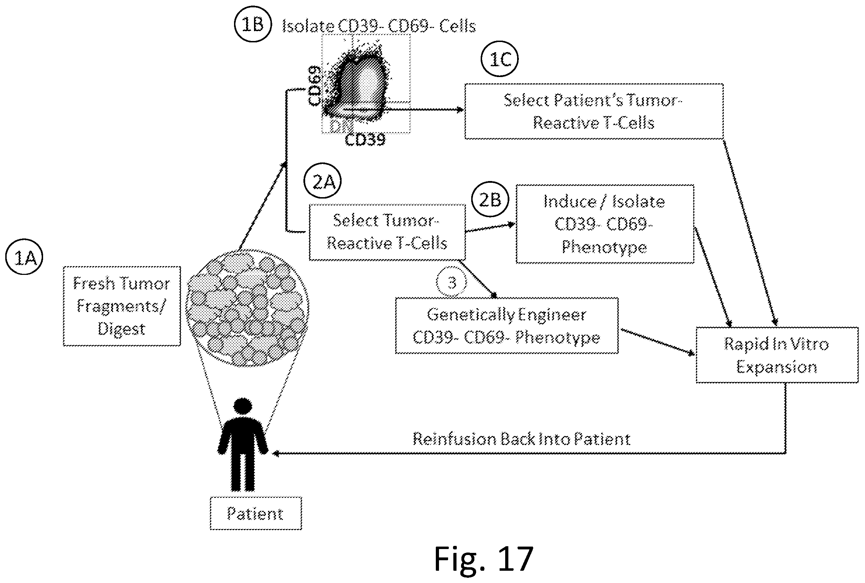

The cell populations enriched for T cells with the phenotype by the inventive methods may themselves be useful for any of a variety of applications, for example, adoptive cell therapy for the treatment of conditions, e.g., cancer. Alternatively or additionally, the cell populations enriched for T cells with the phenotype may be a source of tumor-reactive T cells. Accordingly, in an embodiment of the invention, the method may further comprise isolating tumor-reactive T cells from the separated T cells which have the phenotype. An illustrative example of such an embodiment of the inventive methods is shown by the collection of items 1A, 1B, and 1C of Figure 17.

The tumor-reactive T cells may be isolated from the separated T cells which have the phenotype in any of a variety of ways known in the art. Examples of techniques for isolating tumor-reactive T cells are described in, for example, Pasetto et al., Cancer Immunol. Res., 4: 734-743 (2016); Parkhurst et al., Clin. Cancer Res., 23: 2491-2505 (2017); Cohen et al., J. Clin. Invest., 125: 3981-3991 (2015); Lu et al., Mol. Ther., 26(2): 1-10 (2018); US 2020/0056237; US 2017/0218042; WO 2017/048614; and US 2020/0095548.

The tumor-reactive T cells may have antigenic specificity for a neoantigen. The phrases "antigen-specific" and "antigenic specificity," as used herein, mean that the T cell can specifically bind to and immunologically recognize an antigen, or an epitope thereof, such that binding of the T cell to the antigen, or the epitope thereof, elicits an immune response. Neoantigens are a class of cancer antigens which arise from cancer-specific mutations in expressed protein. The term "neoantigen" relates to a peptide or protein expressed by a cancer cell that includes one or more amino acid modifications compared to the corresponding wild-type (non-mutated) peptide or protein that is expressed by a normal (non-cancerous) cell. A neoantigen may be patient-specific. A "cancer-specific mutation" is a somatic mutation that is present in the nucleic acid of a tumor or cancer cell but absent in the nucleic acid of a corresponding normal, i.e. non-tumorous or non-cancerous, cell.

In another embodiment of the invention, tumor-reactive T cells may first be isolated from the tumor sample, and then the T cells with the phenotype are selected from those isolated tumor-reactive T cells. An illustrative example of such an embodiment of the inventive methods is shown by the collection of items 1A, 2A, and 2B of Figure 17.

In this regard, the invention provides a method of obtaining a cell population enriched for T cells with a phenotype, the method comprising obtaining a bulk population of T cells from a tumor sample of a patient. The bulk population of T cells may be obtained from the tumor sample as described herein with respect to other aspects of the invention.

The method may further comprise isolating tumor-reactive T cells from the tumor sample. This may be accomplished in any of a variety of ways known in the art. Examples of techniques for isolating tumor-reactive T cells from a tumor sample are described in, for example, Pasetto et al., Cancer Immunol. Res., 4: 734-743 (2016); Parkhurst et al., Clin. Cancer Res., 23: 2491-2505 (2017); Cohen et al., J. Clin. Invest., 125: 3981-3991 (2015); Lu et al., Mol. Ther., 26(2): 1-10 (2018); US 2020/0056237; US 2017/0218042; WO 2017/048614; and US 2020/0095548.

The method further comprises specifically selecting T cells with a phenotype comprising markers CD3⁺, CD8+, CD39⁻, and CD69⁻ from the isolated tumor-reactive T cells and separating the selected cells from cells which lack the phenotype to obtain a cell population enriched for T cells with the phenotype. The phenotype may be as described herein with respect to other aspects of the invention. The specific selection of the T cells with the phenotype and the separation of the selected cells from cells which lack the phenotype may be carried out as described herein with respect to other aspects of the invention.

Tumor-reactive T cells with the phenotype may provide one or more of increased self-renewal, expansion, persistence, and anti-tumor response in the body of a patient as compared to T cells without the phenotype. Accordingly, it is contemplated that modifying tumor-reactive T cells to have the phenotype may also provide a T cell population which provides any one or more of these advantages in the body of the patient. An illustrative example of such an embodiment of the inventive methods is shown by the collection of items 1A, 2A, and 2B of Figure 17.

Accordingly, an embodiment of the invention provides a method of obtaining a cell population enriched for T cells with a phenotype comprising markers CD3⁺, CD8⁺, CD39⁻, and CD69⁻, the method comprising obtaining a bulk population of T cells from a tumor sample of a patient and isolating tumor-reactive T cells from the tumor sample. Obtaining the bulk population of T cells from the tumor sample of the patient and isolating tumor-reactive T cells from the tumor sample may be carried out as described herein with respect to other aspects of the invention.

In an embodiment of the invention, the method further comprises introducing a nucleic acid encoding an exogenous TCR into the cells in the population enriched for T cells with the phenotype under conditions to express the exogenous TCR by the cells. By "exogenous" is meant that the TCR is not native to (naturally-occurring on) the T cell. The exogenous TCR may be a recombinant TCR. A recombinant TCR is a TCR which has been generated through recombinant expression of one or more exogenous TCR α-, β-, γ-, and/or δ-chain encoding genes. A recombinant TCR can comprise polypeptide chains derived entirely from a single mammalian species, or the recombinant TCR can be a chimeric or hybrid TCR comprised of amino acid sequences derived from TCRs from two different mammalian species. For example, the antigen-specific TCR can comprise a variable region derived from a murine TCR, and a constant region of a human TCR such that the TCR is "humanized." Any exogenous TCR having antigenic specificity for a cancer antigen (e.g., neoantigen) may be useful in the inventive methods. The TCR generally comprises two polypeptides (i.e., polypeptide chains), such as an α-chain of a TCR, a β-chain of a TCR, a γ-chain of a TCR, a δ-chain of a TCR, or a combination thereof. Such polypeptide chains of TCRs are known in the art. The cancer antigen-specific TCR can comprise any amino acid sequence, provided that the TCR can specifically bind to and immunologically recognize a cancer antigen or epitope thereof. Examples of exogenous TCRs that may be useful in the inventive methods include, but are not limited to, those disclosed in, for example, U.S. Patents 7,820,174; 7,915,036; 8,088,379; 8,216,565; 8,431,690; 8,613,932; 8,785,601; 9,128,080; 9,345,748; 9,487,573; 9,879,065 and U.S. Patent Application Publication Nos. 2013/0116167; and 2014/0378389.

In an embodiment of the invention, the method further comprises introducing a nucleic acid encoding a chimeric antigen receptor (CAR) into the cells in the population enriched for T cells with the phenotype under conditions to express the CAR by the cells. Typically, a CAR comprises the antigen binding domain of an antibody, e.g., a single-chain variable fragment (scFv), fused to the transmembrane and intracellular domains of a TCR. Thus, the antigenic specificity of the CAR can be encoded by a scFv which specifically binds to the cancer antigen, or an epitope thereof. Any CAR having antigenic specificity for a cancer antigen may be useful in the inventive methods. Examples of CARs that may be useful in the inventive methods include, but are not limited to, those disclosed in, for example, U.S. Patents 8,465,743; 9,266,960; 9,765,342; 9,359,447; 9,765,342; 9,868,774; 10,072,078; and 10,287,350.

The term "cancer antigen," as used herein, refers to any molecule (e.g., protein, polypeptide, peptide, lipid, carbohydrate, etc.) solely or predominantly expressed or overexpressed by a tumor cell or cancer cell, such that the antigen is associated with the tumor or cancer. The cancer antigen can additionally be expressed by normal, non-tumor, or non-cancerous cells. However, in such cases, the expression of the cancer antigen by normal, non-tumor, or non-cancerous cells is not as robust as the expression by tumor or cancer cells. In this regard, the tumor or cancer cells can over-express the antigen or express the antigen at a significantly higher level, as compared to the expression of the antigen by normal, non-tumor, or non-cancerous cells. Also, the cancer antigen can additionally be expressed by cells of a different state of development or maturation. For instance, the cancer antigen can be additionally expressed by cells of the embryonic or fetal stage, which cells are not normally found in an adult host. Alternatively, the cancer antigen can be additionally expressed by stem cells or precursor cells, which cells are not normally found in an adult host. Examples of cancer antigens include, but are not limited to, mesothelin, CD19, CD22, CD30, CD70, CD276 (B7H3), gp100, MART-1, Epidermal Growth Factor Receptor Variant III (EGFRVIII), Vascular Endothelial Growth Factor Receptor 2 (VEGFR-2), TRP-1, TRP-2, tyrosinase, human papillomavirus (HPV) 16 E6, HPV 16 E7, NY-BR-1, NY-ESO-1 (also known as CAG-3), SSX-2, SSX-3, SSX-4, SSX-5, SSX-9, SSX-10, MAGE-A1, MAGE-A2, BRCA, MAGE-A3, MAGE-A4, MAGE-A5, MAGE-A6, MAGE-A7, MAGE-A8, MAGE-A9, MAGE-A10, MAGE-A11, MAGE-A12, HER-2, etc. In an embodiment of the invention, the cancer antigen may be a mutated antigen that is expressed or overexpressed by tumor or cancer cells and which is not expressed by normal, non-tumor, or non-cancerous cells. Examples of such cancer antigens may include, but are not limited to, mutated KRAS and mutated p53. T cells having antigenic specificity for a cancer antigen may, advantageously, reduce or avoid cross-reactivity with normal tissues such as, for example, that which may occur using T cells having antigenic specificity for minor histocompatibility antigens. In an embodiment of the invention, the cancer antigen is a neoantigen.

The cancer antigen can be an antigen expressed by any cell of any cancer or tumor, including the cancers and tumors described herein. The cancer antigen may be a cancer antigen of only one type of cancer or tumor, such that the cancer antigen is associated with or characteristic of only one type of cancer or tumor. Alternatively, the cancer antigen may be a cancer antigen (e.g., may be characteristic) of more than one type of cancer or tumor. For example, the cancer antigen may be expressed by both breast and prostate cancer cells and not expressed at all by normal, non-tumor, or non-cancer cells.

Cell populations enriched for T cells with the phenotype which comprise an endogenous cancer antigen-specific TCR (e.g., cancer neoantigen-specific TCR) can also be transformed, e.g., transduced or transfected, with one or more nucleic acids encoding an exogenous (e.g., recombinant) TCR or other recombinant receptor. Such exogenous receptors, e.g., TCRs, can confer specificity for additional antigens to the transformed T cell beyond the antigens for which the endogenous TCR is naturally specific. This can, but need not, result in the production of T cell having dual antigen specificities.

In an embodiment of the invention, a nucleic acid encoding the exogenous TCR or CAR is introduced into any suitable recombinant expression vector. For purposes herein, the term "recombinant expression vector" means a genetically-modified oligonucleotide or polynucleotide construct that permits the expression of an mRNA, protein, polypeptide, or peptide by a host cell, when the construct comprises a nucleotide sequence encoding the mRNA, protein, polypeptide, or peptide, and the vector is contacted with the cell under conditions sufficient to have the mRNA, protein, polypeptide, or peptide expressed within the cell. The vectors of the invention are not naturally-occurring as a whole. However, parts of the vectors can be naturally-occurring. The inventive recombinant expression vectors can comprise any type of nucleotides, including, but not limited to DNA and RNA, which can be single-stranded or double-stranded, synthesized or obtained in part from natural sources, and which can contain natural, non-natural or altered nucleotides. The recombinant expression vectors can comprise naturally-occurring or non-naturally-occurring internucleotide linkages, or both types of linkages. Preferably, the non-naturally occurring or altered nucleotides or internucleotide linkages do not hinder the transcription or replication of the vector. Examples of recombinant expression vectors that may be useful in the inventive methods include, but are not limited to, plasmids, viral vectors (retroviral vectors, gamma-retroviral vectors, or lentiviral vectors), and transposons. The vector may then, in turn, be introduced into the isolated population of T cells by any suitable technique such as, e.g., gene editing, transfection, transformation, or transduction as described, for example, Green and Sambrook, Molecular Cloning: A Laboratory Manual (4th Ed.), Cold Spring Harbor Laboratory Press

(2012). Many transfection techniques are known in the art and include, for example, calcium phosphate DNA co-precipitation; DEAE-dextran; electroporation; cationic liposomemediated transfection; tungsten particle-facilitated microparticle bombardment; and strontium phosphate DNA co-precipitation. Phage or viral vectors can be introduced into host cells, after growth of infectious particles in suitable packaging cells, many of which are commercially available.

In an embodiment of the invention, any of the inventive methods described herein further comprise expanding the number of cells in the population enriched for T cells with the phenotype obtained by the method. Expansion of the numbers of T cells can be accomplished by any of a number of methods as are known in the art as described in, for example, U.S. Patent 8,034,334; U.S. Patent 8,383,099; U.S. Patent Application Publication No. 2012/0244133; Dudley et al., J. Immunother., 26:332-42 (2003); and Riddell et al., J. Immunol. Methods, 128:189-201 (1990). In an embodiment, expansion of the numbers of T cells is carried out by culturing the T cells with one or more non-specific T cell stimuli and one or more cytokines. Examples of non-specific T cell stimuli include, but are not limited to, one or more of irradiated allogeneic feeder cells, irradiated autologous feeder cells, anti-CD3 antibodies, anti-4-1BB antibodies, and anti-CD28 antibodies. In preferred embodiment, the non-specific T cell stimulus may be anti-CD3 antibodies and anti-CD28 antibodies conjugated to beads. Any one or more cytokines may be used in the inventive methods. Exemplary cytokines that may be useful for expanding the numbers of cells include interleukin (IL)-2, IL-7, IL-21, and IL-15. In an embodiment, expansion of the numbers of T cells is carried out by culturing the T cells with OKT3 antibody, IL-2, and feeder PBMC (e.g., irradiated allogeneic PBMC).

An embodiment of the invention further provides an isolated or purified cell population obtained according to any of the inventive methods. The cell population enriched for T cells with the phenotype produced by the inventive methods may provide any one or more of a variety of advantages. The cell population enriched for T cells with the CD39⁻ CD69⁻ phenotype produced by the inventive methods may provide one or more of increased self-renewal, expansion, persistence, and anti-tumor response in the body of a patient as compared to CD39⁺CD69⁺ T cells.

The term "isolated," as used herein, means having been removed from its natural environment. The term "purified," as used herein, means having been increased in purity, wherein "purity" is a relative term, and not to be necessarily construed as absolute purity. For example, the purity can be at least about 50%, can be greater than about 60%, about 70% or about 80%, about 90% or can be about 100%.

The cell population enriched for T cells with the phenotype produced by the inventive methods may be a substantially homogeneous population, in which the population comprises mainly T cells produced by any of the inventive methods described herein. The cell population enriched for T cells with the phenotype produced by the inventive methods can also be a clonal population of cells, in which all cells of the population are clones of a single T cell. In one embodiment of the invention, the population of cells is a clonal population comprising T cells comprising a recombinant expression vector encoding the exogenous TCR or CAR as described herein.

It is contemplated that the cell population enriched for T cells with the phenotype prepared by any of the inventive methods described herein may be included in a composition, such as a pharmaceutical composition. In this regard, an embodiment of the invention provides a method of obtaining a pharmaceutical composition comprising a cell population enriched for T cells with a phenotype, the method comprising obtaining a cell population enriched for T cells with the phenotype according to any of the inventive methods described herein; and combining the cell population enriched for T cells with the phenotype with a pharmaceutically acceptable carrier to obtain a pharmaceutical composition comprising a cell population enriched for T cells with the phenotype.

Preferably, the carrier is a pharmaceutically acceptable carrier. With respect to pharmaceutical compositions, the carrier can be any of those conventionally used for the administration of cells. Such pharmaceutically acceptable carriers are well-known to those skilled in the art and are readily available to the public. It is preferred that the pharmaceutically acceptable carrier be one which has no detrimental side effects or toxicity under the conditions of use.

The choice of carrier may be determined in part by the particular method used to administer the cell population enriched for T cells with the phenotype. Accordingly, there are a variety of suitable formulations of the pharmaceutical composition of the invention. Suitable formulations may include any of those for parenteral, subcutaneous, intravenous, intramuscular, intraarterial, intrathecal, intratumoral, or interperitoneal administration. More than one route can be used to administer the cell population enriched for T cells with the phenotype, and in certain instances, a particular route can provide a more immediate and more effective response than another route.

Preferably, the cell population enriched for T cells with the phenotype is administered by injection, e.g., intravenously. A suitable pharmaceutically acceptable carrier for the cells for injection may include any isotonic carrier such as, for example, normal saline (about 0.90% w/v of NaCl in water, about 300 mOsm/L NaCl in water, or about 9.0 g NaCl per liter of water), NORMOSOL R electrolyte solution (Abbott, Chicago, IL), PLASMA-LYTE A (Baxter, Deerfield, IL), about 5% dextrose in water, or Ringer's lactate. In an embodiment, the pharmaceutically acceptable carrier is supplemented with human serum albumen.

For purposes of the invention, the dose, e.g., number of T cells administered should be sufficient to effect, e.g., a therapeutic or prophylactic response, in the mammal over a reasonable time frame. For example, the number of T cells administered should be sufficient to bind to a cancer antigen or treat or prevent cancer in a period of from about 2 hours or longer, e.g., 12 to 24 or more hours, from the time of administration. In certain embodiments, the time period could be even longer. The number of T cells administered will be determined by, e.g., the efficacy of the particular population of T cells to be administered and the condition of the mammal (e.g., human), as well as the body weight of the mammal (e.g., human) to be treated.

Many assays for determining an administered number of T cells are known in the art. For purposes of the invention, an assay, which comprises comparing the extent to which target cells are lysed or one or more cytokines such as, e.g., IFN-y and IL-2 is secreted upon administration of a given number of such T cells to a mammal among a set of mammals of which is each given a different number of the T cells, could be used to determine a starting number to be administered to a mammal. The extent to which target cells are lysed or cytokines such as, e.g., IFN-y and IL-2 are secreted upon administration of a certain number can be assayed by methods known in the art. Secretion of cytokines such as, e.g., IL-2, may also provide an indication of the quality (e.g., phenotype and/or effectiveness) of a T cell preparation.

The number of T cells administered also will be determined by the existence, nature and extent of any adverse side effects that might accompany the administration of a particular population of T cells. Typically, the attending physician will decide the number of T cells with which to treat each individual patient, taking into consideration a variety of factors, such as age, body weight, general health, diet, sex, route of administration, and the severity of the condition being treated. By way of example and not intending to limit the invention, the number of T cells to be administered can be about 10 x 10⁶ to about 10 x 10¹¹ cells per infusion, about 10 x 10⁹ cells to about 10 x 10¹¹ cells per infusion, or 10 x 10⁷ to about 10 x 10⁹ cells per infusion.

It is contemplated that the cell populations enriched for T cells with the phenotype produced according to the inventive methods can be used in methods of treating or preventing cancer in a mammal. In this regard, the invention provides a method of treating or preventing cancer in a mammal, comprising administering to the mammal any of the pharmaceutical compositions or populations of T cells described herein in an amount effective to treat or prevent cancer in the mammal.

An embodiment of the invention further comprises lymphodepleting the mammal prior to administering the T cells. Examples of lymphodepletion include, but may not be limited to, nonmyeloablative lymphodepleting chemotherapy, myeloablative lymphodepleting chemotherapy, total body irradiation, etc.

The references to the methods of treatment by therapy or prevention are to be interpreted as references to compounds, pharmaceutical compositions and medicaments of the present invention. The terms "treat," and "prevent" as well as words stemming therefrom, as used herein, do not necessarily imply 100% or complete treatment or prevention. Rather, there are varying degrees of treatment or prevention of which one of ordinary skill in the art recognizes as having a potential benefit or therapeutic effect. In this respect, the inventive methods can provide any amount of any level of treatment or prevention of cancer in a mammal. Furthermore, the treatment or prevention provided by the inventive method can include treatment or prevention of one or more conditions or symptoms of the disease, e.g., cancer, being treated or prevented. Also, for purposes herein, "prevention" can encompass delaying the onset or recurrence of the disease, or a symptom or condition thereof.

For purposes of the inventive methods, wherein populations of T cells are administered, the T cells can be cells that are allogeneic or autologous to the mammal. Preferably, the cells are autologous to the mammal.

With respect to the inventive methods, the cancer can be any cancer, including any of leukemia (e.g., B cell leukemia), sarcomas (e.g., synovial sarcoma, osteogenic sarcoma, leiomyosarcoma uteri, and alveolar rhabdomyosarcoma), lymphomas (e.g., Hodgkin lymphoma and non-Hodgkin lymphoma), hepatocellular carcinoma, glioma, head-neck cancer, acute lymphocytic cancer, acute myeloid leukemia, bone cancer, brain cancer, breast cancer, cancer of the anus, anal canal, or anorectum, cancer of the eye, cancer of the intrahepatic bile duct, cancer of the joints, cancer of the neck, gallbladder, or pleura, cancer of the nose, nasal cavity, or middle ear, cancer of the oral cavity, cancer of the vulva, chronic lymphocytic leukemia, chronic myeloid cancer, colon cancer (e.g., colon carcinoma), esophageal cancer, cervical cancer, gastrointestinal carcinoid tumor, hypopharynx cancer, larynx cancer, liver cancer, lung cancer, malignant mesothelioma, melanoma, multiple myeloma, nasopharynx cancer, ovarian cancer, pancreatic cancer, peritoneum, omentum, and mesentery cancer, pharynx cancer, prostate cancer, rectal cancer, renal cancer, small intestine cancer, soft tissue cancer, stomach cancer, testicular cancer, thyroid cancer, ureter cancer, and urinary bladder cancer.

The CD39⁻CD69⁻ T cell phenotype described herein may be useful for selecting therapies for cancer patients. Accordingly, an embodiment of the invention provides a method of selecting a therapy for a cancer patient. This method comprises obtaining a bulk population of T cells from a tumor sample of a patient. The bulk population of T cells may be obtained from the tumor sample as described herein with respect to other aspects of the invention.

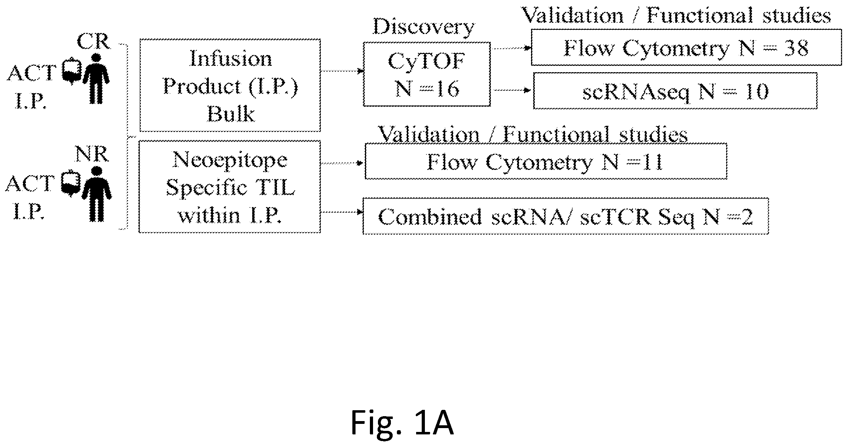

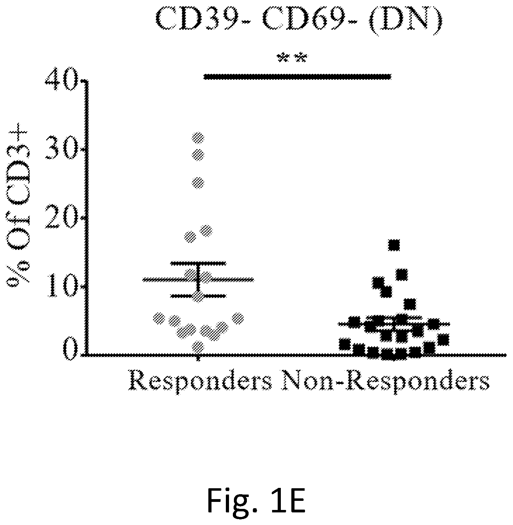

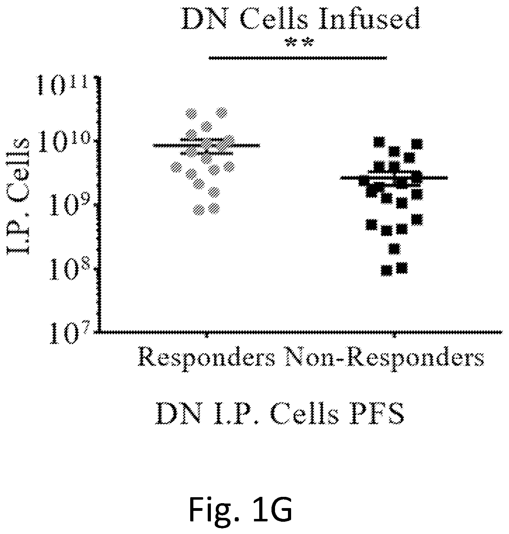

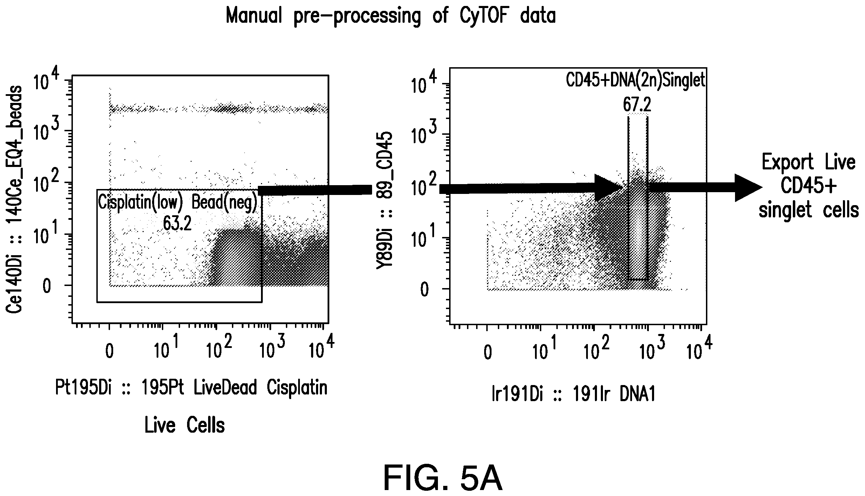

The method further comprises measuring the proportion of T cells in the bulk population which have a phenotype, wherein the phenotype comprises markers CD3⁺, CD8+, CD39⁻, and CD69⁻; and comparing the proportion of T cells which have the phenotype to a control. The method further comprises measuring the proportion of T cells in the bulk population which lack a phenotype, wherein the phenotype comprises markers CD3⁺, CD8+, CD39⁻, and CD69⁻; and comparing the proportion of T cells which (lack the phenotype to a control. The control may be, for example, the results of a study already carried out to determine the proportion of T cells which have (or lack) the phenotype in the infusion product administered to responders and non-responders to ACT in a particular cohort, e.g., a particular cancer type. For example, as shown in Fig. 1E, a median greater than 10% (of T cells with the phenotype in the total population) can be defined as being prognostic of potential response to ACT (found in complete responders) for melanoma. As shown in Figs. 5A-5C, patient infusion products can be classified with a >80% accuracy. The proportion of T cells in the bulk population which have the phenotype may be measured using any suitable method known in the art, for example, flow cytometry.

The method further comprises selecting the patient for a therapy which is not immunotherapy when (i) the proportion of T cells which have the phenotype is lower than the control or (ii) the proportion of T cells which lack the phenotype is equal to or higher than the control; or (e) selecting the patient for an immunotherapy when (i) the proportion of T cells which have the phenotype is equal to or higher than the control or (ii) the proportion of T cells which lack the phenotype is lower than the control.

Another embodiment of the invention provides a method of treating cancer in a patient. The method may comprise receiving an identification of a therapy selected for a cancer patient, wherein the therapy has been selected by the any of the methods described herein with respect to other aspects of the invention. The method may further comprise (I) treating the patient by administering a therapy which is not immunotherapy to the patient in an amount effective to treat cancer in the patient when (i) the proportion of T cells which have the phenotype is lower than the control or (ii) the proportion of T cells which lack the phenotype is equal to or higher than the control; or (II) treating the patient by administering an immunotherapy to the patient in an amount effective to treat cancer in the patient when (i) the proportion of T cells which have the phenotype is equal to or higher than the control or (ii) the proportion of T cells which lack the phenotype is lower than the control. The control may be as described herein with respect to other aspects of the invention.

Immunotherapy encompasses the treatment of cancer by activating or suppressing the immune system. Examples of immunotherapies include, but are not limited to, NK cell therapy, T cell therapy, B cell therapy, immune checkpoint blockade therapy, chimeric antigen receptor (CAR) therapy, antibody therapy, immune system modulator therapy, anticancer vaccine therapy, or any combination thereof. In an embodiment of the invention, the immunotherapy is ACT, immune checkpoint blockade therapy, immune system modulator therapy, T cell therapy, and/or CAR therapy.

Examples of therapies which are not immunotherapy include, but are not limited to, surgical resection, chemotherapy, radiotherapy, stem cell therapy, hormone therapy, targeted drug inhibitor therapy, or any combination thereof.

Another embodiment of the invention provides a method for predicting the clinical response to immunotherapy in a cancer patient. The method comprises obtaining a bulk population of T cells from a tumor sample from the cancer patient. The bulk population of T cells may be obtained from the tumor sample as described herein with respect to other aspects of the invention. The method further comprises measuring the proportion of T cells in the bulk population which (i) have or (ii) lack a phenotype, wherein the phenotype comprises markers CD3⁺, CD8⁺, CD39⁻, and CD69⁻, which may be carried out as described herein with respect to other aspects of the invention.

The method further comprises comparing the proportion of T cells which (i) have or (ii) lack the phenotype to a control. The method may further comprise identifying the patient as likely to have a negative clinical response to the immunotherapy when (i) the proportion of T cells which have the phenotype is lower than the control or (ii) the proportion of T cells which lack the phenotype is equal to or higher than the control. Conversely, the method may comprise identifying the patient as likely to have a positive clinical response to the immunotherapy when (i) the proportion of T cells which have the phenotype is equal to or higher than the control or (ii) the proportion of T cells which lack the phenotype is lower than the control. The control may be as described herein with respect to other aspects of the invention.

The method for predicting the clinical response to immunotherapy in a cancer patient may be carried out before immunotherapy is administered to the patient, after immunotherapy is administered to the patient, or both before immunotherapy is administered to the patient and again after immunotherapy is administered to the patient.

The following examples further illustrate the invention but, of course, should not be construed as in any way limiting its scope.

### EXAMPLES

The following Materials and Methods were employed in the experiments described in Examples 1-5.

### Generation of TILs

Metastatic melanoma tumor deposits were surgically resected and processed for generation of tumor infiltrating lymphocytes (TILs) either by single-cell suspensions generated by enzymatic digest or plate culture of 2-3mm³ tumor fragments as previously described (Tran et al., J. Immunother., 31: 742-751 (2008); Dudley et al., J. Immunother., 26: 332-342 (2003)). Clinical infusion products were generated by rapid expansion of lymphocytes in culture with irradiated PBMC, anti- CD3 antibody, and IL-2 as previously described (Jin et al., J. Immunother., 35: 283-292 (2012)).

### Clinical Protocols

All patients were enrolled in one of the following experimental TIL with IL-2 ACT protocols (NCT00001832, NCT00513604, NCT01319565, NCT01468818, NCT01585415, orNCT01993719) approved by the institution research board of the National Cancer Institute, NIH (Goff et al., J. Clin. Oncol., 34: 2389-2397 (2016); Dudley et al., Clin. Cancer Res., 16: 6122-6131 (2010)). Informed consent was obtained and documented in accordance with the principles set forth in the Declaration of Helsinki. Patients were required to be 18 years of age or older, to have measurable metastatic melanoma, to be of good performance status, to be free of systemic infections, to have received no prior anti-PD-1-targeting therapy, and to be eligible to receive high-dose IL-2 therapy. In addition, for NCT01319565, patients were required to be eligible to receive total body irradiation (TBI). Protocol treatment consisted of a non-myeloablative chemotherapy regimen of cyclophosphamide and fludarabine followed by a single infusion of autologous TILs and high-dose IL-2 to tolerance. A subset of patients received TBI during chemotherapy before cell infusion. Baseline cross-sectional imaging was performed, and extent of disease was evaluated at periodic intervals using Response Evaluation Criteria in Solid Tumors (RECIST) 1.0. Outcomes were classified as complete responses (CR), partial responses (PR) or progressive disease (NR).

### Study Cohort Description

Patient samples were drawn from the clinical protocols according to clinical response and specimen availability. To exclude the influence of prior immunotherapies affecting CD8 TIL differentiation states, patients who had received prior anti-PD-1 immunotherapy or genetically engineered T cell or TCR therapies were excluded. Patients with prior anti-CTLA-4 therapy were not excluded. For this study, complete responders (CRs) were defined as any patients who experienced a complete response per RECIST 1.0 criteria. Among the patients whose disease progressed (NRs, non- responders) following ACT, in order to focus on those patients whose TIL showed a complete lack of antitumor efficacy, and to exclude tumor-intrinsic treatment failures (such as acquired resistance due to treatment), non-responders were stringently identified as the subset of patients with progressive disease whose target lesions experienced no greater than 10% reductions at any time following ACT. These criteria thus excluded patients with stable disease and partial responders from this study resulting in 54 patients with viable TIL infusion product samples available for analysis. The clinical endpoint used in the analyses was progression-free survival (PFS) survival, or melanoma-specific survival (MSS) defined as the length of time from treatment start to time of event (melanoma, death attributed to melanoma, or censor).

### Mass Cytometry Staining

Frozen patient ACT infusion product samples were thawed in warm media, rested overnight without any cytokines in TIL media (RPMI with 10% human serum, L-Glutamine, and Anti-Anti) with DNAse. All reagents were from Fluidigm except where noted. Cells were first incubated with a 5µM solution of cisplatin in PBS to mark dead cells. Cells were then washed and resuspended in MAXPAR Cell Staining Buffer (MCSB) (Fluidigm, South San Francisco, CA). Human Fc-receptor blocking solution (BioLegend, San Diego, CA) was added to each sample and incubated for 10 minutes at room temperature. All metal-labeled antibodies were purchased from Fluidigm. Biotin anti-human CD39 (BioLegend) was detected with QDOT-streptavidin conjugate (Thermo Fisher Scientific, Waltham, MA). Antibodies were diluted in MCSB at concentrations validated for minimal channel spill-over, added to each sample, and incubated for 30 minutes at room temperature, with primary staining consisting of anti-CD39 and secondary staining consisting of all other metal-coupled antibodies + Streptavidin (QDOT-streptavidin conjugate). After each staining, cells were washed with MCSB two times. Cells were then fixed for 15 minutes in a 1.6% solution of paraformaldehyde (MilliporeSigma, Burlington, MA) in PBS. Cell intercalation solution was prepared by adding CELL-ID Intercalator-Ir into MAXPAR Fix and Perm Buffer (Fluidigm) to a final concentration of 125nM. Cells were incubated with cell intercalation solution overnight at 4°C. Cells were then washed once with MCSB and with MAXPAR Water (Fluidigm) twice. Cells were finally resuspended in a water solution containing EQ Four Element Calibration Beads (Fluidigm) at a cell concentration of 10⁶ cells/mL and filtered into cell strainer cap tubes, immediately prior to CyTOF data acquisition. Data were acquired on a HELIOS Mass Cytometer (Fluidigm) as previously described (Bendall et al., Science. 332: 687-696 (2011)).

### Computational Methods and Statistical Analysis of Mass Cytometry data

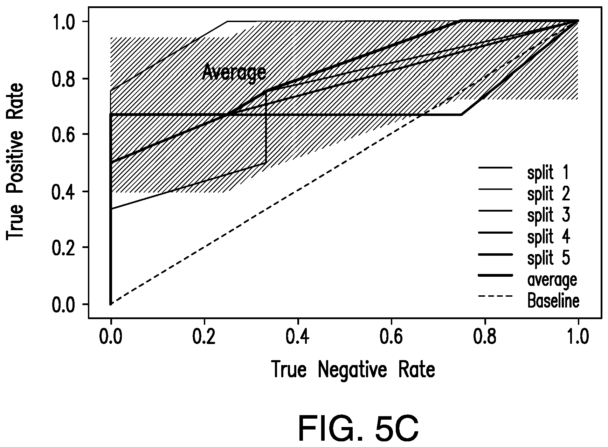

Raw mass cytometry data were normalized with the Normalizer algorithm as recommended by the software developers (Finck et al., Cytometry A., 83: 483-494 (2013)). Data was analyzed using a custom Python script pipeline (available at github.com/Immunodynamics/CyTOF-Processing). Data were first gated as live singlet cells, using [EQ4⁻Cisplatin⁻] and [CD45⁺DNA(2n)] manual gates (Figure 5A). Automatic identification of clusters of differentiation based on surface marker expression was performed using Hierarchical Agglomerative Learning (HAL-x). A custom-programmed algorithm (Figure 5B) relies on cross-validated identification of high-density regions, followed by rapid random forest classification in the 35-dimensional space of surface markers identifying 22 leukocyte populations with distinctive patterns of surface marker expression. HAL-x then labeled all the cells in all samples and computed the inverse hyperbolic sine and normalized frequencies for each leukocyte population. A support-vector machine (SVM) algorithm used the TIL features to determine if samples were taken from patients undergoing complete response (CR) or progressive disease (NR) (Figure 5C and Table 2). Table 2 shows a confusion matrix for classification of patients for their clinical outcome, based on the phenotype of the T cell infusion products (n=1700 runs). True positive (TP), true negative (TN), false positive (FP) false negative (FN). Subsequent analysis prioritized 10 activation features that best SVM-classify patients according to their clinical outcome.

**TABLE 2**

| | | Actual Clinical Outcome | |
|---|---|---|---|
| | | CR | NR |
| Predicted Clinical Outcome | CR | TP=48% | FP=11% |
| | NR | FN=10% | TN=31 % |

### Mass Cytometry Cluster Visualization

To visualize CyTOF data from the 4.8 million cells acquired from patient infusion products, FLOWJO v10.5.3 software (Ashland, OR) was used. The data was gated on live DNA⁺, cisplatin-, CD45⁺ cells, and subsequently gated on CD3⁺ cells to obtain 2.5 million concatenated T cells. Concatenated T cells from all the patient samples were downsampled to 100,000 cells on FLOWJO software, and gates and sample IDs were retransferred to the downsampled population. Dimensional reduction and clustering was performed using the barnes-hut approximation plugin on the program with the following parameters: Perplexity: 90, Learning rate (ETA): 200, Iterations: 1000, Theta: 0.5 and were visualized on the t- SNE space (van der Maaten et al., Machine Learning, 87: pp. 33-55 (2012)). Clusters were defined using the autogating function on FLOWJO software. Cluster frequencies and protein expression per marker were exported, z-scaled, and analyzed using R v3.5.2 statistical environment (R. C. Team, R: A language and environment for statistical computing. Vienna, Austria: R Foundation for Statistical Computing (2014)).

### Flow Cytometry Phenotyping

Frozen patient ACT I.P. samples were thawed and rested overnight without any cytokines in TIL media (RPM1 with 10% human serum) and DNAse. I.P. cells were washed once in staining buffer (1X PBS, 0.5% BSA, 2mM EDTA) and stained with the following antibodies: anti-CD3-APC- Cy7, (SK7; 1:25, BD Biosciences, Franklin Lakes, NJ), anti-CD8-PE-Cy7 (RPA-T8, 1:300, BD Biosciences), anti-CD4-BV785 (OKT4; 1:66, BioLegend, San Diego, CA), anti-CD39-FITC (A1; 1:200, BioLegend), anti-CD69-APC (FN50; 1:25, BD Biosciences, Franklin Lakes, NJ), anti-PD-1-AF700 (EH12.2H7; 1:66, BioLegend), anti-TIM3-BV650 (7D3; 1:100, BD Biosciences), anti-CD62L-BV421 (DREG-56; 1:50, BioLegend), anti-SLAMF6-PE (hSF6.4.20, 1:50, BD Biosciences), anti-CD27-BV421 (O323, 1:50, BioLegend). Samples incubated for 30 min on ice then washed twice and resuspended in staining buffer with propidium iodide to exclude dead cells. For intracellular staining of TCF7, I.P. TILs were washed twice and fixed using TRUE-NUCLEAR Transcription Factor Buffer Set (BioLegend) according to manufacturer's instructions. Fixed and permeabilized cells were washed once and then anti-TCF7-PE antibody (1:50) was added for 20 minutes washed twice, followed by staining with CD3, CD8, CD39 and CD69 antibodies as described above. For neoantigen tetramer phenotyping samples were stained with tetramer first before adding the antibodies above. Samples were acquired on the BD LSRFORTESSA cell analyzer (BD Biosciences) and analyzed on FLOWJO v10.5.3 software. Frequencies of DN, DP, SP, and individual markers were acquired for each patient in two independent biological replicates and the median % was used to compare CRs and NRs.

### Statistical Analysis

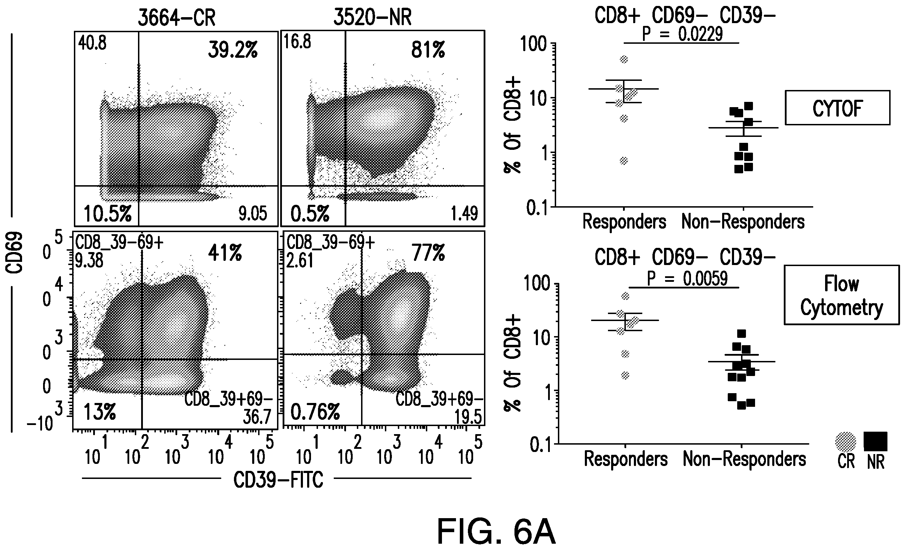

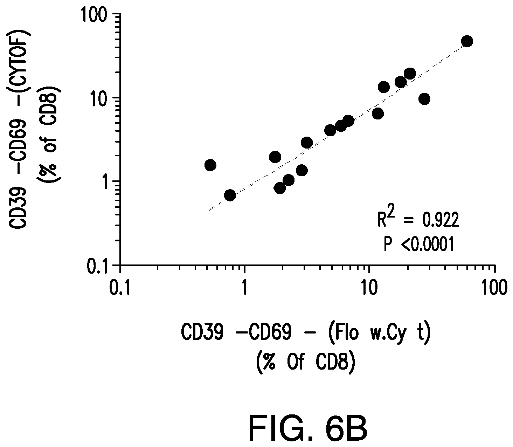

For CyTOF visual cluster comparisons, responder and non-responder clusters were first assessed using Shapiro-Wilk test for normality, and their frequencies were then compared by the Wilcoxon rank-sum test. P-values were corrected with Bonferroni multiple corrections and considered for statistical significance with an alpha of *0.05.* All flow cytometric comparisons between CR and NR I.P. were similarly analyzed using the two-sided Wilcoxon rank-sum test with standard statistical significance levels **P* < 0.05 ***P* < 0.01 ****P* < 0.001 *****P* < 0.0001. Survival analyses (PFS and MSS) were performed using the Kaplan-Meier estimator. Briefly, median T cell numbers infused in the I.P. were first calculated by adjusting total reported cells infused with % of CD3⁺ cells, and by then calculating CD8, DN, and DP % of CD3 to estimate the total CD8 cells infused, total DN cells infused and DP cells infused per each patient. Median was used to stratify high vs. low cell numbers (Fig. 1A-11) and tertiles of cell numbers (Fig. 6A-6C) were used to stratify low, medium, and high cell numbers infused for each of the populations. Mantel-Cox log-rank test was used to determine statistical significance of the survival distributions between patients with calculated hazard ratios (HR). DN versus DP marker expression and progenitor-daughter population was compared using paired t- tests and multi-cytokine comparison was done using paired Wilcoxon tests. For tetramer analyses, all neoantigen-specific TILs were expressed as a % of tetramer⁺ and CR vs. NR sub-group analysis was done using the Wilcoxon rank-sum test adjusting for Bonferroni correction for multiple group comparisons. For differential gene expression analyses, a false discovery rate (FDR) of 0.1 was used to determine DEG genes. Since scRNA data is non-parametric, all CR vs. NR comparisons were performed using the Wilcoxon rank-sum test corrected by the total cell numbers being compared.

Comparison of the TCR CDR3s in patients was performed with the Wilcoxonrank sum test. All statistical analysis was either performed on Graphpad v7.05 or in R Statistical Computing environment v3.5.2 (rproject.org) (R. C. Team, R, *supra*).

### Neoantigen Identification

Tumor whole-exome sequencing (WES): genomic DNA (gDNA) was purified from fresh tumor (FrTu) and matched normal from autologous apheresis samples using the Qiagen ALLPREP DNA/RNA kit (Qiagen, Venlo, Netherlands), as per the manufacturer's suggestions. Whole exome library preparations and sequencing were performed by Novogene (Novogene Corporation Inc., Sacramento, CA) using the Agilent SureSelectXT2 Human All Exon V6 Kit for exome capture and Illumina platforms for paired- end, 2×150 bp sequencing at ~200X on-target coverage. Additional WES for previously published patient tumors was performed by Personal Genome Diagnostics, the Broad Institute, and in the Surgery Branch, NCI on tumor tissue and normal peripheral blood cells as previously described (Parkhurst et al., Cancer Discov., 9: 1022-1035 (2019)).

Exome variant calling: alignments were performed using novoalign MPI from Novocraft to human genome build hg19. Duplicates were marked with Picard's MarkDuplicates tool. Indel realignment and base recalibration were carried out according to the GATK best practices workflow. Variants were called using Varscan2, SomaticSniper, Strelka, and Mutect. Following variant calling VCF files were merged using GATK CombineVariants tools and annotated with Annovar. The parameters to retain a called variant are as follows: a tumor and normal coverage of greater than 10, a variant allele frequency of 7% or above, variant read counts of 4 or above, and two of the four callers identifying mutations.

RNA-seq alignment, processing and variant calling: alignments were performed using the STAR two pass method to human genome build hg19. Duplicates were marked and sorted using Picard's MarkDuplicates tool. Reads were then split and trimmed using the GATK SplitNTrim tool, after which indel realignment and base recalibration were performed as done with whole exome data. A pileup file was created using the final recalibrated bam file and variants were called using Varscan2 only.

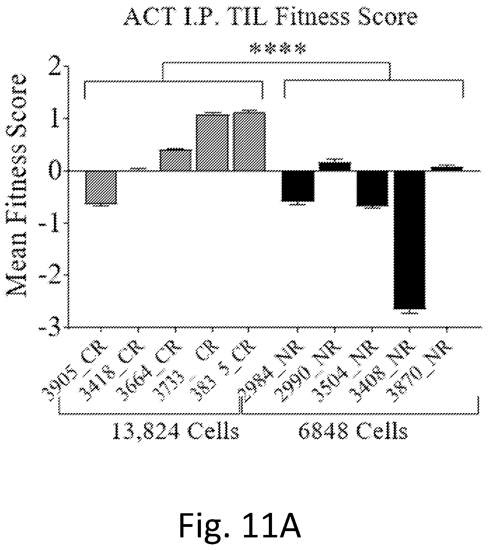

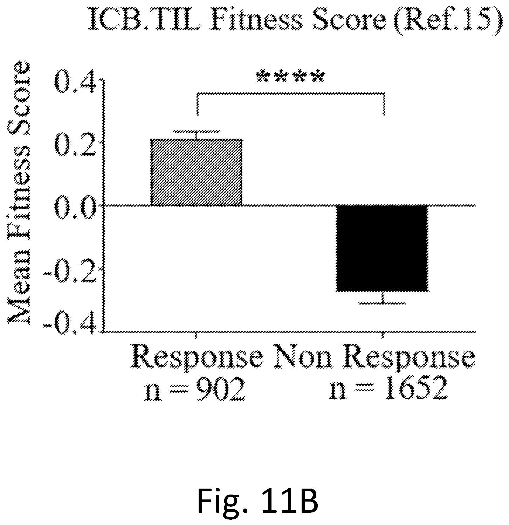

Tandem minigene screening: minigenes designed as 25mers, centered around mutated amino acids, and encompassing tumor mutations were concatenated into TMGs (10-12 TMGs per patient, 15-20 mingenes per TMG) and synthesized in pcRNA6SL plasmid (Genscript, NJ) for *in vitro* RNA transcription (mMESSAGE IVT Kit, Thermo Fisher Scientific) to be used for neoantigen identification as described before (Robbins et al., Nat. Med., 19: 747-752 (2013); Parkhurst et al., Cancer Discov., 9: 1022-1035 (2019); Tran et al., Science, 344: 641-645 (2014); Zacharakis et al., Nat. Med., 24: 724-730 (2018)). Briefly, autologous patient-derived immature dendritic cells (DC) or CD40L-activated B-cells were generated as previously described (Parkhurst et al., Cancer Discov., 9: 1022-1035 (2019); Zacharakis et al., Nat. Med., 24: 724-730 (2018)), and electroporated with individual TMG RNAs (BTX or Lonza 4D), to be processed and presented by any of the patient's class I HLA molecules. Patient I.P. or pre-infusion TIL was thawed, rested overnight in TIL media with IL-2, and then cocultured the next day with RNA-electroporated autologous APCs and evaluated for recognition via interferon gamma cytokine release by ELISpot and TIL activation by 4-1BB expression as described before (Parkhurst et al., Cancer Discov., 9: 1022-1035 (2019)). Once positive "hits" of TMGs were obtained in the preliminary screen, predicted candidate HLA class I minimal peptides from each potential TMG hit were synthesized using fmoc chemistry in-house. Autologous APCs were peptide pulsed in an independent deconvolution experiment (10ug/mL) to evaluate IFNγ ELISpot and 4-1BB TIL activation to identify candidate minimal neoepitopes. Potential minimal peptides were then resynthesized at HPLC purity (Genscript, NJ) to be further used for HLA-restriction mapping (described below) and mutation specificity. Mutation specificity tests were done by peptide pulsing HPLC-grade wild-type and mutant peptides at different concentrations on autologous APCs followed by co-culturing with TIL or TCR-transduced cells to determine titration of mutant and wild-type peptides (Fig. 11A-11B).

HLA restriction mapping: rapid transfection of COS7 cells was performed using patient-specific HLA class I alleles. Gene plasmids for each individual HLA allele (100ng/well) and the tandem minigene (TMG) containing the mutated neoantigen (50ng/well) were combined with LIPOFECTAMINE 2000 transfection reagent (Thermo Fisher Scientific) in a flat-bottom 96-well plate. COS7 cells were added to the plate at 3×10⁴ cells/well in culture media containing DMEM, 10% fetal bovine serum, L-glutamine, penicillin/streptomycin, and amphotericin B. Cells were incubated overnight at 37°C. The following day, the cells were washed with PBS, trypsinized, and transferred to an IFNγ capture antibody-coated ELISpot plate for co-culture with T cells (2×10⁴ cells/well) in RPMI 1640, 10% human serum, HEPES, L-glutamine, and penicillin/streptomycin. Following overnight co-culture at 37°C, T cell response was measured using IFNγ secretion and expression of CD137/4-1BB by flow cytometry.

### Neoantigen TIL Phenotyping

Once mutation specificity was demonstrated by mutant versus wildtype peptide titration of T cell populations, neoantigen-specific T cells in the I.P. were detected and phenotyped using UV exchangeable HLA-monomers for the corresponding HLA as described before (Kvistborg et al., Sci. Transl. Med., 6: 254ra128 (2014)). Briefly, neopeptides with confirmed mutant specificity were produced in-house, and 45µg of neopeptide was co-incubated with 10µg UV-exchangeable biotinylated HLA monomer in a 50µL volume for 90 minutes under 366nm UV light on ice in dark. Following neopeptide exchange, HLA tetramerswere made adding 10µL of Streptavidin-PE (Thermo Fisher Scientific) and 10µL of Streptavidin- APC or Streptavidin-BV421 (BioLegend) in 2µL increments every 15 minutes for 2 hours. Neoepitope tetramers were then centrifuged for 5 minutes 2000 rpm before use. For tetramer staining, cells were first resuspended in 100µL AIM-V media (Gibco) and 1mM Dasatinib (Thermo Fisher Scientific) for 30 minutes, followed by 0.5-2µL tetramer in PE and APC per 100µL staining buffer for 30 minutes on ice. Tetramer-stained cells were washed once and then phenotyping antibodies were stained and acquired as described above. Dual colored tetramers were used to acquire tetramer⁺ events and setting of neoantigen gates. Tetramer-PE was used along with other antibodies for phenotyping. Note that Pt. 3713 had other neoantigens that were non tetramerizable but with verified NeoTCRs are shown in S11. Neoantigen TIL phenotypes were expressed as a % of Tetramer+ events to normalize CR and NR neoantigen TILs for phenoyptic comparison.

### Neoantigen-specific TCR (NeoTCR) Identification

Neoantigen-specific tetramer⁺ TILs from I.P. were sorted using the SH800S Cell Sorter, or the MA900 Multi-Application Cell Sorter (Sony Biotechnologies, San Jose, CA) into 96-well plates with lysis buffer. One of the following two methods were used to identify NeoTCRs.

scRT-PCR: tetramer⁺ or TMG-reactive TILs were directly sorted into RT-PCR buffer containing gene-specific primers targeting constant regions of human TCRα and TCRβ as well as forward primers pools of TCRα and TCRβ family as described before (Pasetto et al., Cancer Immunol. Res., 4: 734-743 (2016)). Briefly, RT-PCR master mixes were developed using CELLSDIRECT one-step qRT-PCR kit (Thermo Fisher Scientific) using: 50°C for 15 min, 95°C for 2 min, 20 cycles of 95°C 15 sec, 60°C for 30 sec. A second PCR was individually done for TCRA and TCRB using nested TCRA and TCRB family primers with a touchdown PCR program. 3µl of the first RT-PCR product was added as template in total 25µl PCR mix using PLATINUM II Hot-Start PCR Master Mix (Thermo Fisher Scientific). The touchdown cycling conditions were 95°C 5 min; higher annealing temperature step (94°C 30s, 60°C 15s, 72°C 30 min × 5 cycles); high annealing temperature step (94°C 30s, 55°C 15s, 72°C 30 min × 5 cycles), low annealing temperature step (94°C 30s, 50°C 15s, 72°C 30 min × 40 cycles), 72°C 10 min; 4°C. The PCR products were purified and sequenced by Sanger method with an internally nested Cα and Cβ regions primers by Beckman Coulter (Schaumburg, IL).

scTCR profiling kit: tetramer⁺ or TMG-reactive TILs were sorted into plates using either SMARTER Human scTCR a/b Profiling Kit (Takara Bio, Shiga, Japan, Cat #634432) or the Takara SMARTER Human scTCR a/b Profiling Kit - 96 (Takara Bio, USA) according to manufacturer instructions. Briefly, single cells from populations of interest were sorted into wells of a 96-well plate and subjected to cDNA synthesis and amplification using SMART technology to incorporate cellular barcoding. cDNA corresponding to TCRα and TCRβ transcripts was further amplified and prepared for sequencing on an Illumina MiSeq instrument. Sequencing was performed by paired-end, 2×300bp reads using the MiSeq Reagent Kit v3 (600 cycle) (Illumina, San Diego, CA, MS-102-3003). Read extraction and clonality counts were determined by MiXCR package (milaboratory.com/software/mixcr/). Both methods yielded TCRs that were reconstructed in the pMSGV1 vector with a murine constant TCR that was transduced into a healthy donor PBL for *in vitro* testing of wild-type and mutant neoepitopes as described above (Pasetto et al., Cancer Immunol. Res., 4: 734-743 (2016); Parkhurst et al., Clin. Cancer Res. 23: 2491-2505 (2017)).

### Single-Cell Transcriptome Analysis

Single-cell capture and library preparation: TILs were washed in PBS with 0.04% BSA twice by pelleting cells at 300g for 5 min at 4°C in a swinging-bucket centrifuge. Cells were counted for cell viability on LUNAFL automated fluorescent cell counter (Logos Biosystems, South Korea) using AO- PI. Cells were partitioned and lysed, followed by reverse transcription of mRNA with single-cell barcoding performed using the Chromium controller with either 3' gene expression chemistry v3.0 or with 5' immune cell profiling chemistry v1.0 and v1.1 (10x Genomics, Pleasanton, CA). Single-cell cDNA amplification, TCR enrichment, and library preparation was performed according to respective user guides. Following patient I.P. was sequenced using 3' GEX v3.0 for transcriptome: 2984-NR, 2990-NR, 3504-NR 3408-NR, 3870-NR, 3905-CR, 3418-CR, 3664-CR, 3733-CR, 3835-CR. The following patient I.P. were sequenced using 5' GEX + TCR v1.0 for combined RNA transcriptome and TCR identification: 3713-CR and 4000-NR.

Single-cell sequencing: single-cell gene expression libraries were sequenced on a NEXTSEQ 550 sequencer with an insert read length of 98 base pairs. For samples containing a TCR-enriched library, the VDJ region was sequenced by paired-end 150 base pair sequencing on either a MiSeq or a NEXTSEQ 550 sequencer.

scRNA data processing: sequencing output was processed using the Cell Ranger 3.0 pipeline (10x Genomics). In short, sequencing output was demultiplexed and converted to fastq file sets. The fastqs associated with gene expression libraries were aligned to the GRCh38 reference provided by 10x Genomics (refdata-cellranger-GRCh38-3.0.0) and for TCR libraries, refdata-cellranger-vdj- GRCh38-alts-ensembl-3.1.0. Unique molecular identifier- (UMI-) collapsed read counts were attributed to individual cell barcodes to generate single-cell gene expression matrices, and in the case of TCR libraries, a single-cell TCR clonotype list that included alpha and beta sequences was generated. Sequencing and single-cell assay performance were evaluated to ensure reliable gene expression and TCR clonotype results, with a target read depth of over 30,000 mean reads per single cell for gene expression and over 5,000 mean reads per single cell for VDJ datasets. Gene expression matrices representing the UMI-collapsed reads count per annotated gene for each cell barcode were generated. Cells with less than 200 genes and genes with less than 5 read counts across all cells were filtered out of the expression matrix. TCR clonotypes were defined based on TCR variable CDR3β nucleotide sequences, and single cells with two different TCR variable CDR3β nucleotide sequences (likely doublets) were excluded. The expression matrix was converted to TPM and cell barcodes were used to uniquely match to associated TCR clonotypes. CD4 cells defined by transcriptome were excluded from scRNA analysis. Cells were first normalized with convolution methods (Lun et al., Genome Biol., 17: 75 (2016)). The normalized data was then decomposed using randomized principal component analysis (Halko et al., SIAM Review, 53: 217-288 (2011)).

scRNA analysis: all scRNA and scTCR analysis were done using R package Seurat v2.4 (Butler et al., Nat. Biotechnol., 36: 411-420 (2018)). For scRNA transcriptome analysis of responders and non-responders, a single batch-corrected gene expression matrix was used as the input. Briefly, gene expression matrix was subject to standard pre- processing by regressing out highly variable genes and mitochondrial genes followed by normalization and scaling. Principal components (PCs) were generated and elbow and Jackstraw plots were used to define significant PCs for clustering. t-SNE plots were generated by the clustered PCs; clusters and super-clusters were defined for comparing CRs and NRs. Cluster markers were obtained for S.Cluster A and B by comparing individual clusters using default parameters. For combined scRNA/scTCR I.P. analysis (3713-CR and 4000-NR), an individual gene expression matrix was generated for each patient. All TRAV/TRBV genes were excluded to remove endogenous TCR expression as a source of clustering bias prior to processing by Seurat. For two cluster solution, lower resolution clustering of first and second principal components was carried out followed by analysis of frequency distribution of all CR and NR cells to denote Super Cluster A and Super Cluster B. Individual cluster markers were obtained for each cluster by default parameters on Seurat.

Combined scRNA and scTCR analysis: neoepitope-reactive TCR clonotypes (NeoTCRs) associated with individual cell barcodes using TCRB and TCRA CDR3 sequences were generated into an independent metadata file. Cells that had good quality reads for TCRA or TCRB were included in subsequent analysis. NeoTCR⁺ cell barcode metadata was back-projected ont-SNE transcriptome clusters identified within patient clusters and were scored for DN/DP gene signatures as described below. As noted above, endogenous cellular TCRs were removed to reduce TRAV/TRBV gene expression as a source of clustering bias. Cells which failed pre-processing for transcriptome were excluded from analyses even if they had intact TCRB/TCRA reads.

Differential expression of genes (DEG) analysis: ENTPD1 (CD39) and CD69 transcript expression from single cells in patient I.P. (3664-CR, 3733-CR, 3408-NR, 3504-NR) were used to define CD39⁻ CD69⁻ cells (bottom two quartiles of CD39, CD69 TPM) and CD39⁺CD69⁺ cells (top two quartiles of CD39,CD69 TPM) (Fig. 6A). DEG comparisons between DN and DP single cells were generated with the edgeR package with FDR < 0.1 and fold change of 0.5. For public gene signature analysis, a gene signature matrix was generated, derived from ImmunesigDB selected by human gene sets restricted to CD8 T cells and other gene sets for a total of 568 gene sets (Godec et al., Immunity, 44: 194-206 (2016)). GSEA was run using default parameters on R as described before in (gseamsigdb.org/gsea/index.jsp). Top 100 statistically significant genes that were enriched in CD39⁻CD69⁻ and CD39⁺CD69⁺ were considered as DN and DP gene signatures respectively.

Single-cell gene signature analysis: single-cell gene set enrichment analysis (scGSEA), adapted from single-sample gene set enrichment analysis (ssGSEA), is a rankbased gene signature metric that computes the expression score of a gene list relative to all other genes in RNA expression (bioconductor.org/packages/release/bioc/html/GSVA.html) on a per-sample basis ( 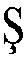 enbabao 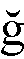 lu et al., *Genome Biol.,* 17: 231 (2016)). In brief, normalized scRNA TIL I.P. data with barcodes was used as input with gene set lists. scGSEA scores for all gene signatures were z-scaled for cross-signature comparisons across cells and samples. Clustered correlation matrix between various gene signatures from single T cells was generated using the R package Corrplot. Top two quartiles of DN and DP scGSEA scores were projected on t-SNE plots to identify clusters enriched in the respective gene signatures. Mean DN, and DP scGSEA scores were used to assess responders and non-responders using the Wilcoxon rank- sum test. Phenotypic fitness scores were calculated as the difference between each cell's DN and DP scGSEA score (DN minus DP) indicating enrichment of relatively stem-like phenotypes at the single cell level. Mean fitness scores were calculated for each patient I.P. and compared between responders and non-responders of ACT and ICB. For cross-study comparison of DN and DP subsets with gene signatures, a correlation matrix was constructed between various gene signatures from single T cells using the package Corrplot and displayed after hierarchical clustering. For TCR clonotype comparison, the mean scGSEA scores of all cells expressing a particular NeoTCR were calculated, projected on t-SNE plots, and compared between clonotypes.

### Short-term TIL stimulation

Patient I.P. were thawed, rested overnight without cytokines, and DN (CD39⁻ CD69⁻) and DP (CD39⁺CD69⁺) subsets were flow-sorted and rested for one hour. 10⁵ cells per each TIL population were then stimulated with plate-bound anti-CD3/CD28 (Invitrogen, Waltham, MA) for 48 hours in a round-bottom 96-well plate.

### Multicytokine Analysis of stimulated TIL

Following 48 hours of CD3/CD28 stimulation, 96-well plates containing the stimulated TIL were centrifuged for 5 min at 300g and 25µL cleared supernatant was subjected to the CD8/NK panel LEGENDPLEX (Cat. # 740267, BioLegend) assay using V-bottom plates in technical replicates per manufacturer's instructions. Captured cytokines were analyzed by flow cytometry on the BD LSRFORTESSA cell analyzer (BD Biosciences). Standard curves for each cytokine were generated and concentration of secreted cytokines from stimulated TIL populations were generated using the manufacturer software (VigeneTech v8 from BioLegend).

### In vitro anti-tumor TIL expansion experiment

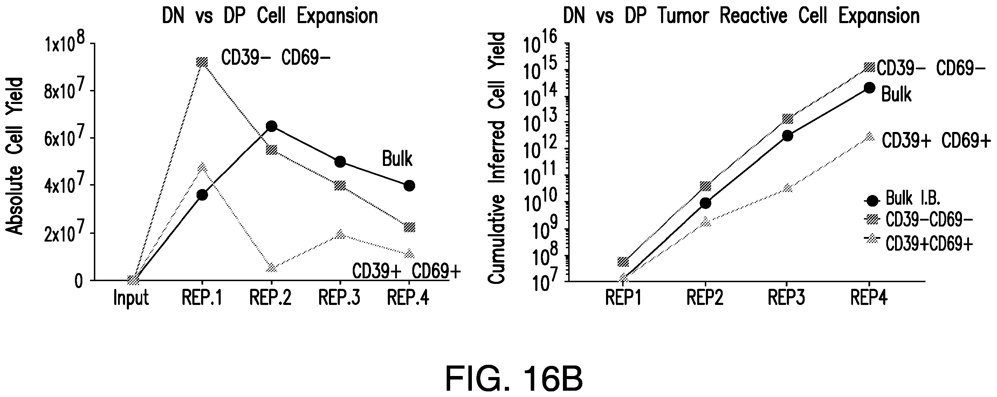

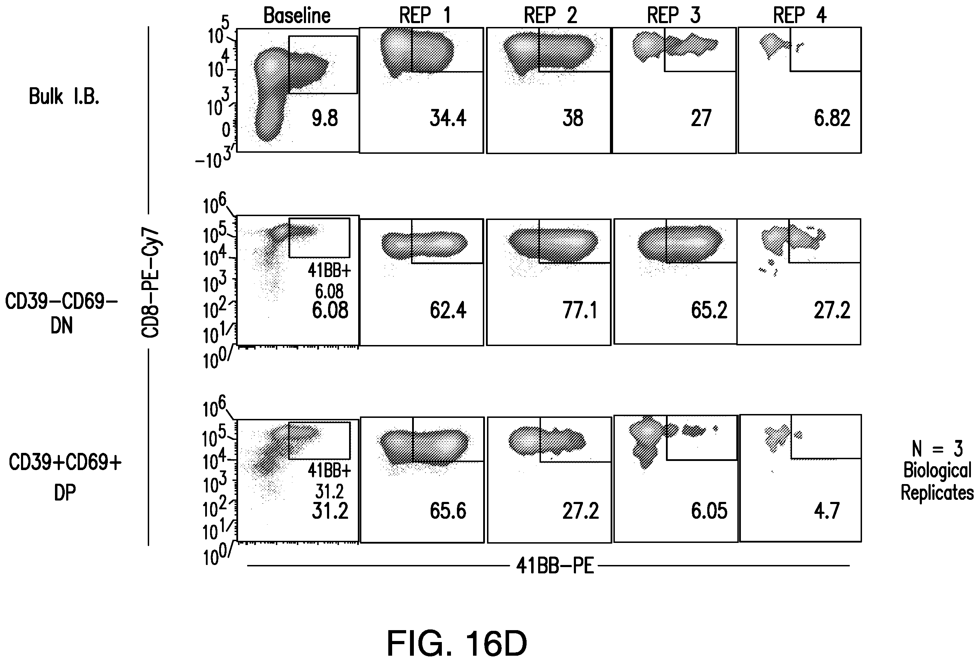

3733-CR I.P. was thawed and rested overnight without cytokines. The following day, CD39⁻ CD69⁻ (DN) and CD39⁺CD69⁺ (DP) CD8 T cells were isolated by flow sorting; DN, DP, and bulk I.P. T cells were co-cultured overnight with the autologous 3733-mel tumor cell line at a ratio of 1:1. Tumor-reactive cells were flow-sorted from CD8⁺4-1BB⁺ from DN, DP, and bulk populations (DN 4-1BB⁺, DP 4-1BB⁺, and bulk 4-1BB⁺) and 10⁵ cells were subjected to rapid expansion (REP) as described before (Dudley et al., J. Immunother., 26: 332-342 (2003)). At the end of each REP, cells were counted and 10⁵ of the resulting cellswere subjected to an additional REP while the remainder were cryopreserved. At the completion of the third REP, cryopreserved samples were thawed and recovered, and tumor reactivity was assessed by co-culture with autologous 3733-mel tumor cell line at a ratio of 1:1 in biological triplicate, with 4- 1BB⁺ as the readout of tumor reactivity. Viable cell numbers were assessed at the end of each REP. For theoretical cell expansion yield calculations (Fig. 16B), the % of 4-1BB⁺ tumor-reactive fraction was expressed as a % of total CD8⁺ live cells and the total cell yield from 1e5 cells was used to infer theoretical tumorspecific cell yield.

### CDR3β survey sequencing and tracking

Cryopreserved infusion product or PBL samples were thawed, tested for viability, and counted. The cells were either first flow-sorted for markers including CD39 and CD69 as indicated or directly pelleted, snap frozen, and sent to Adaptive Biotechnologies (Seattle, WA) for genomic DNA extraction and IMMUNOSEQ TCRB survey sequencing (v4) (Pasetto et al., Cancer Immunol. Res., 4: 734-743 (2016)). Frequencies of known neoantigen- reactive CDR3β were then compared across different samples from a given patient.

### NY-ESO-1-reactive TCR phenotyping

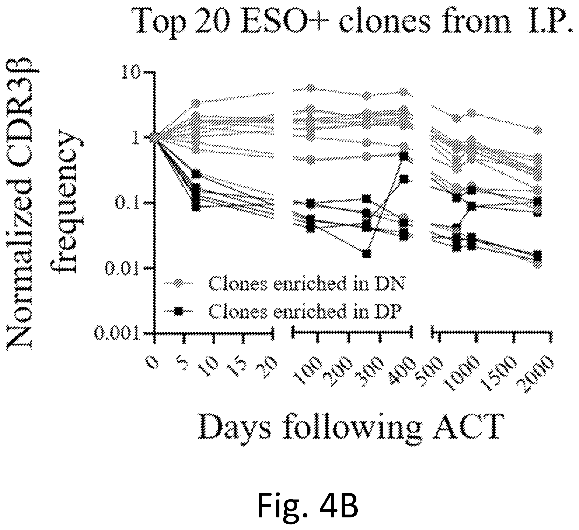

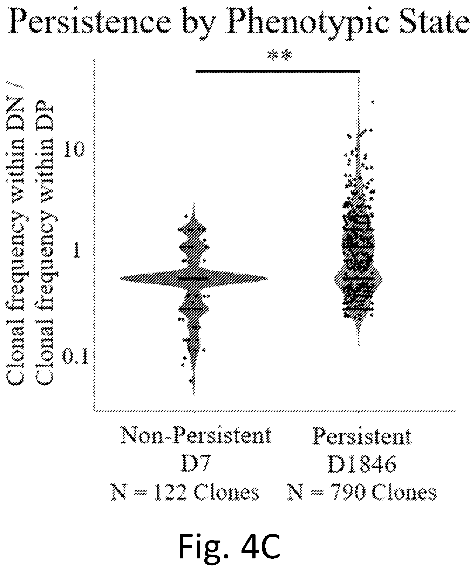

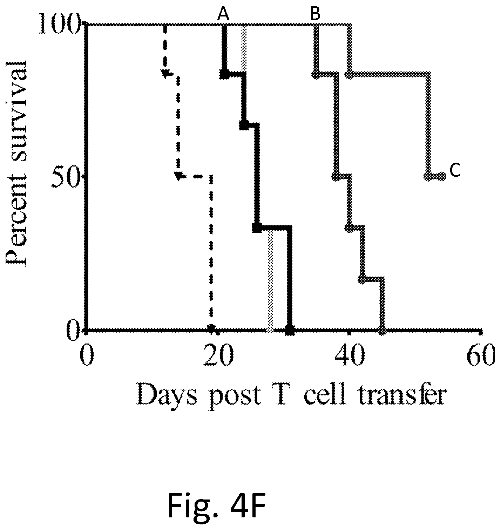

For experiments involving the sequencing of T cells from a patient (ESO.CR) treated with NY-ESO-1-specific TCR-transduced cells, thawed I.P. were stained for CD8, NY-ESO-1 TCR (HLA-A*02:01-SLLMWITQC (SEQ ID NO: 17) peptide tetramer, ProImmune, UK) conjugated to APC, CD39-PE, and CD69-FITC, and three populations were isolated for CDR3β survey sequencing: CD8⁺NY-ESO- 1 TCR⁺(bulk), CD8⁺NY-ESO-1 TCR⁺CD39⁻CD69⁻ (DN), and CD8⁺NY-ESO-1 TCR⁺CD39⁺CD69⁺ (DP). The true endogenous CDR3β frequencies within the TCR-transduced cells were estimated by normalizing endogenous CDR3β sequences to the frequency of the CDR3β of the NY-ESO-1-specific TCR construct (CASSYVGNTGELFF) (SEQ ID NO: 18). In Fig. 4B, using the normalized NY-ESO-1 TCR- expressing I.P. CDR3β data, the top 20 clones in the infusion bag were divided into DN enriched and DP enriched based on the ratio of the frequency of a given clone in the sorted DN cells to its frequency in the sorted DP cells, any clone with a DN / DP ratio > 1 is considered DN enriched and any clone with a DN / DP ratio < 1 is considered DP enriched. For Fig. 4C, 1311 top common clones were defined as those present in each of the bulk, DN, and DP populations. They were analyzed for their short-term (7 days) and long-term (51 months) persistence according to the ratios of their frequency within the DN and DP populations of cells sorted from the I.P.

### Murine T cell culture

Pmel splenocytes were isolated and pulsed with human gp100(25-33) peptide and expanded for 5 days in the presence of 60IU IL2 and complete mouse T cell media as previously described (24). (Klebanoff et al., Clin. Cancer Res., 17: 5343-5352 (2011)). Day 5 cells were collected and restimulated with plate bound anti-CD3 (2µg/ml) and soluble CD28 (1µg/ml) in the presence of IL2 and expanded until day 10. After 10 days in culture the cells were fractionated into DN (CD39⁻CD69⁻) and DP (CD39⁺CD69⁺) for tumor treatment experiments.

### Murine tumor treatment

Adult 6-8-week-old female B6 NCR (B6; Ly5.2⁺) mice were purchased from Charles River Laboratories at NCI Frederick. B6. SJL-Ptprca Pepcb/BoyJ (Ly5.1⁺) mice obtained from The Jackson Laboratory (Bar Harbor, ME) were maintained under specific pathogen-free conditions. For tumor treatment experiments, female B6 mice aged 6-8 weeks were injected with 3.5 × 10⁵ of B16 melanoma cell line that overexpresses the chimeric human/mouse gp100 antigen KVPRNQDWL (SEQ ID NO: 19) (aa25-33) (Hanada et al., JCI Insight, 4 (2019), doi:10.1172/jci.insight.124405). The tumor cells were allowed to establish for ten days, and tumor-bearing mice received 6 Gy total body irradiation before the injection of the T cells. One day later, the animals were treated with FACS sorted DN (CD39⁻ CD69⁻) and DP (CD39⁺CD69⁺) in two doses (3e5, 5e5 per mouse). Pmel T cells that were *in vitro* activated and expanded for ten days at the indicated doses. Control mice receiving 1X PBS were included as controls. All treated animals received daily injections of 12 µg IL-2 i.p. for three days. All tumor measurements were performed double-blinded by an independent investigator.

### ATAC sequencing analysis

ATAC-seq fastq files were aligned to genome build 38 using bowtie2 with --very-sensitive flag. Aligned bam files had duplicates removed using Picard's mark duplicates tool. Mitochondrial reads were removed and peaks were called using Homer v4.11.1 under the peak finding style DNase setting. Bedtools was used to merge overlapping peaks and record counts of 5' end of reads within each peak for each sample. This count matrix was used as input to EdgeR to identify differential regions using estimateGLMTagwiseDisp function. A likelihood ratio test was then performed using glmFit and glmLRT. Peaks were identified as significant if their FDR q-value as <= 0.01.

Transcription Factor Motif analysis: All human Transcription factor Motifs were downloaded from CIS-BP database on 07-07-2020. GOMER (Sade-Feldman et al., Cell, 176: 404 (2019); Liu et al., Genome Res., 16: 1517-1528 (2006)) was used to generate a binding score for all differential peaks for each motif. Python's scipy.stats hypergeom module was used to calculate a minimum hypergeometric mean test over background peak rate (nonsignificant peaks used to generate background rate for each motif) for each motif for both double positive and double negative peaks using the top N up to 3000 scoring peaks. The p-values were then corrected by Bejamini-Hochberg FDR using python's statsmodels.sandbox.stats.multicomp multipletests module counting each test as independent.

### EXAMPLE 1

This example demonstrates that a CD39⁻CD69⁻ T cell phenotype in administered T cells is associated with a positive response to ACT.

The phenotypic differences that could distinguish ACT infusion products (I.P.) administered to patients who had complete response to therapy (complete responders, CRs, N = 24) from those whose disease progressed following ACT (non-responders, NRs, N = 30) (Fig. 1A) was compared. These ACT I.P. were from a cohort of stage IV metastatic melanoma patients who had been treated with autologous *in vitro*-expanded TILs and had not experienced prior immunotherapies in the form of genetically engineered T cell therapy or anti-PD-1 blockade (Goff et al., J. Clin. Oncol., 34: 2389-2397 (2016); Dudley et al., Clin. Cancer Res., 16: 6122-6131 (2010)).

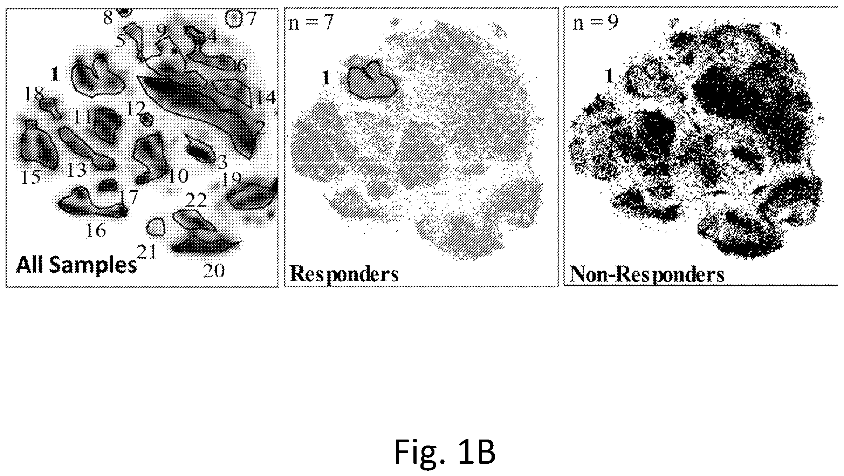

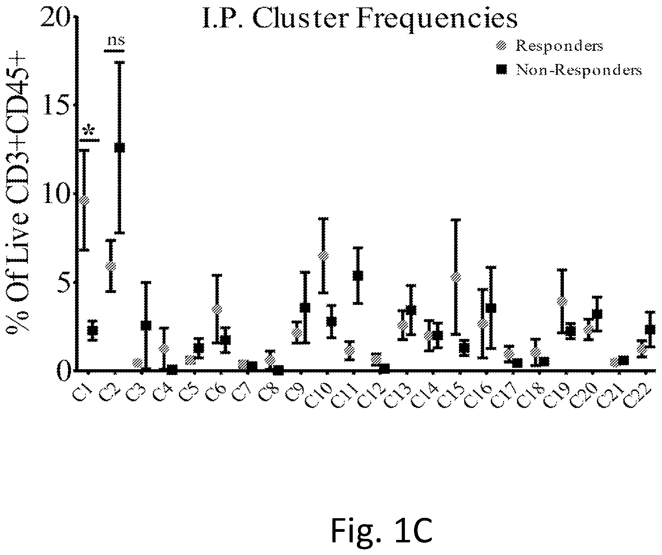

Initial single-cell analysis of a discovery set of 4.8 million I.P. TILs from 7 CRs and 9 NRs by mass cytometry (CyTOF) revealed heterogeneous expression of 34 cell surface markers. Supervised analysis of TIL CyTOF profiles identified clusters that appeared prevalent in either CRs or NRs (Fig. 1B). Cluster 1, which was four-fold more abundant in CR I.P. relative to NR I.P. (corrected *P* = 0.0264, Fig. 1C), corresponded to CD8⁺ T cells with abundant CD44, CD27, and CD28, and low expression of TIM3, characterized in prior studies as memory-like (Sade-Feldman et al., Cell, 176: 404 (2019)) and stem-like (Jansen et al., Nature, 576: 465-470 (2019)) T cells. Notably, cluster 1 also had low expression of inhibitory marker CD39 and T cell activation marker CD69. Machine learning-based unsupervised clustering of T cell activation/exhaustion states to classify patients according to their clinical outcome further confirmed CD69 and CD39 expression as two significant features of most clinical relevance (Fig. 5A-5C). Of note, frequency of cluster 2, with high expression of CD39 and CD69 but lower levels of CD44, CD27, and CD28, trended higher in NR I.P. than in CR I.P., although this difference was not statistically significant (Fig. 1C).

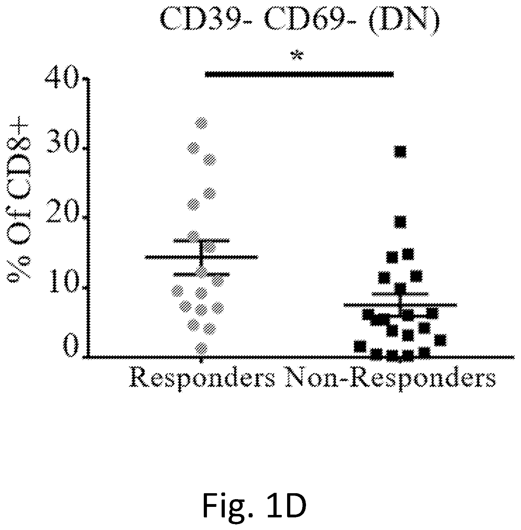

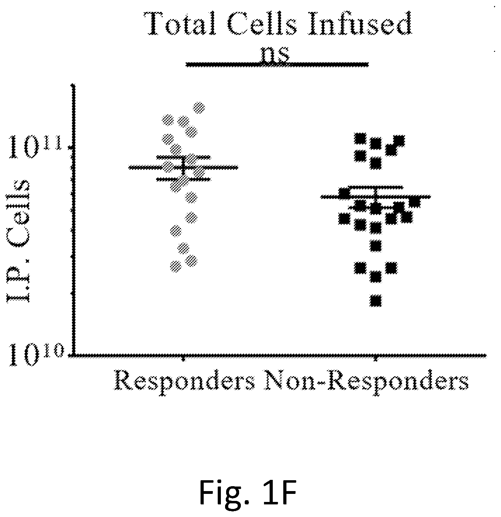

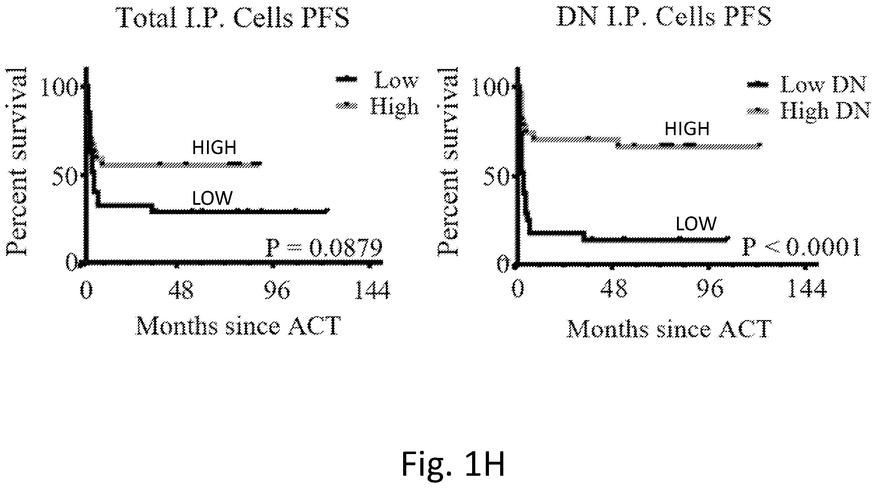

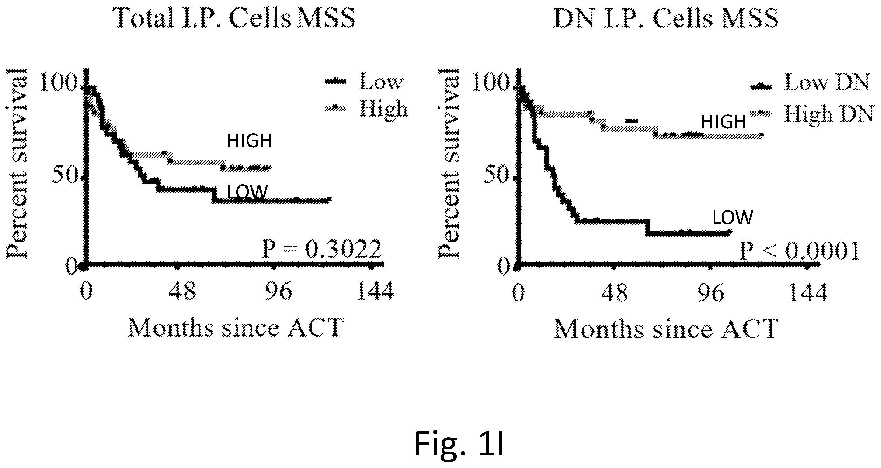

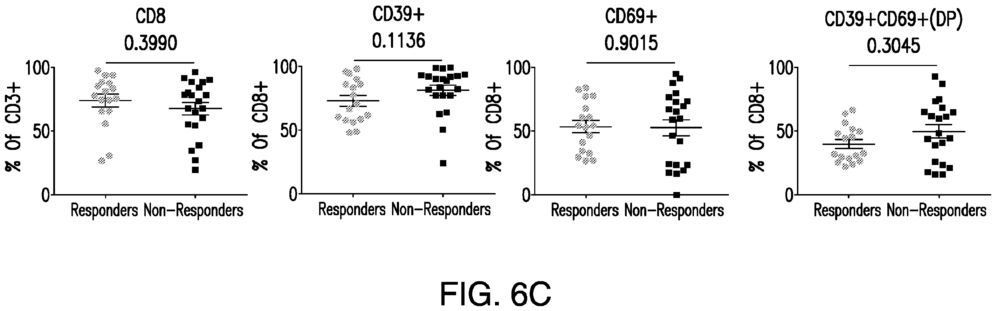

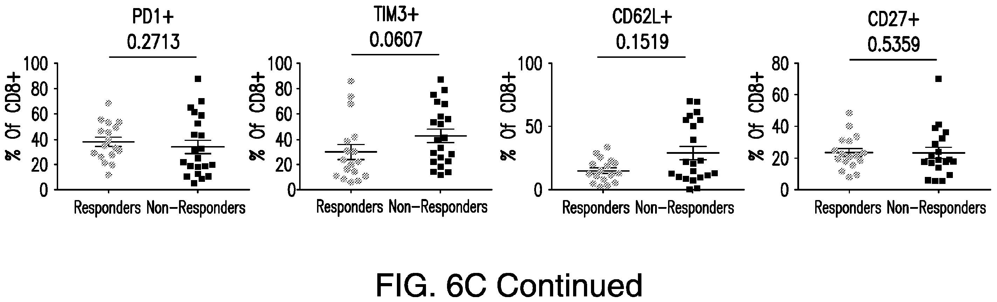

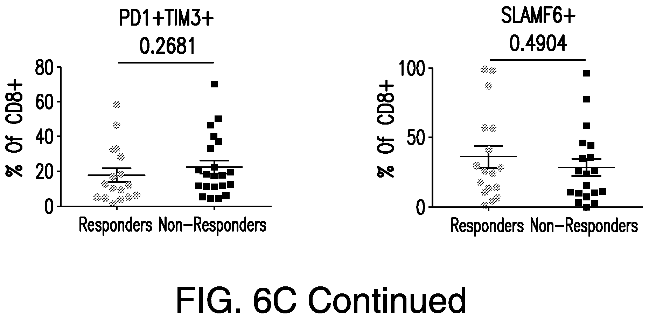

Flow cytometric analysis of the 16 discovery samples defining CD8⁺ CD39⁻CD69⁻ cells as members of cluster 1 recapitulated mass cytometry data with high confidence (Fig. 6A-6B). An evaluation of an independent set of 38 I.P. (CRs n =17, NRs n=21) by multiparameter flow cytometry revealed that the frequency of the CD8⁺CD39⁻CD69⁻ TIL population was 2.5-fold higher in CR I.P. relative to NR I.P. (P = 0.0096, % of CD3) supporting the association between this subset and ACT response (Fig. 1D-E). While the total number of infused T cells did not differ significantly between CR and NR I.P. in this set of 38 patient samples, the total number of infused CD8⁺CD39⁻CD69⁻ cells was four-fold higher in CR I.P. than NR I.P. (P = 0.0031, Fig. 1F-G). Querying individual markers, there was a modest trend towards an association between CD8+ TIM3+ TILs and non-response to ACT (Fig. 6C). Analyzing patient survival from all 54 patients, it was found that whereas the absolute number of infused CD39⁺CD69⁺ T cells, which comprised most of the CD8⁺ TILs in patient infusion products (Fig. 6C), did not significantly affect melanoma-specific survival (MSS) or progression-free survival (PFS) in this cohort (Figs. 7A-7C), higher numbers of CD8⁺CD39-CD69- cells in the I.P. were significantly associated with improved progression-free survival (PFS, P < 0.0001, HR = 0.255, 95% confidence interval (CI) 0.1257 to 0.5186, Fig. 1H) and melanoma-specific survival (MSS, P < 0.0001, HR= 0.217, 95% CI 0.101 to 0.463, Fig. 1I) in a dose-dependent manner (Fig. 7A-7C tertile analysis). Furthermore, the ratio of CD8⁺CD39⁻CD69⁻ to CD8⁺CD39⁺CD69⁺ cells in the infusion products significantly affected MSS and PFS, suggesting that the ACT responses in this cohort were associated with the infusion of higher numbers of CD8⁺CD39⁻CD69⁻ cells and not fewer CD8⁺CD39⁺CD69⁺ TILs.

### EXAMPLE 2

This example demonstrates that positive response-associated CD39⁻CD69⁻ TILs are in a progenitor memory stem-like state while CD39⁺CD69⁺ TILs are in a terminally differentiated state.

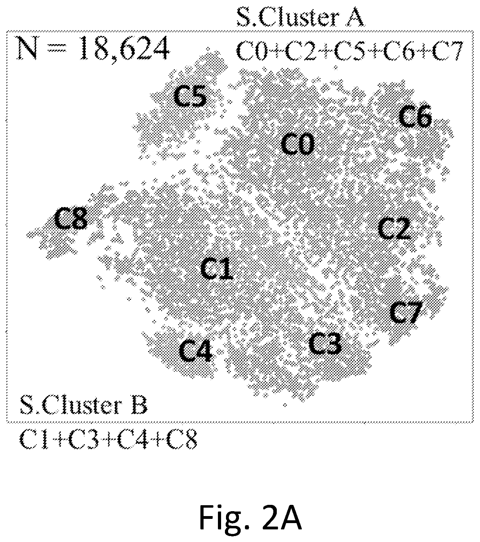

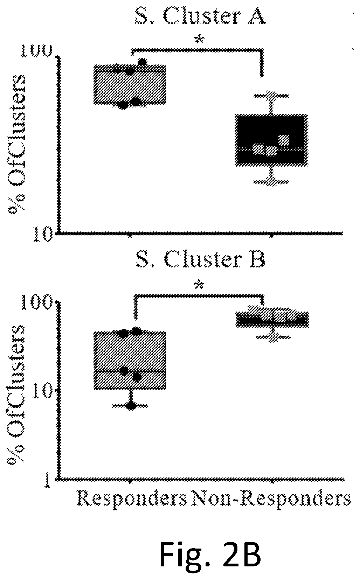

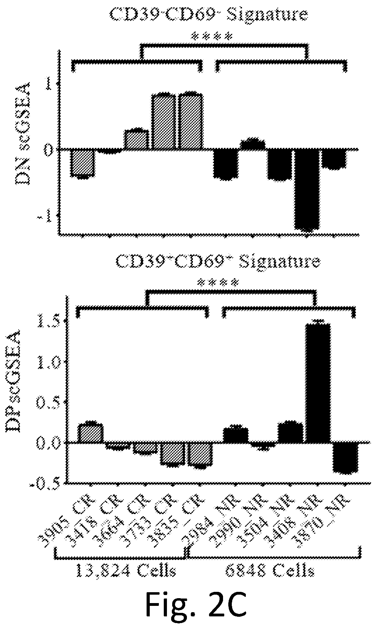

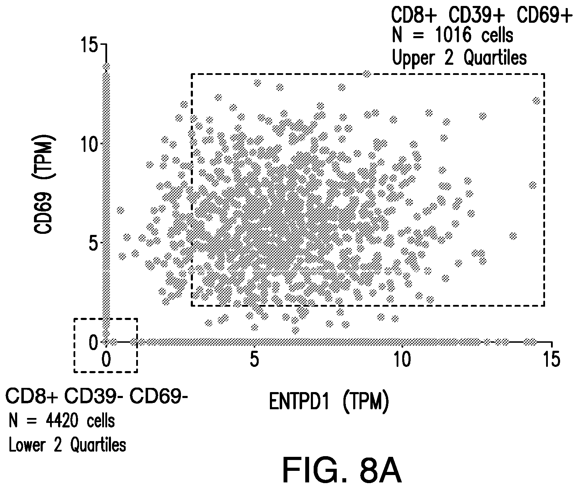

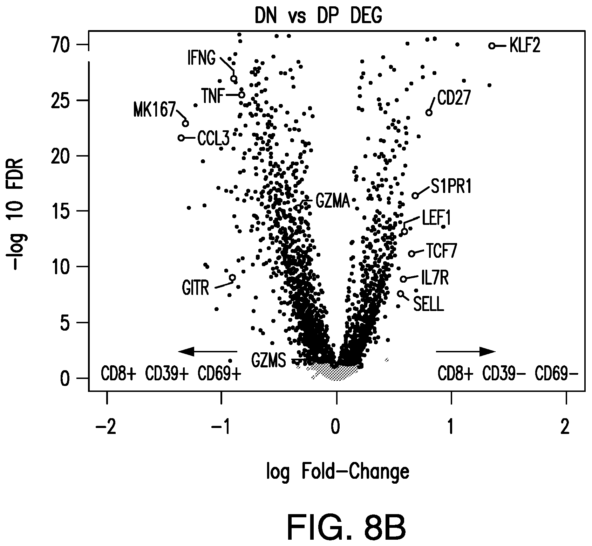

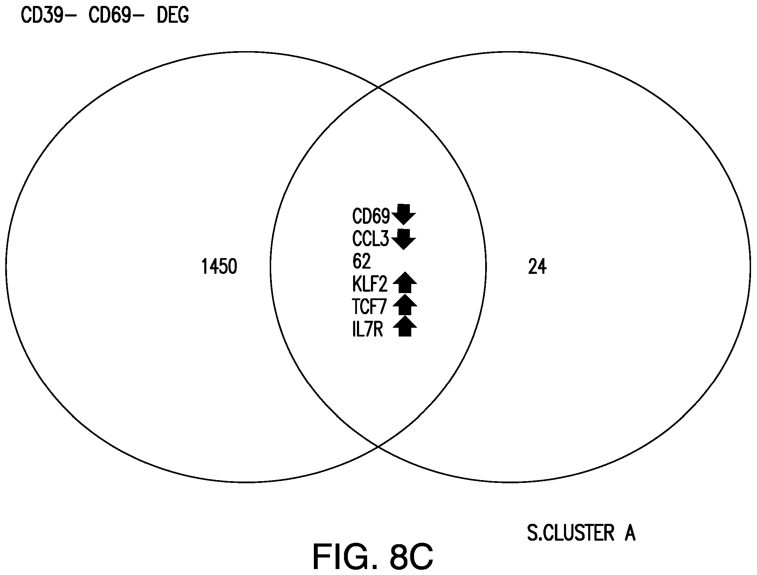

To further explore the potential significance of CD8⁺CD39⁻CD69⁻ TIL (cluster 1, double-negative: DN) and the I.P. predominant CD8⁺CD39⁺CD69⁺TII, (cluster 2, doublepositive: DP), the transcriptome profile of these two subsets was evaluated (Fig. 8A). DN TILs had increased expression of quiescent T-stem-cell markers *KLF2, TCF7, SIPRI, LEF1,* IL7R, CD27, and SELL (CD62L) while DP TILs expressed CD38, *MK167,* GITR, GZMA, TNF, and IFNG found in differentiated, activated T cells (Fig. 8B, Table 3). Unsupervised clustering of 20,672 CD8+ TILs by single- cell transcriptome analysis (scRNA) from 10 patient I.P. (5 CRs, 5 NRs) defined 8 clusters (Fig. 2A). Analysis of the distribution of TILs from CR and NR I.P. indicated that they segregated into a two-cluster solution (Sade-Feldman et al., Cell, 176: 404 (2019)) defined by S.Cluster.A (Super Cluster A, comprising clusters C0, C2, C5, C6, C7) and S.Cluster.B (Super ClusterB, comprising clusters C1, C3, C4, C8). Responder TILs were distributed more in S.Cluster.A (P = 0.0317), while S.Cluster.B largely comprised non-responder TILs (P = 0.03, Fig. 2B). TILs in S.Cluster.A were enriched for DN gene signature (81% overlap, Fig. 8C) while TILs in S.Cluster.B were enriched for DP gene signature. Consistent with these analyses, CR TILs and NR TILs scored by single cell gene set enrichment analysis (scGSEA) of DN and DP gene signatures indicated that CR TILs had higher DN scores on average while NR TILs had higher DP scores (*P* < 4.5 x 10⁻¹² for both, Fig. 2C).

**TABLE 3**

| **CD39⁻ CD69⁻ Signature** | **CD39⁺ CD69⁺ Signature** |
|---|---|
| KLF2⁺ | ENTPD1⁺ |
| AQP3⁺ | CD69⁺ |
| CLIC3⁺ | RRM2⁺ |
| LINC00861⁺ | TYMS⁺ |
| CD8B⁺ | CD8B⁺ |
| FAM65B⁺ | KIAA0101⁺ |
| FGFBP2⁺ | CCL3⁺ |
| RASA3⁺ | TUBA1B⁺ |
| C10orf54⁺ | MKI67⁺ |
| SAMD3⁺ | UBE2C⁺ |
| CD27⁺ | HLA-DQA2⁺ |
| FAIM3⁺ | CDK1⁺ |
| S100A10⁺ | STMN1⁺ |
| LYAR⁺ | IL5⁺ |
| BIN1⁺ | NDFIP2⁺ |
| CTD-3184A7.4⁺ | DUSP6⁺ |
| KANSL1-AS1⁺ | CSF2⁺ |
| UBXN11⁺ | XCL1⁺ |
| ZFP36L2⁺ | UBE2S⁺ |
| PPP2R5C⁺ | TOP2A⁺ |
| LITAF⁺ | HIST1H4C⁺ |
| ZNF683⁺ | AIF1⁺ |
| S1PR1⁺ | TUBB⁺ |
| TCF7⁺ | CLSPN⁺ |
| GIMAP4⁺ | IF127⁺ |
| MYC⁺ | HMMR⁺ |
| S1PR4⁺ | EGR1⁺ |
| CD52⁺ | KPNA2⁺ |
| STK38⁺ | TPX2⁺ |
| NEATI⁺ | ZWINT⁺ |
| LGALS3⁺ | TK1⁺ |
| GZMM⁺ | CD38⁺ |
| SORL1⁺ | UBE2T⁺ |
| AL592183.1⁺ | NUSAP1⁺ |
| R3HDM4⁺ | PCNA⁺ |
| MIAT⁺ | CENPF⁺ |
| ANXA4⁺ | H2AFX⁺ |
| LEF1⁺ | CRTAM⁺ |
| TSC22D3⁺ | ASPM⁺ |
| IL7R⁺ | GTSE1⁺ |
| P2RY8⁺ | TNFRSF18⁺ |
| PLEC⁺ | MYBL2⁺ |
| ERN1⁺ | CKS1B⁺ |
| TSPAN32⁺ | SPC25⁺ |
| S100A4⁺ | CDKN3⁺ |
| SELL⁺ | IFNG⁺ |
| RASGRP2⁺ | AURKA⁺ |
| GADD45B⁺ | FEN1⁺ |
| C11orf21⁺ | GINS2⁺ |
| PIK3IP1⁺ | CD40LG⁺ |
| RNASET2⁺ | AURKB⁺ |
| S100A6⁺ | NCAPG⁺ |
| CCDC109B⁺ | TNFSF10⁺ |
| RAMP1⁺ | PLK1⁺ |
| RP11-640M9.1⁺ | IL2RA⁺ |
| GPR155⁺ | AGPAT9⁺ |
| TXNIP⁺ | CCND2⁺ |
| CDC25B⁺ | CENPE⁺ |
| ISG20⁺ | CDC20⁺ |
| TRADD⁺ | CCNB1⁺ |
| CRBN⁺ | SMC2⁺ |
| GIMAP7⁺ | BIRC5⁺ |
| C16orf54⁺ | TUBA1C⁺ |
| FBXL8⁺ | CCNA2⁺ |
| ODF2L⁺ | CKS2⁺ |
| LDLRAP1⁺ | BIRC3⁺ |
| MXI1⁺ | KRT7⁺ |
| RCBTB2⁺ | CENPW⁺ |
| HSD17B11⁺ | MCM7⁺ |
| CLEC2B⁺ | ADAM 19⁺ |
| EMP3⁺ | TNF⁺ |
| SH3BP5⁺ | C15orf48⁺ |
| RP11-539L10.2⁺ | DLGAP5⁺ |
| MIR142⁺ | DUSP5⁺ |
| CLUAP1⁺ | KIF11⁺ |
| YPEL3⁺ | CCNB2⁺ |
| SLCO3A1⁺ | ACOT7⁺ |
| NOSIP⁺ | CASC5⁺ |
| IL10RA⁺ | PBK⁺ |
| CD55⁺ | CDCA3⁺ |
| RP11-395B7.4' | TESC⁺ |
| MED15⁺ | DUT⁺ |
| ATM⁺ | CENPM⁺ |
| KLHL24⁺ | KIF23⁺ |
| ZNF276⁺ | ATAD2⁺ |
| ANXA1⁺ | SGOL2⁺ |
| PLP2⁺ | TPM4⁺ |
| AHNAK⁺ | AGTRAP⁺ |
| DDI2⁺ | MAD2L1⁺ |
| VNN2⁺ | NDC80⁺ |
| VCL⁺ | NUDT1⁺ |
| EPB41⁺ | CDT1⁺ |
| DND1⁺ | CENPA⁺ |
| MAPKAPK5-AS1⁺ | RPL39L⁺ |
| CDC42SE1⁺ | SHCBP1⁺ |
| PXN⁺ | CDCA8⁺ |
| SYNJ2⁺ | CEP55⁺ |
| SIGIRR⁺ | BRCA1⁺ |
| PDE6G⁺ | ORC6⁺ |
| SLAMF6⁺ | CISH⁺ |
| TIMP1⁺ | MX1⁺ |

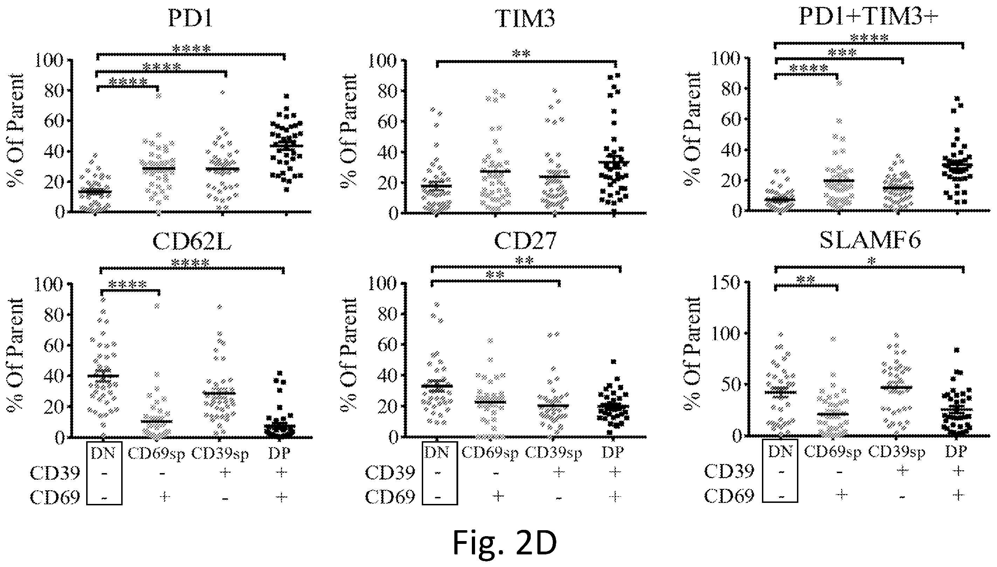

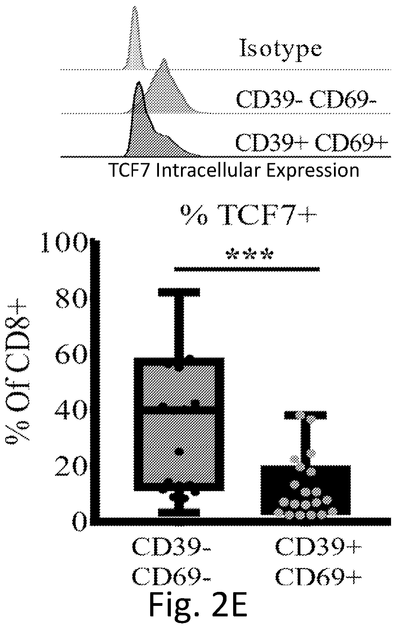

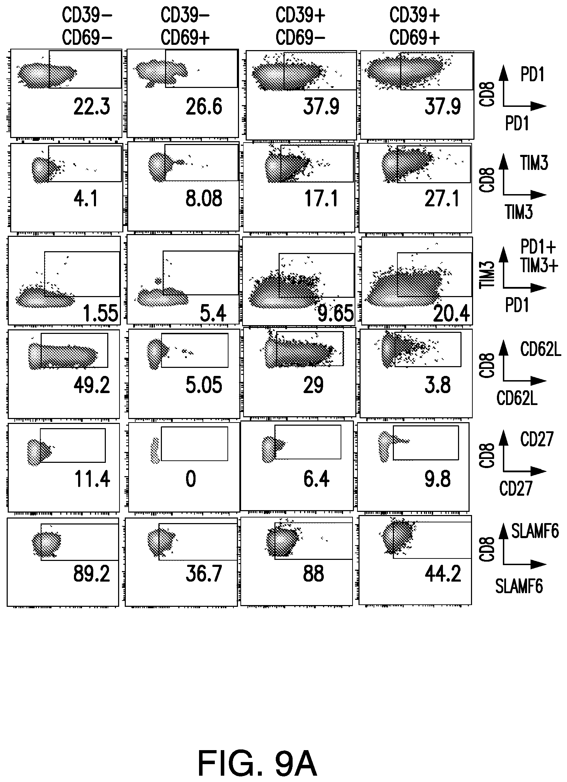

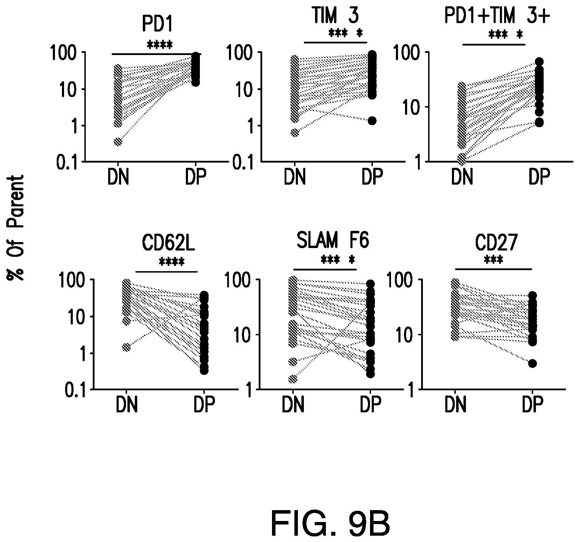

Cell surface expression of inhibitory and memory markers was analyzed by flow cytometry within CD39/CD69 TIL subsets from the validation samples (n = 38 I.P., Fig. 2D). Lower cell surface expression and coexpression of the exhaustion markers PD-1 and TIM3 in the DN subset relative to single positive (SP, CD39⁺CD69⁻ and CD39⁻CD69⁺) and DP subsets was observed (Fig. 2D, Fig. 9A-9B). Conversely, expression of memory markers CD62L and CD27 and progenitor marker SLAMF6 were increased in DN relative to DP subsets with variable expression within SP populations (Fig. 2D,Fig. 9A-9B). Protein expression of memory stem-like T cell marker TCF7 was five-fold higher in DN TILs relative to DP TILs *(P* = 0.0002, n=18 I.P., Fig. 2E) consistent with the scRNA analysis. Although DN TILs in infusion products expressed higher levels of both SLAMF6 and TCF7 transcripts, TILs coexpressing TCF7 and SLAMF6 were not significantly enriched within DN subsets, and addition of SLAMF6 to DN as a surface marker was comparable to DN alone in delineating TILs associated with survival benefit in this patient subset. By contrast, DP TILs had significantly higher transcript and protein expression of the effector and exhaustionassociated transcription factor TOX relative to DN TILs. Notably, although the transcriptional profile of DN TILs resembled that of peripheral blood stem cell memory (SCM) T cells (Gattinoni et al., Nat. Med., 17: 1290-1297 (2011); Lugli et al., J. Clin. Invest., 123: 594-599 (2013)), cell surface expression of the canonical SCM markers CD95 and CCR7 was not associated with the DN phenotype in patient TIL infusion products.

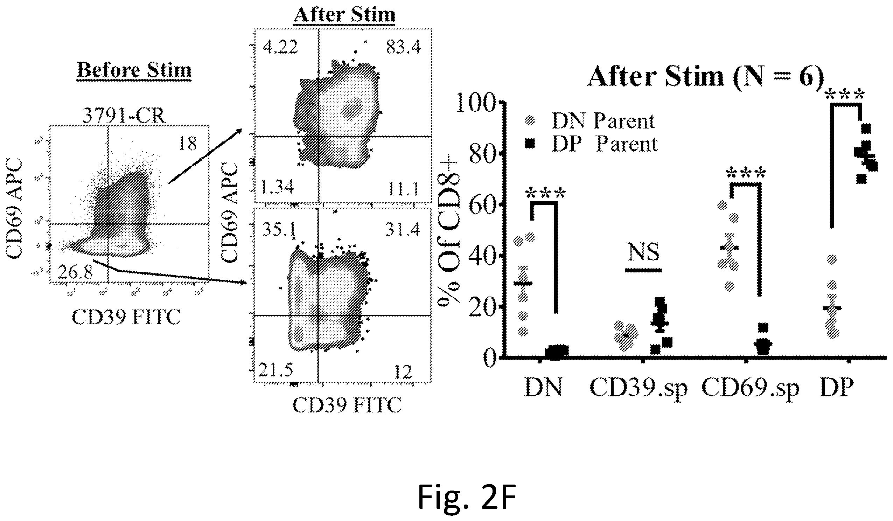

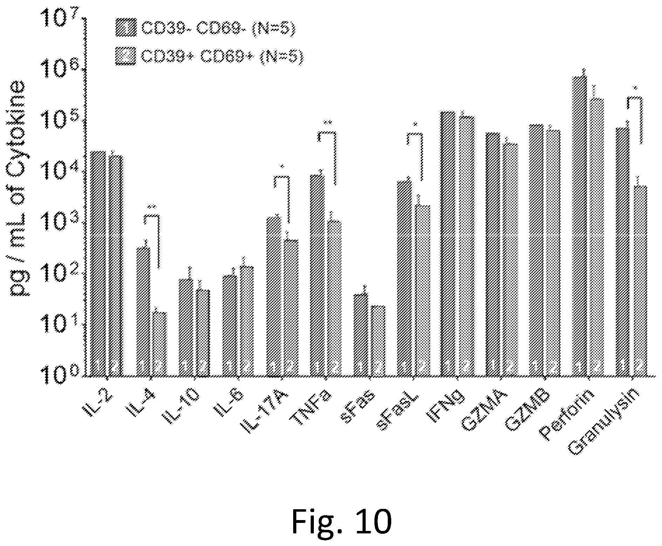

To assess if CD39⁻CD69⁻ I.P. TILs were stem-like T cells, the DN and DP subsets were isolated and stimulated using plate-bound anti-CD3/anti-CD28. Upon T cell receptor (TCR) stimulation, DN TILs underwent self-renewal, and gave rise to both single positive and CD39⁺CD69⁺ DP daughter populations, while stimulated DP TILs largely remained in the same DP state, indicating a DN-progenitor state (n = 6 I.P., Fig. 2F). Time-course kinetics of the daughter populations indicated that essentially all DN TILs underwent CD69 activation, but a subset of DN TILs (median = 17%) rested back to the CD39⁻CD69⁻ stem-like state 5 days after initial stimulation. By contrast, both CD39⁺ parent populations (CD39 SP and DP) appeared to terminally differentiate into a stable CD39⁺CD69⁺ state after TCR stimulation. In addition, while DP and DN TILs secreted similar levels of IL-2, IFNγ, GZMA, GZMB, and perforin in response to polyclonal stimulation, DN TIL stimulation resulted in higher levels of secreted IL-17A, TNF-a, IL-4, sFasL and granulysin than DP TILs (Fig. 10). Taken together, these results indicate that ACT response-associated CD39⁻CD69⁻ TILs are in a progenitor memory stem-like state capable of differentiation into other subsets, whereas the infusion product-dominant CD39⁺CD69⁺ TILs are in a terminally differentiated state.

To understand the key regulators of these two TIL states in the infusion products, matched DN and DP TILs were isolated from three patient infusion products and their epigenetic profiles were analyzed using ATAC (assay for transposase accessible chromatin) sequencing. The presence of 4314 human transcription factor (TF) motifs in open chromatin regions of DN and DP TILs was queried. Open chromatin regions within stem-like DN TILs were enriched for numerous binding sites for SOX and C2H2-ZF family TFs including *KLF4, TCF7, LEF 1,* and *TCF7L1* consistent with those of less differentiated human T cells (Sade-Feldman et al., Cell, 176: 404 (2019); Lynn et al., Nature, 576: 293-300 (2019)). By contrast, chromatin regions within DP TILs were extensively enriched for multiple binding motifs of bZIP TFs *FOSL1, FOS, JUNB,* and *JUND*, indicating that epigenetic imprinting may be involved in maintaining the state of terminal differentiation resulting from chronic tumor cell activation (Lynn et al., Nature, 576: 293-300 (2019); Blank et al., Nat. Rev. Immunol., 19: 665-674 (2019)).

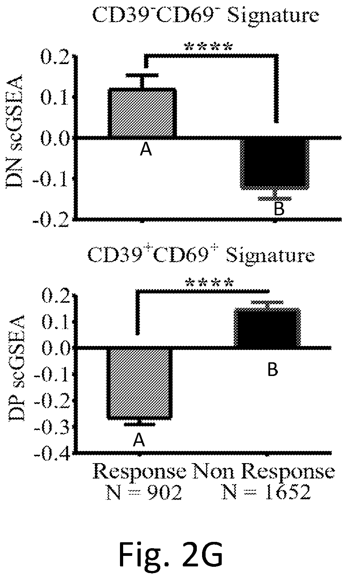

To understand if TIL states in the I.P. corresponded to those within fresh tumor, the transcriptome profiles of 6001 CD8⁺ T cells from a previous melanoma ICB response study (Sade-Feldman et al., Cell, 176: 404 (2019)) were re-analyzed using DN and DP gene signatures described above (Fig. 2G). It was found that prior to immune checkpoint therapy, TILs from ICB-responding lesions had higher CD39⁻CD69⁻ signature scores (*P* = 1.9 x 10⁻¹⁰) than non-responding lesions, while TILs from lesions that progressed during ICB had higher CD39⁺CD69⁺ signature scores (*P* < 2.2 x 10⁻¹⁶). Hierarchical clustering correlation analysis with T cell dysfunctional and progenitor gene signatures from other recent studies revealed that the signatures of CD39⁻CD69⁻ TILs were most similar to ICB response-associated memory and progenitor-exhausted TILs, while the gene signatures of CD39⁺CD69⁺ TILs were highly correlated with TOX⁺ and terminally-exhausted TILs associated with poor ICB response (Sade-Feldman et al., Cell, 176: 404 (2019); Miller et al., Nat. Immunol., 20: 326-336 (2019); Scott et al., Nature, 571: 270-274 (2019); Tirosh et al., Science, 352: 189-196 (2016)). A phenotypic fitness score was developed, defined as the difference between DN stem-like signature and DP differentiated signature (DN minus DP), and scRNA from ACT I.P. and melanoma ICB TILs were scored. Single-cell fitness scores re-confirmed that responder TILs have on average more stem-like fitness compared to non-responder TILs (Fig. 11A-11B). These data suggest that stem-like TILs found in ACT responder infusion products resemble those found in ICB responses.

### EXAMPLE 3

This example demonstrates that at least in the context of melanoma ACT, not all CD39⁻ TILs are bystander T cells, and that responders retain a proportion of tumor neoantigen-reactive TILs in a CD39⁻ stem-like state.

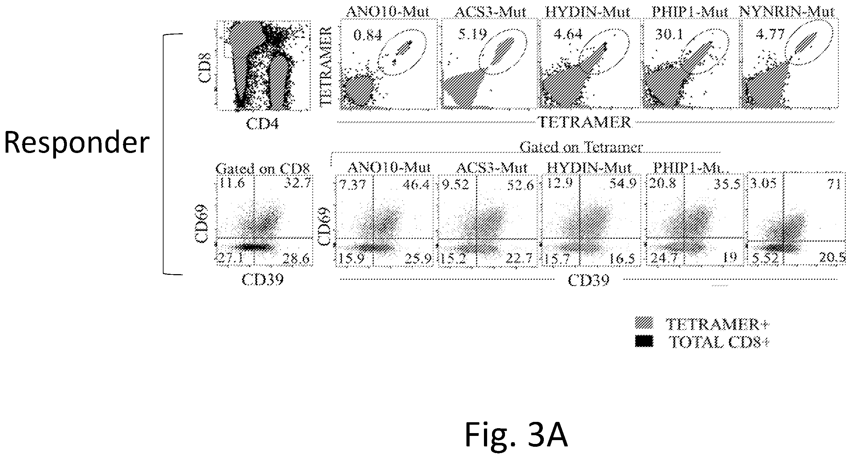

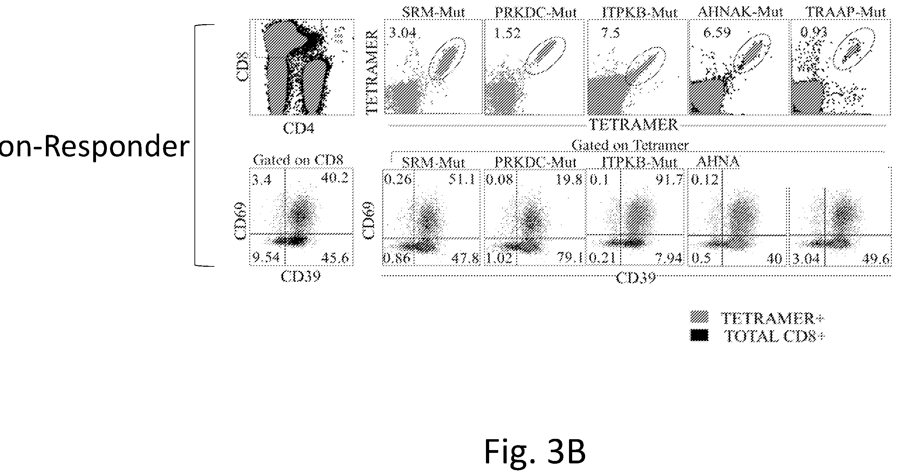

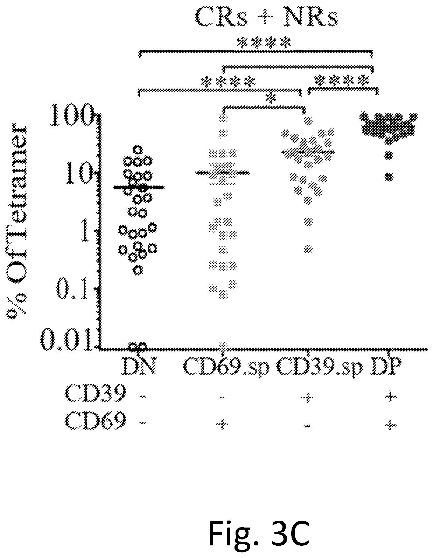

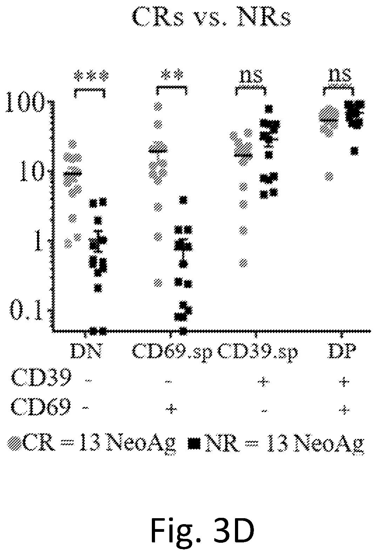

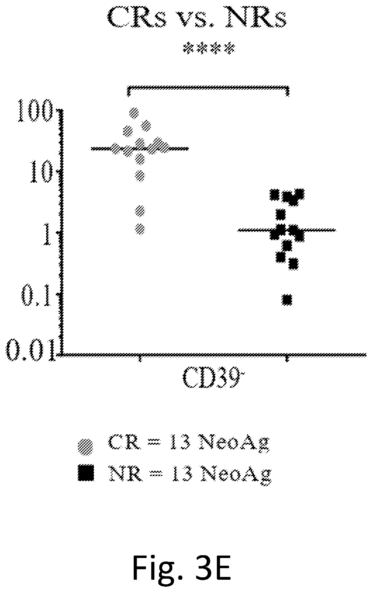

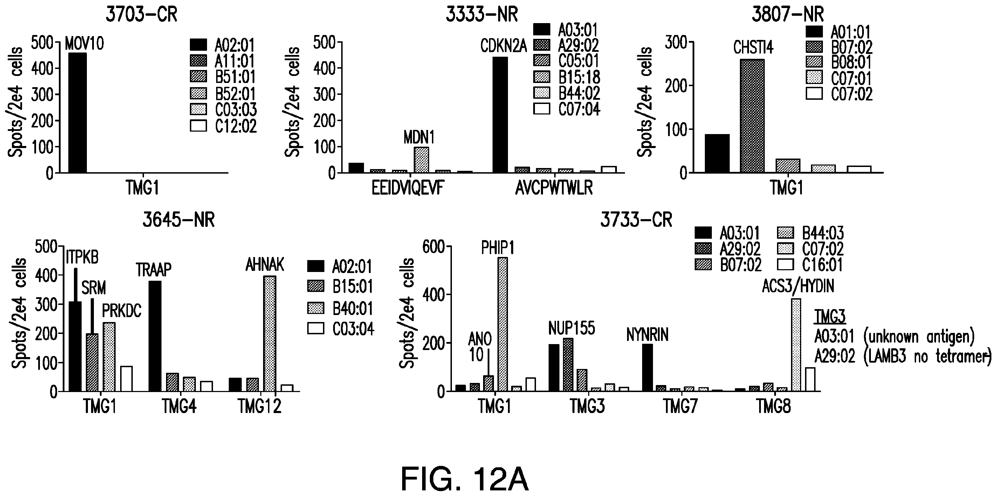

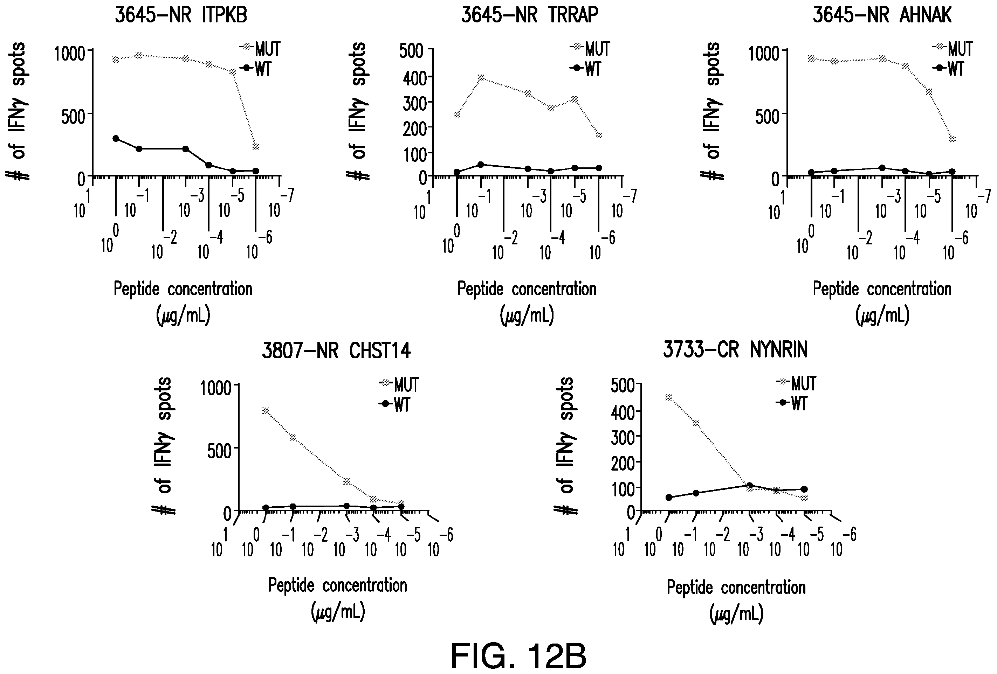

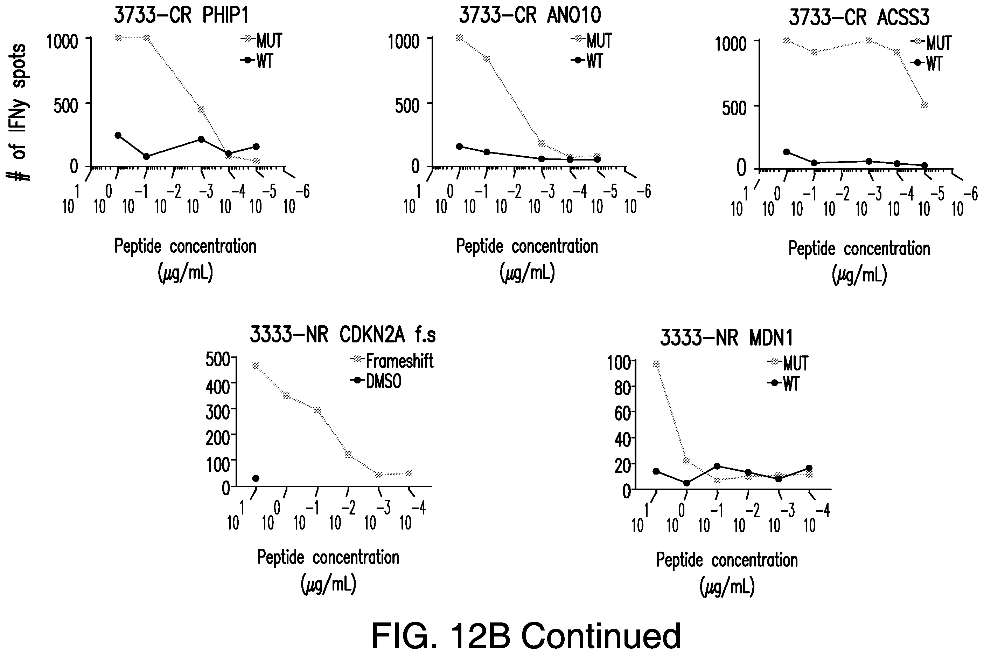

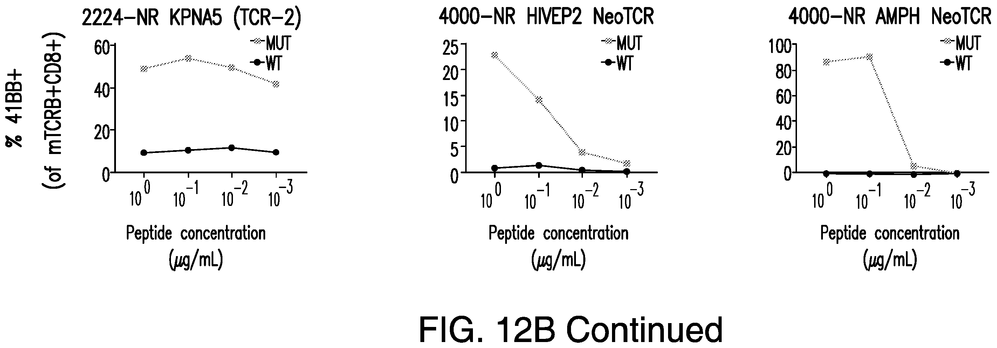

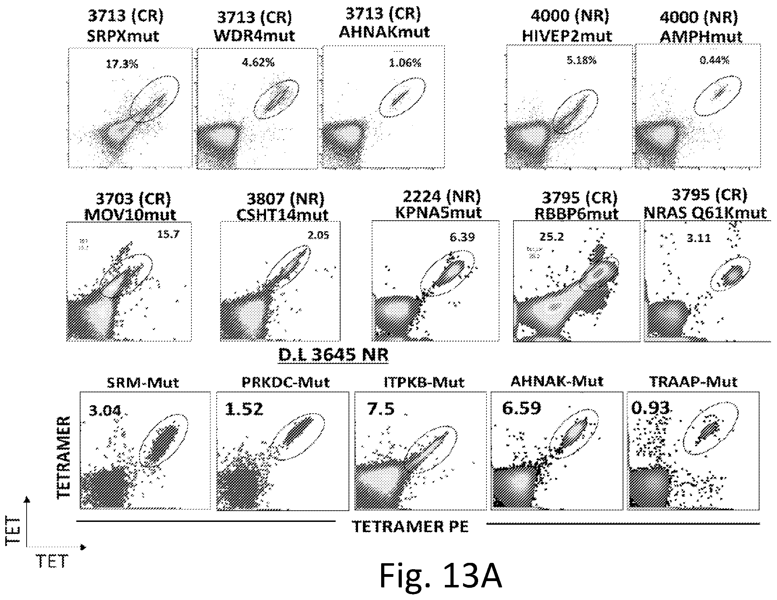

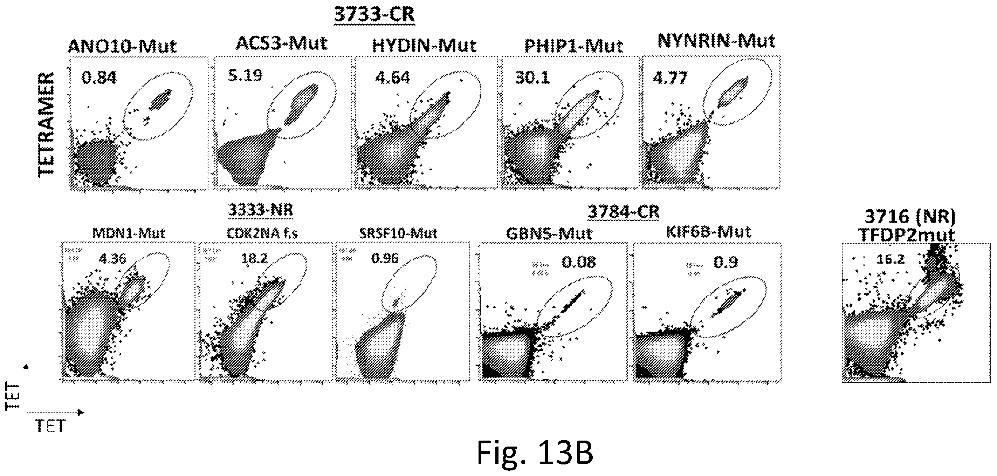

Previous studies on stem-like and differentiated human TIL subsets were performed on bulk TIL populations lacking specific analyses of anti-tumor T cells (Sade-Feldman et al., Cell, 176: 404 (2019); Jansen et al., Nature, 576: 465-470 (2019)). Given recent findings suggesting that neoantigen-specific TILs are nearly exclusively CD39⁺ whereas CD39⁻ TILs represent bystander cells (Simoni et al., Nature,557: 575-579 (2018); Duhen et al., Nat. Commun, 9: 2724 (2018)), it was sought to determine whether the CD39⁻ CD69⁻ stem-like state associated with ACT-response exist within the neoantigen-reactive T cell populations in I.P. Using the previously described tandem minigene neoantigen screening platform (Parkhurst et al., Cancer Discov., 9: 1022-1035 (2019); Tran et al., Science, 344: 641-645 (2014); Zacharakis et al., Nat. Med., 24: 724-730 (2018); Tran et al., N. Engl. J. Med., 375: 2255-2262 (2016)), 26 HLA class I restricted neoantigens from I.P. were defined for phenotypic evaluation (n = 11 patients, Fig. 12A-12B and 13). Stem-like and differentiated TIL subsets defined by their CD39/CD69 expression were readily detectable within HLA-neoantigen tetramer⁺ TILs as shown in representative CR and NR (Fig. 3A-3B). Combined analysis of all 26 neoantigen-specific T-cell populations indicated that they largely existed in the CD39⁺CD69⁺ terminally differentiated state, consistent with other studies reporting CD39 enrichment of neoantigen-specific T cells (median DP = 62.7%, Fig. 3C) (Simoni et al., Nature,557: 575-579 (2018)). Stratification of the data by ACT response status, however, revealed that the frequency of DN cells was significantly higher in neoantigen-specific TILs from CR I.P. (median = 8.8%) than in NR I.P. (median = 0.5%, *P =* 1.2 x 10⁻⁴, Fig. 3D). CD39 as a single marker indicated that CD39-negative (CD39⁻) neoantigen-reactive TILs were twenty-fold higher in CR TILs relative to NR TILs (median = 23.6%, *P* = 1.86 x 10⁻⁵, Fig. 3E). Adjusting for tumor-specific cells significantly enhanced these differences between CR and NR infusion products, wherein neoantigen-specific DN TILs were 23-fold higher in CRs (median = 2.3 × 10⁸ cells), and CD39⁻ TILs were 40.4-fold higher in CR (median = 1.06 × 10⁹ cells). No differences were found in the total number of neoantigen reactive TILs, or neoantigen-reactive CD39⁺ or DP TILs between CR and NR infusion products. These results indicate that at least in the context of melanoma ACT infusion products, not all CD39⁻ TILs are bystander T cells as previously reported, and that a subset of tumor neoantigen-reactive TILs exist in a CD39⁻ stem-like state in CRs. Furthermore, these data indicate that enrichment of tumor reactive neoantigen-specific TILs in differentiated subsets does not necessarily correspond to their frequency in stem-like states.

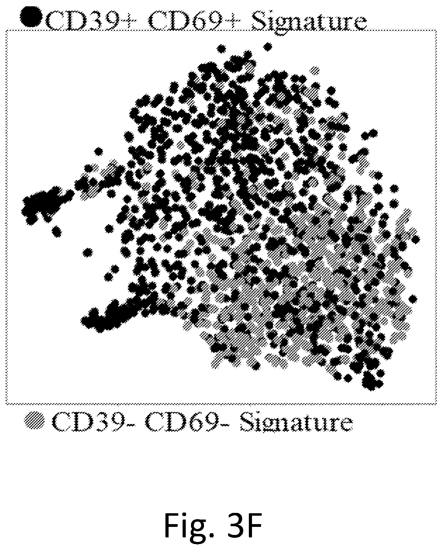

In order to explore the heterogeneity in stem-like and differentiated states of neoantigen- specific TILs at the single cell transcriptomic level, combined scRNA and scTCR sequencing were performed on I.P. from a CR (Pt. 3713) and an NR (Pt. 4000), with defined neoantigen-reactive TCRs (Fig. 3F-3L) (Prickett et al., Cancer Immunol. Res., 4: 669-678 (2016); Pasetto et al., Cancer Immunol Res., 4: 734-743 (2016); Parkhurst et al., Clin. Cancer Res., 23: 2491-2505 (2017); Cohen et al., J. Clin. Invest., 125: 3981-3991 (2015)).

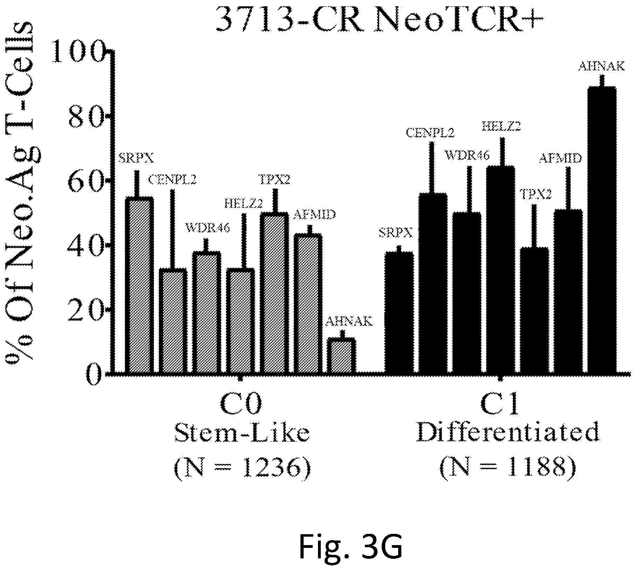

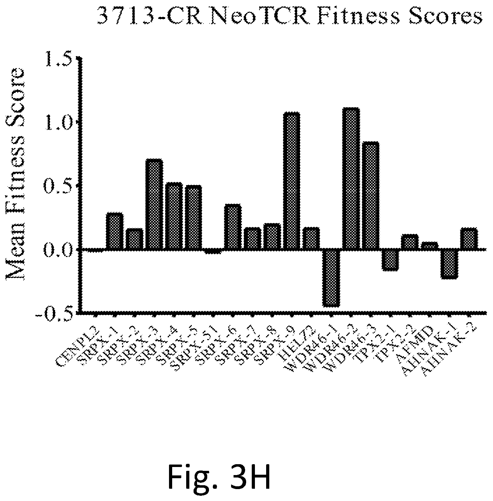

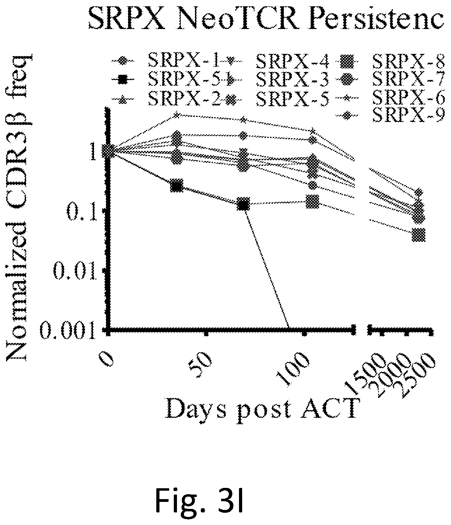

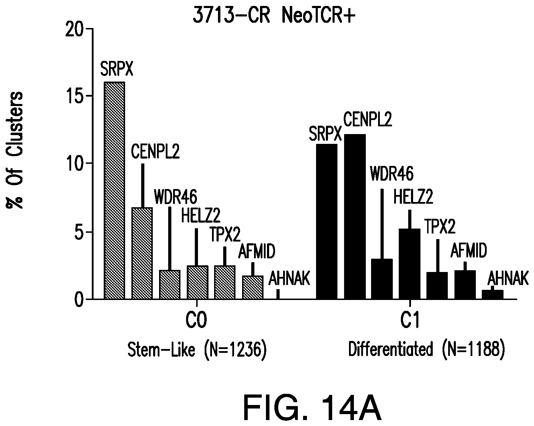

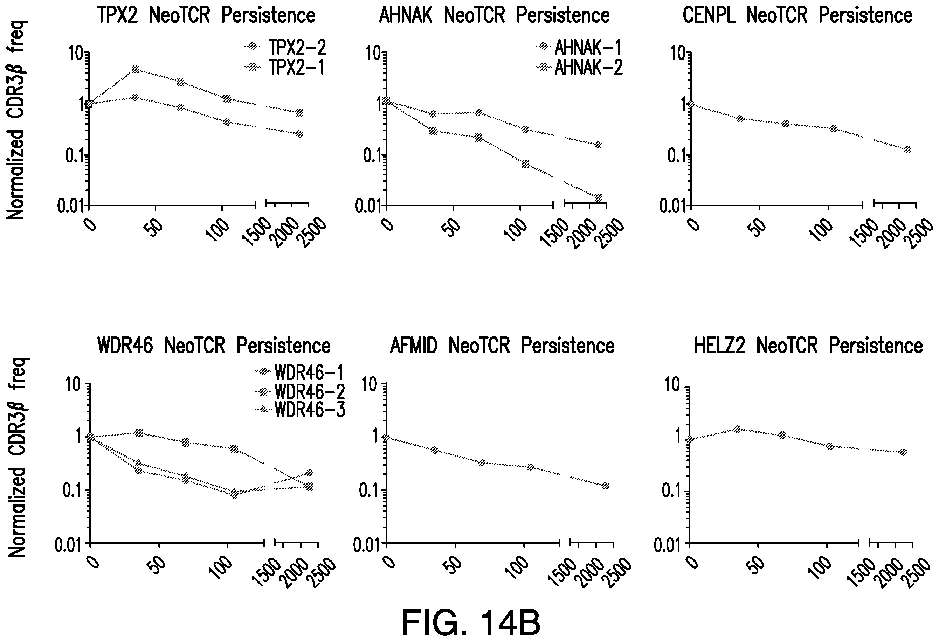

I.P. from responder Pt.3713 was dominated by two major clusters CO and C1, where CO represented the stem-like DN state and C1, the differentiated DP state (Fig. 3F). Projection of the 20 neoantigen-specific TCR⁺ (NeoTCR⁺) clonotypes showed a broad distribution among the two clusters (Fig. 3F-3G). The immunodominant SRPX mutation-specific NeoTCRs (SRPXₘᵤₜ) were enriched in cluster CO while other NeoTCRs varied in their prevalence within the two clusters (Fig. 3G). CDR3β sequencing of FACS-sorted DN and DP I.P. TILs confirmed this observation (Fig.14A). Finer analysis of SRPXₘᵤₜNeoTCR⁺ single cells indicated that 9/10 of these clonotypes (and 15/20 of all NeoTCR clonotypes) were enriched for positive phenotypic fitness scores (Fig. 3H). As prior studies have suggested that T cell-intrinsic differences potentially impact post-ACT clonotypic persistence (Gattinoni et al., Nat. Med., 17: 1290-1297 (2011); Rosenberg et al., Clin. Cancer Res., 17: 4550-4557 (2011)), the post-ACT peripheral blood of the patient 3713 was analyzed. TCR repertoire sequencing revealed that the majority of NeoTCR clonotypes (18/20) persisted up to 75 months consistent with the idea of TIL expansion from the progenitor stem-like state SRPXₘᵤₜ (Fig.3I, other NeoTCRs: Fig. 14B). Interestingly, among the 10 SRPXₘᵤₜ-NeoTCRs targeting the same neo-epitope, one clonotype (TCR-51) with the lowest phenotypic fitness score declined in frequency and became undetectable only 3 months post treatment (Fig. 3H-3I).

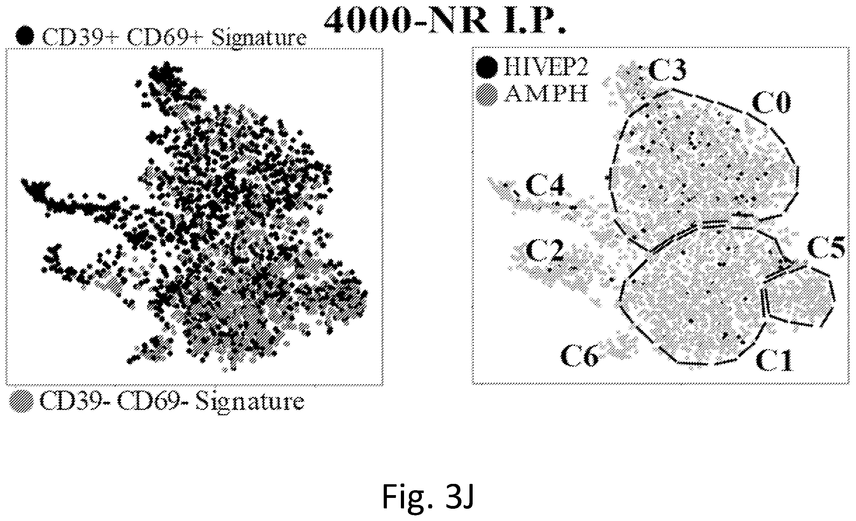

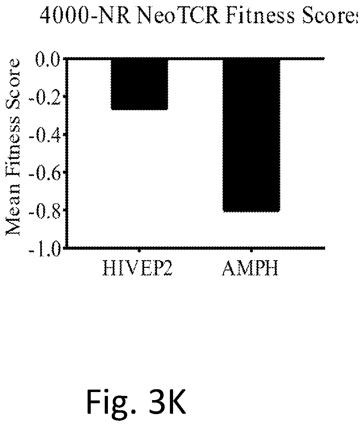

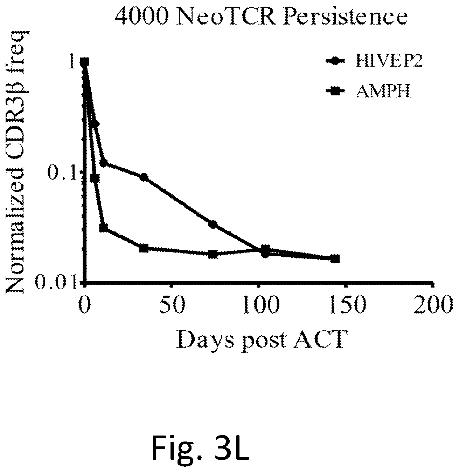

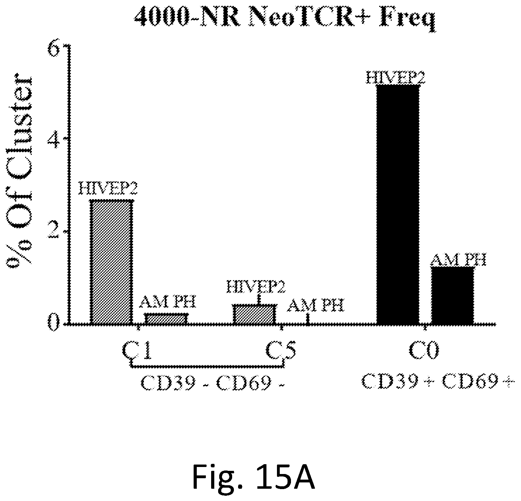

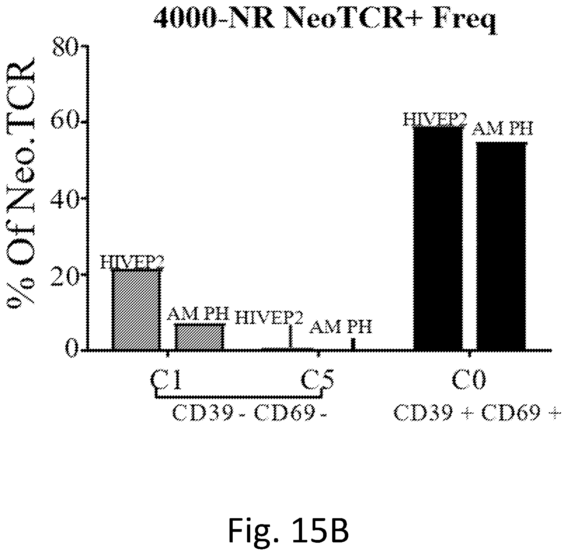

Combined scRNA and scTCR sequencing of the non-responder Pt. 4000 I.P. showed two major stem-like clusters (C1, C5) and one major differentiated cluster (CO) (Fig. 3J). However, NeoTCR+ cells (HIVEP2ₘᵤₜ, AMPHₘᵤₜ) were largely concentrated in the differentiated CO cluster (~60%, Fig. 3J, 15A-15B). scGSEA scores from Pt. 4000 indicated that both NeoTCR clonotypes had negative phenotypic fitness scores (Fig. 3K). In stark contrast to the 3713-CR persistent clonotypes, Pt. 4000 NeoTCRs did not persist post-ACT expansion; rather both infused NeoTCRs clonotypes rapidly declined in peripheral blood following treatment (Fig. 3L). These results support prior observations and other published reports that have linked cell therapy response to post-treatment TCR persistence (Rosenberg et al., Clin. Cancer Res., 17: 4550-4557 (2011); Zhou et al., Immunity, 33: 229-240 (2010); Kalos et al., Sci. Transl. Med., 3: 95ra73 (2011)).

### EXAMPLE 4

This example demonstrates that T cells with the CD39⁻CD69⁻ phenotype mediate anti-tumor activity and TCR persistence.

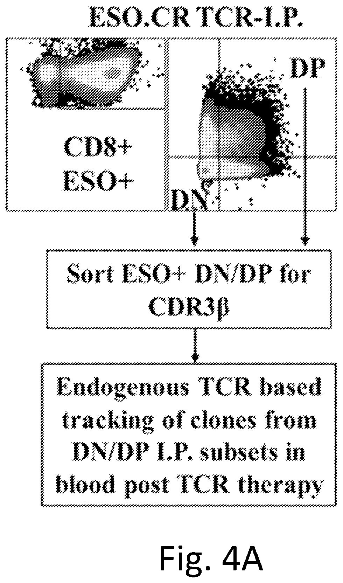

While the results described in Examples 1-3 indicated that T cell-intrinsic phenotypic differences are associated with persistence following ACT, additional factors such as TCR avidity against variable tumor antigens may also play a role in T cell persistence. To address this issue, an exploratory analysis of T cell persistence was performed in a metastatic synovial cell sarcoma patient who experienced a CR following the adoptive transfer of autologous PBMC transduced with a TCR targeting a single HLA-A*02:01-restricted NY-ESO-1 epitope. Endogenous human TCRβ sequences were used as barcodes to track stem-like DN clones and differentiated DP clones within the TCR- transduced population in post-treatment blood (Fig. 4A) (D'Angelo et al., Cancer Discov., 8: 944-957 (2018)). Fourteen out of the top 20 ESO TCR+I.P. clonotypes were enriched in stem-like DN state while six were enriched in the differentiated DP state. Stem-like clones demonstrated a gradual decrease in their frequency over a period of five years following TCR transfer, whereas the differentiated clones declined rapidly to minor frequencies in post-treatment blood of the patient (Fig. 4B). Among 1311 top clones, long-term persistent clones (detectable at 51 months post-ACT, n=790) were significantly enriched in the stem-like state in the I.P. when compared to poorly persistent clones (those undetectable at 7 days post treatment, n = 122) (*P* = 0.0035, Fig. 4C). These analyses indicate that intrinsic stem- like phenotypes can modulate the behavior of T cell clonotypes following ACT, although further studies in larger patient cohorts in TCR and CAR trials targeting other antigens are needed to test these hypotheses.

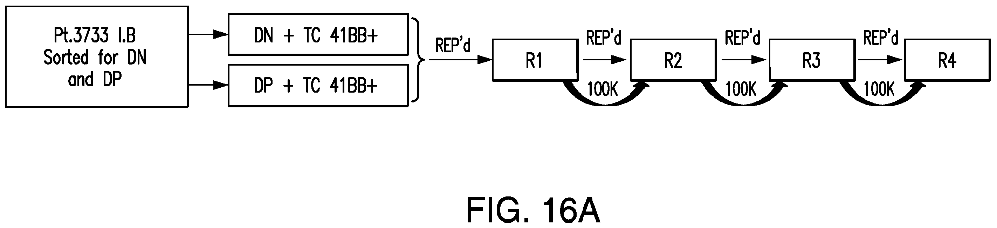

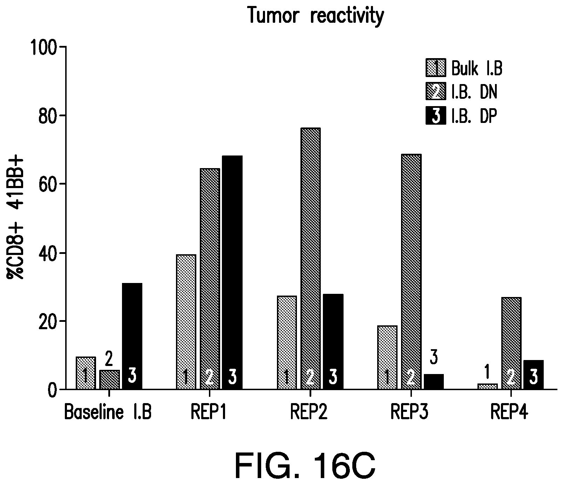

The findings suggested that treatment with CD39⁻CD69⁻ tumor-reactive TIL might result in superior tumor control. This hypothesis was first tested *in vitro* by isolating tumor-reactive DN and DP populations from a CR I.P. (Pt. 3733) by co-culture with autologous 3733-mel tumor line to select for tumor-reactive TILs, followed by multiple rounds of rapid expansion to evaluate their proliferative potential and tumor recognition (Fig. 16A). It was found that the expansion of the tumor-reactive stem-like DN subsets was approximately one thousand-fold higher than the differentiated DP subset (Fig. 16B). The stem-like DN subset maintained tumor recognition while the differentiated DP subset lost tumor reactivity following subsequent rounds of expansion (Fig. 16C-16D).

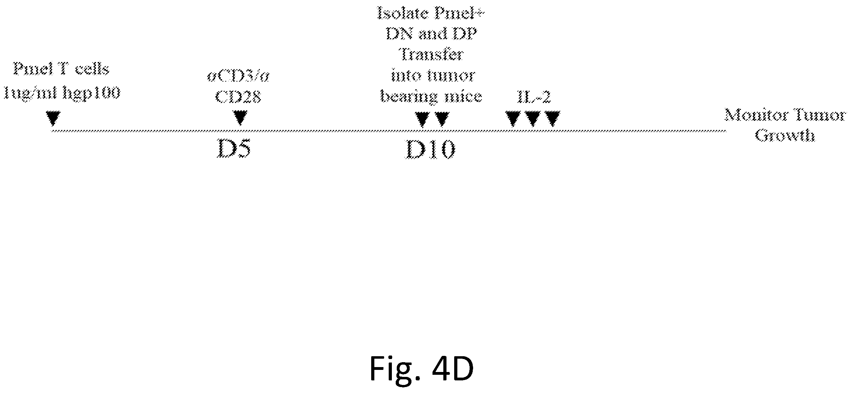

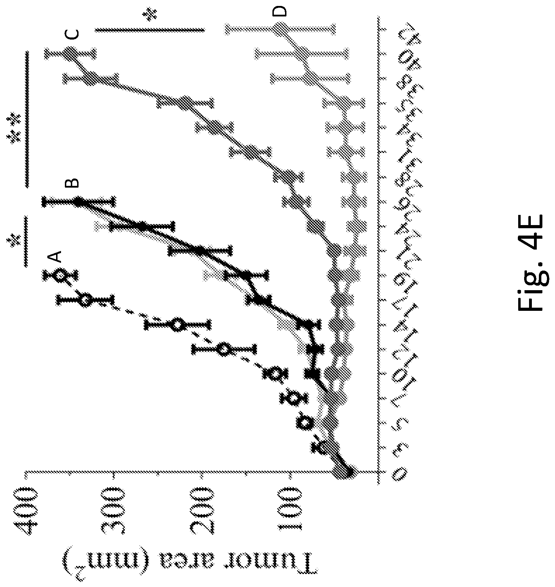

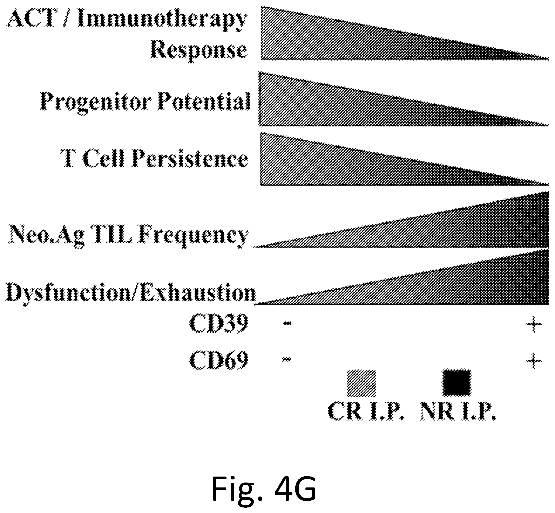

To assess the *in vivo* anti-tumor effects of stem-like DN and differentiated DP subsets, CD39⁻CD69⁻ and CD39⁺CD69⁺ CD8⁺ murine T cell populations were isolated from *in vitro*-expanded Pmel transgenic TCR splenocytes and adoptive transfer of the isolated subsets into mice implanted with B16-F10 melanoma engineered to express the human gp100 antigen was performed (Fig. 4D) (Hanada et al., JCI Insight, 4 (2019), doi: 10.1172/jci.insight.124405). While the adoptive transfer of either 3×10⁵ or 5×10⁵ differentiated DP Pmel T cells had a modest effect on tumor control and mouse survival, the transfer of the same numbers of stem-like DN T cells led to substantial tumor regression and improved survival in a dose-dependent manner (Fig. 4E-4F). The data also suggest that anti-tumor neoantigen-specific TIL, subsets enriched for tumor reactivity (e.g. CD39⁺CD69⁺) might be terminally differentiated TILs with a relatively poor proliferative potential *in vivo* (Fig. 4G) (van der Leun et al., Nat. Rev. Cancer. 20: 218-232 (2020)).

### EXAMPLE 5

This example demonstrates the identification of a gene expression profile which distinguishes neoantigen-specific T cells from bystander T cells.

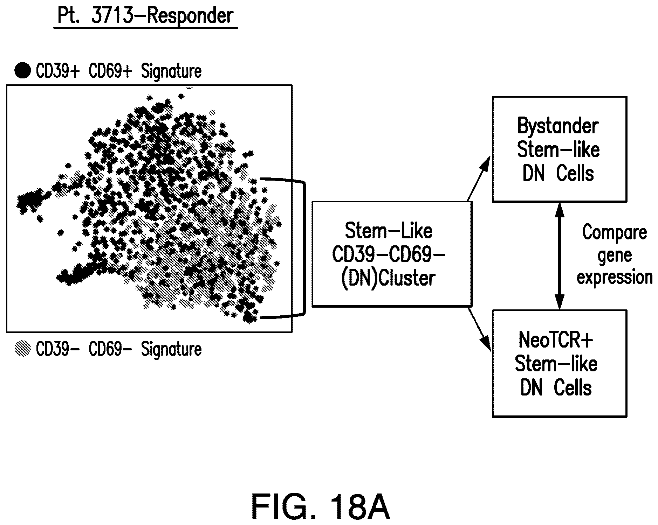

A reanalysis of single cell transcriptome data was done on stem-like cluster CO (Fig. 14A) to identify whether there are any differences in gene expression selectively between neoantigen-specific T cells and bystander T cells within stem-like clusters, as shown in Fig. 18A.

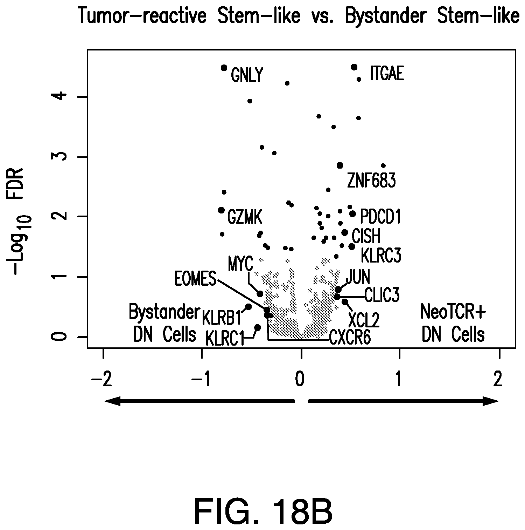

The results are shown in Fig. 18B. As shown in Fig. 18B, differential gene expression was observed, in which selected genes were upregulated in stem-like neoantigen specific T cells (right side of Fig. 18B) relative to bystander T cells (left side of Fig. 18B).

The differentially expressed genes and the fold change values are shown in Table 4. In Table 4, positive logFC values indicate higher expression of the indicated gene in CD39⁻CD69⁻ neoantigen-specific T cells as compared to other T cells in the tumor sample of the cancer patient. Negative logFC values indicate higher expression of the indicated gene in CD39⁻CD69⁻ bystander T cells as compared to other T cells in the tumor sample of the cancer patient.

**TABLE 4**

| **Gene** | **logFC** | **LR** | **PValue** | **FDR** |
|---|---|---|---|---|
| GTSF1 | 0.904722 | 52.68409 | 3.92E-13 | 7.39E-10 |
| HIST1H2AG | 0.830456 | 19.79952 | 8.60E-06 | 0.001443 |
| IGFBP3 | 0.727607 | 28.15751 | 1.12E-07 | 3.67E-05 |
| RBPMS | 0.582377 | 27.32684 | 1.72E-07 | 5.19E-05 |
| RAB34 | 0.577936 | 23.86354 | 1.03E-06 | 0.000228 |
| ITGAE | 0.534229 | 28.70424 | 8.43E-08 | 3.18E-05 |
| PDCD1 | 0.516747 | 15.83315 | 6.92E-05 | 0.008839 |
| KLRC3 | 0.508533 | 12.8874 | 0.000331 | 0.031211 |
| PRSS57 | 0.49132 | 16.47669 | 4.93E-05 | 0.006884 |
| CISH | 0.43821 | 14.23973 | 0.000161 | 0.018131 |
| CDCA7 | 0.410032 | 12.99531 | 0.000312 | 0.030218 |
| AHl1 | 0.397383 | 14.93706 | 0.000111 | 0.012947 |
| ZNF683 | 0.390924 | 19.89687 | 8.17E-06 | 0.001402 |
| VSIR | 0.3893 | 16.0342 | 6.22E-05 | 0.008096 |
| INTS4 | 0.369107 | 10.71772 | 0.001061 | 0.082572 |
| TNFRSF10A | 0.367481 | 10.78281 | 0.001024 | 0.081329 |
| CDC27 | 0.361885 | 10.37136 | 0.00128 | 0.092353 |
| DDX60 | 0.358084 | 10.19647 | 0.001407 | 0.096581 |
| ZBP1 | 0.35556 | 10.58324 | 0.001141 | 0.085283 |
| CTSH | 0.35461 | 12.03861 | 0.000521 | 0.045735 |
| SNAP47 | 0.332793 | 13.68651 | 0.000216 | 0.022362 |
| CD68 | 0.329098 | 10.32952 | 0.001309 | 0.092353 |
| ITGB1 | 0.327208 | 23.12084 | 1.52E-06 | 0.000319 |
| PPM1M | 0.29741 | 10.76497 | 0.001034 | 0.081329 |
| HLA-DPA1 | 0.272018 | 17.92958 | 2.29E-05 | 0.003605 |
| HMGN3 | 0.271023 | 15.58643 | 7.88E-05 | 0.009753 |
| LIME1 | 0.267168 | 11.64913 | 0.000642 | 0.054063 |
| HLA-DRB1 | 0.24803 | 13.68182 | 0.000217 | 0.022362 |
| CTSW | 0.229738 | 11.72008 | 0.000618 | 0.053293 |
| LGALS1 | 0.229152 | 13.34317 | 0.000259 | 0.025759 |
| HLA-DRB5 | 0.219798 | 11.424 | 0.000725 | 0.060136 |
| ANXA5 | 0.207529 | 14.60361 | 0.000133 | 0.015172 |
| ALOX5AP | 0.187616 | 15.80371 | 7.03E-05 | 0.008839 |
| CD74 | 0.187555 | 14.93143 | 0.000111 | 0.012947 |
| S100A11 | 0.178481 | 24.04901 | 9.39E-07 | 0.000215 |
| IL32 | 0.157492 | 16.35793 | 5.24E-05 | 0.007182 |
| COTL1 | 0.156357 | 11.07356 | 0.000876 | 0.071071 |
| S100A4 | 0.154139 | 16.32749 | 5.33E-05 | 0.007182 |
| CD99 | 0.129953 | 13.65856 | 0.000219 | 0.022362 |
| TPT1 | -0.08952 | 10.34621 | 0.001297 | 0.092353 |
| RPL18 | -0.09533 | 10.21895 | 0.00139 | 0.096581 |
| RPL37 | -0.09537 | 10.60361 | 0.001129 | 0.085192 |
| RPL41 | -0.09863 | 16.66852 | 4.45E-05 | 0.006339 |
| MT-CO2 | -0.10308 | 12.62993 | 0.00038 | 0.034522 |
| RPL18A | -0.10578 | 10.93483 | 0.000944 | 0.075779 |
| MT-CO1 | -0.12835 | 16.85439 | 4.04E-05 | 0.005858 |
| RPL13A | -0.13976 | 11.64248 | 0.000645 | 0.054063 |
| EEF1A1 | -0.14122 | 26.91187 | 2.13E-07 | 6.09E-05 |
| RPLPO | -0.16158 | 12.70984 | 0.000364 | 0.033482 |
| MRPL57 | -0.25044 | 10.34765 | 0.001296 | 0.092353 |
| CLDND1 | -0.26097 | 10.17924 | 0.00142 | 0.096581 |
| RGS10 | -0.26899 | 20.90167 | 4.83E-06 | 0.000869 |
| IL27RA | -0.31123 | 10.60669 | 0.001127 | 0.085192 |
| GIMAP4 | -0.34021 | 12.80429 | 0.000346 | 0.032226 |
| SFMBT2 | -0.34713 | 10.19583 | 0.001408 | 0.096581 |
| EPB41 | -0.35904 | 12.96128 | 0.000318 | 0.030383 |
| GIMAP7 | -0.39558 | 21.41986 | 3.69E-06 | 0.000696 |
| ASXL2 | -0.39846 | 11.71096 | 0.000621 | 0.053293 |
| FCMR | -0.41059 | 14.16248 | 0.000168 | 0.018613 |
| ERN1 | -0.42202 | 13.88012 | 0.000195 | 0.020714 |
| LINC01943 | -0.45099 | 10.5145 | 0.001184 | 0.087647 |
| CD6 | -0.51714 | 25.32291 | 4.85E-07 | 0.000118 |
| AQP3 | -0.77688 | 17.73469 | 2.54E-05 | 0.003912 |
| GNLY | -0.7781 | 28.56785 | 9.05E-08 | 3.25E-05 |
| TRGV10 | -0.79636 | 13.9903 | 0.000184 | 0.019814 |
| GZMK | -0.80595 | 16.16195 | 5.82E-05 | 0.0077 |

The use of the terms "a" and "an" and "the" and "at least one" and similar referents in the context of describing the invention (especially in the context of the following claims) are to be construed to cover both the singular and the plural, unless otherwise indicated herein or clearly contradicted by context. The use of the term "at least one" followed by a list of one or more items (for example, "at least one of A and B") is to be construed to mean one item selected from the listed items (A or B) or any combination of two or more of the listed items (A and B), unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention. Variations of those preferred embodiments may become apparent to those of ordinary skill in the art upon reading the foregoing description. The inventors expect skilled artisans to employ such variations as appropriate, and the inventors intend for the invention to be practiced otherwise than as specifically described herein.

## Claims

1. A method of obtaining a cell population enriched for T cells with a phenotype comprising markers CD3⁺, CD8⁺, CD39⁻, and CD69⁻, the method comprising:
(a) obtaining a bulk population of T cells from a tumor sample from a patient;
(b) specifically selecting T cells with a phenotype comprising markers CD3⁺, CD8⁺, CD39⁻, and CD69⁻ from the bulk population;
(c) separating the cells selected in (b) from cells which lack the phenotype; and
(d) isolating tumor-reactive T cells from the separated T cells which have the phenotype to obtain a cell population enriched for T cells with the phenotype.

2. A method of obtaining a cell population enriched for T cells with a phenotype comprising markers CD3⁺, CD8⁺, CD39⁻, and CD69⁻, the method comprising:
(a) obtaining a bulk population of T cells from a tumor sample from a patient;
(b) isolating tumor-reactive T cells from the tumor sample;
(c) specifically selecting T cells with a phenotype comprising markers CD3⁺, CD8⁺, CD39⁻, and CD69⁻ from the isolated tumor-reactive T cells; and
(d) separating the cells selected in (c) from cells which lack the phenotype to obtain a cell population enriched for T cells with the phenotype.

3. The method of claim 1 or 2, wherein selecting the isolated T cells which have the phenotype comprises:
(i) carrying out t-Distributed Stochastic Neighbor Embedding (t-SNE) analysis;
(ii) carrying out Cellular Indexing of Transcriptomes and Epitopes by Sequencing (CITE-Seq) analysis;
(iii) detecting the presence or absence of RNA encoding one or more markers of the phenotype in the T cells; and/or
(iv) detecting the presence or absence of cell surface expression of one or more markers of the phenotype by the T cells.

4. A method of obtaining a pharmaceutical composition comprising a cell population enriched for T cells with a phenotype comprising markers CD3⁺, CD8⁺, CD39⁻, and CD69⁻, the method comprising:
obtaining a cell population according to the method of any one of claims 1-3; and
combining the cell population enriched for T cells with the phenotype with a pharmaceutically acceptable carrier to obtain a pharmaceutical composition comprising a cell population enriched for T cells with the phenotype.

5. The method according to any one of claims 1-4, further comprising:
(i) introducing a nucleic acid encoding an exogenous TCR into the cells in the population enriched for T cells with the phenotype under conditions to express the exogenous TCR by the cells;
(ii) introducing a nucleic acid encoding a chimeric antigen receptor (CAR) into the cells in the population enriched for T cells with the phenotype under conditions to express the CAR by the cells; or
(iii) expanding the number of cells in the population enriched for T cells with the phenotype obtained by the method.

6. An isolated or purified cell population obtained according to the method of any one of claims 1-3 or 5.

7. A pharmaceutical composition comprising the isolated or purified cell population of claim 6 and a pharmaceutically acceptable carrier.

8. An isolated or purified cell population obtained according to the method of any one of claims 1-3 or 5 for use in the treatment or prevention of cancer in a mammal.

9. A method of selecting a therapy for a cancer patient, the method comprising:
(a) obtaining a bulk population of T cells from a tumor sample from a patient;
(b) measuring the proportion of T cells in the bulk population which (i) have or (ii) lack a phenotype, wherein the phenotype comprises markers CD3⁺, CD8⁺, CD39⁻, and CD69⁻; and
(c) comparing the proportion of T cells which (i) have or (ii) lack the phenotype to a control; and
(d) selecting the patient for a therapy which is not immunotherapy when (i) the proportion of T cells which have the phenotype is lower than the control or (ii) the proportion of T cells which lack the phenotype is equal to or higher than the control; or
(e) selecting the patient for an immunotherapy when (i) the proportion of T cells which have the phenotype is equal to or higher than the control or (ii) the proportion of T cells which lack the phenotype is lower than the control.

10. An active agent selected from cells suitable for adoptive cell therapy (ACT), an immune checkpoint inhibitor, an immune system modulator, a T cell and/or a chimeric antigen receptor for use in treating a cancer patient, wherein the cancer patient has been selected by the method of claim 9, step (e).

11. An active agent selected from a stem cell suitable for stem cell therapy, a hormone and/or a targeted drug inhibitor for use in treating a cancer patient, wherein the cancer patient has been selected by the method of claim 9, step (d).

12. A method for predicting the clinical response to immunotherapy in a cancer patient, the method comprising:
(a) obtaining a bulk population of T cells from a tumor sample from the cancer patient;
(b) measuring the proportion of T cells in the bulk population which (i) have or (ii) lack a phenotype, wherein the phenotype comprises markers CD3⁺, CD8⁺, CD39⁻, and CD69⁻; and
(c) comparing the proportion of T cells which (i) have or (ii) lack the phenotype to a control; and
(d) identifying the patient as likely to have a negative clinical response to the immunotherapy when (i) the proportion of T cells which have the phenotype is lower than the control or (ii) the proportion of T cells which lack the phenotype is equal to or higher than the control; or
(e) identifying the patient as likely to have a positive clinical response to the immunotherapy when (i) the proportion of T cells which have the phenotype is equal to or higher than the control or (ii) the proportion of T cells which lack the phenotype is lower than the control,
optionally wherein the immunotherapy is adoptive cell therapy (ACT), immune checkpoint blockade therapy, immune system modulator therapy, T cell therapy, and/or chimeric antigen receptor (CAR) therapy.

13. The method of claim 12, wherein the method is carried out before immunotherapy is administered to the patient.

14. The method of claim 12, wherein the method is carried out after immunotherapy is administered to the patient.

15. The method of claim 12, wherein the method is carried out before immunotherapy is administered to the patient and again after immunotherapy is administered to the patient.

16. The method of any one of claims 1-15, wherein the phenotype further comprises one or more of marker(s):
(i) AHNAK⁺, AL592183.1⁺, ANXA1⁺, ANXA4⁺, AQP3⁺, ATM⁺, BIN1⁺, C10orf54⁺, C11orf21⁺, C16orf54⁺, CCDC109B⁺, CD27⁺, CD52⁺, CD55⁺, CDC25B⁺, CDC42SE1⁺, CLEC2B⁺, CLIC3⁺, CLUAP1⁺, CRBN⁺, CTD-3184A7.4⁺, DD12⁺, DND1⁺, EMP3⁺, EPB41⁺, ERN1⁺, FAIM3⁺, FAM65B⁺, FBXL8⁺, FGFBP2⁺, GADD45B⁺, GIMAP4⁺, GIMAP7⁺, GPR155⁺, GZMM⁺, HSD17B11⁺, IL10RA⁺, IL7R⁺, ISG20⁺, KANSL1-AS1⁺, KLF2⁺, KLHL24⁺, LDLRAP1⁺, LEF1⁺, LGALS3⁺, LINCO0861⁺, LITAF⁺, LYAR⁺, MAPKAPK5-AS1⁺, MED15⁺, MIAT⁺, MIR142⁺, MXI1⁺, MYC⁺, NEAT1⁺, NOSIP⁺, ODF2L⁺, P2RY8⁺, PDE6G⁺, PIK3IP1*, PLEC⁺, PLP2⁺, PPP2R5C⁺, PXN⁺, R3HDM4⁺, RAMP1⁺, RASA3⁺, RASGRP2⁺, RCBTB2⁺, RNASET2⁺, RP11-395B7.4⁺, RP11-539L10.2⁺, RP11-640M9.1⁺, S100A10⁺, S100A4⁺, S100A6⁺, S1PR1⁺, S1PR4⁺, SAMD3⁺, SELL⁺, SH3BP5⁺, SIGIRR⁺, SLAMF6⁺, SLC03A1⁺, SORL1⁺, STK38⁺, SYNJ2⁺, TCF7⁺, TIMP1⁺, TRADD⁺, TSC22D3⁺, TSPAN32⁺, TXNIP⁺, UBXN11⁺, VCL⁺, VNN2⁺, YPEL3⁺, ZFP36L2⁺, ZNF276⁺, and ZNF683⁺;
(ii) ACOT7⁻, ADAM19⁻, AGPAT9⁻, AGTRAP⁻, AIF1⁻, ASPM⁻, ATAD2⁻, AURKA⁻, AURKB⁻, BIRC3⁻, BIRC5⁻, BRCA1⁻, C15orf48⁻, CASC5⁻, CCL3⁻, CCNA2⁻, CCNB1⁻, CCNB2⁻, CCND2⁻, CD38⁻, CD40LG⁻, CDC20⁻, CDCA3⁻, CDCA8⁻, CDK1⁻, CDKN3⁻, CDT1⁻, CENPA⁻, CENPE⁻, CENPF⁻, CENPM⁻, CENPW⁻, CEP55⁻, CISH⁻, CKS1B⁻, CKS2⁻, CLSPN⁻, CRTAM⁻, CSF2⁻, DLGAP5⁻, DUSP5⁻, DUSP6⁻, DUT⁻, EGR1⁻, ENTPD1⁻, FEN1⁻, GINS2⁻, GTSE1⁻, H2AFX⁻, HIST1H4C⁻, HLA⁻DQA2⁻, HMMR⁻, IFI27⁻, IFNG⁻, IL2RA⁻, IL5⁻, KIAA0101⁻, KIF11⁻, KIF23⁻, KPNA2⁻, KRT7⁻, MAD2L1⁻, MCM7⁻, MKI67⁻, MX1⁻, MYBL2⁻, NCAPG⁻, NDC80⁻, NDFIP2⁻, NUDT1⁻, NUSAP1⁻, ORC6⁻, PBK⁻, PCNA⁻, PLK1⁻, RPL39L⁻, RRM2⁻, SGOL2⁻, SHCBP1⁻, SMC2⁻, SPC25⁻, STMN1⁻, TESC⁻, TK1⁻, TNF⁻, TNFRSF18⁻, TNFSF10⁻, TOP2A⁻, TPM4⁻, TPX2⁻, TUBA1B⁻, TUBA1C⁻, TUBB⁻, TYMS⁻, UBE2C⁻, UBE2S⁻, UBE2T⁻, XCL1⁻, and ZWINT⁻;
(iii) AH11⁺, ALOX5AP⁺, ANXA5⁺, CD68⁺, CD74⁺, CD99⁺, CDC27⁺, CDCA7⁺, CISH⁺, COTL1⁺, CTSH⁺, CTSW⁺, DDX60⁺, GTSF1⁺, HIST1H2AG⁺, HLA-DPA1⁺, HLA-DRB1⁺, HLA-DRB5⁺, HMGN3⁺, IGFBP3⁺, IL32⁺, INTS4⁺, ITGAE⁺, ITGB1⁺, KLRC3⁺, LGALS1⁺, LIME1⁺, PDCD1⁺, PPM1M⁺, PRSS57⁺, RAB34⁺, RBPMS⁺, S100A11⁺, S100A4⁺, SNAP47⁺, TNFRSF10A⁺, VSIR⁺, ZBP1⁺, and ZNF683⁺; and/or
(iv) AQP3⁻, ASXL2⁻, CD6⁻, CLDND1⁻, EEF1A1⁻, EPB41⁻, ERN1⁻, FCMR⁻, GIMAP4⁻, GIMAP7⁻, GNLY⁻, GZMK⁻, IL27RA⁻, LINC01943⁻, MRPL57⁻, MT⁻CO1⁻, MT⁻CO2⁻, RGS10⁻, RPL13A⁻, RPL18⁻, RPL18A⁻, RPL37⁻, RPL41⁻, RPLPO⁻, SFMBT2⁻, TPT1⁻, and TRGV10⁻

## Patentansprüche

1. Verfahren zur Gewinnung einer Zellpopulation, die an T-Zellen mit einem Phänotyp angereichert ist, welcher die Marker CD3⁺, CD8⁺, CD39⁻ und CD69⁻ umfasst, wobei das Verfahren umfasst:
(a) Gewinnen einer Gesamtpopulation von T-Zellen aus einer Tumorprobe eines Patienten;
(b) spezifische Selektion von T-Zellen mit einem Phänotyp, der die Marker CD3⁺, CD8⁺, CD39⁻ und CD69⁻ umfasst, aus der Gesamtpopulation;
(c) Abtrennen der in (b) ausgewählten Zellen von Zellen, denen der Phänotyp fehlt; und
(d) Isolieren tumorreaktiver T-Zellen aus den abgetrennten T-Zellen, die den Phänotyp aufweisen, um eine Zellpopulation zu erhalten, die an T-Zellen mit dem Phänotyp angereichert ist.

2. Verfahren zur Gewinnung einer Zellpopulation, die an T-Zellen mit einem Phänotyp angereichert ist, welcher die Marker CD3⁺, CD8⁺, CD39⁻ und CD69⁻ umfasst, wobei das Verfahren umfasst:
(a) Gewinnen einer Gesamtpopulation von T-Zellen aus einer Tumorprobe eines Patienten;
(b) Isolieren tumorreaktiver T-Zellen aus der Tumorprobe;
(c) spezifische Selektion von T-Zellen mit einem Phänotyp, der die Marker CD3⁺, CD8⁺, CD39⁻ und CD69⁻ umfasst, aus den isolierten tumorreaktiven T-Zellen; und
(d) Abtrennen der in (c) ausgewählten Zellen von Zellen, denen der Phänotyp fehlt, um eine Zellpopulation zu erhalten, die an T-Zellen mit dem Phänotyp angereichert ist.

3. Verfahren nach Anspruch 1 oder 2, wobei die Auswahl der isolierten T-Zellen, die den Phänotyp aufweisen, umfasst:
(i) Durchführen einer t-Distributed Stochastic Neighbor Embedding (t-SNE) Analyse;
(ii) Durchführen einer zellulären Indexierung von Transkriptomen und Epitopen mittels Sequenzierung (CITE-Seq) Analyse;
(iii) Nachweis des Vorhandenseins oder der Abwesenheit von RNA, die für einen oder mehrere Marker des Phänotyps in den T-Zellen kodiert; und/oder
(iv) Nachweis der Anwesenheit oder Abwesenheit der Zelloberflächenexpression eines oder mehrerer Marker des Phänotyps durch die T-Zellen.

4. Verfahren zur Gewinnung einer pharmazeutischen Zusammensetzung, die eine Zellpopulation umfasst, die an T-Zellen mit einem Phänotyp angereichert ist, welcher die Marker CD3⁺, CD8⁺, CD39⁻ und CD69⁻ umfasst, wobei das Verfahren umfasst:
Gewinnen einer Zellpopulation gemäß dem Verfahren nach einem der Ansprüche 1-3; und
Kombinieren der Zellpopulation, die an T-Zellen mit dem Phänotyp angereichert ist mit einem pharmazeutisch verträglichen Träger, um eine pharmazeutische Zusammensetzung zu erhalten, die eine Zellpopulation, die an T-Zellen mit dem Phänotyp angereichert ist, umfasst.

5. Verfahren nach einem der Ansprüche 1-4, ferner umfassend:
(i) Einbringen einer Nukleinsäure, die einen exogenen TCR kodiert, in die Zellen in der Population, die an T-Zellen mit dem Phänotyp angereichert ist, unter Bedingungen zur Expression des exogenen TCRs durch die Zellen; oder
(ii) Einbringen einer Nukleinsäure, die für einen chimären Antigenrezeptor (CAR) kodiert, in die Zellen in der Population, die an T-Zellen mit dem Phänotyp angereichert ist, unter Bedingungen zur Expression des CARs durch die Zellen; oder
(iii) Vergrößern der Anzahl der Zellen in der Population, die an T-Zellen mit dem Phänotyp durch das Verfahren angereichert wurde.

6. Isolierte oder aufgereinigte Zellpopulation, die gemäß dem Verfahren nach einem der Ansprüche 1-3 oder 5 erhalten wurde.

7. Pharmazeutische Zusammensetzung, die die isolierte oder aufgereinigte Zellpopulation nach Anspruch 6 und einen pharmazeutisch akzeptablen Träger umfasst.

8. Isolierte oder aufgereinigte Zellpopulation, die gemäß dem Verfahren nach einem der Ansprüche 1 bis 3 oder 5 erhalten wurde, zur Verwendung bei der Behandlung oder Vorbeugung von Krebs in einem Säugetier.

9. Verfahren zur Auswahl einer Therapie für einen Krebspatienten, wobei das Verfahren umfasst:
(a) Gewinnen einer Gesamtpopulation von T-Zellen aus einer Tumorprobe eines Patienten;
(b) Messen des Anteils der T-Zellen in der Massenpopulation, die (i) einen Phänotyp haben oder (ii) nicht haben, wobei der Phänotyp die Marker CD3⁺, CD8⁺, CD39⁻ und CD69⁻ umfasst; und
(c) Vergleichen des Anteils der T-Zellen, die (i) den Phänotyp aufweisen oder (ii) nicht aufweisen, mit einer Kontrolle; und
(d) Auswahl des Patienten für eine Therapie, bei der es sich nicht um eine Immuntherapie handelt, wenn (i) der Anteil der T-Zellen, die den Phänotyp aufweisen, niedriger ist als bei der Kontrolle oder (ii) der Anteil der T-Zellen, denen der Phänotyp fehlt, gleich oder höher ist als bei der Kontrolle; oder
(e) Auswahl des Patienten für eine Immuntherapie, wenn (i) der Anteil der T-Zellen, die den Phänotyp aufweisen, gleich oder höher ist als bei der Kontrolle oder (ii) der Anteil der T-Zellen, denen der Phänotyp fehlt, niedriger als bei der Kontrolle ist.

10. Wirkstoff, ausgewählt aus Zellen, die für die adoptive Zelltherapie (ACT) geeignet sind, einem Immuncheckpoint-Inhibitor, einem Modulator des Immunsystems, einer T-Zelle und/oder einem chimären Antigenrezeptor, zur Verwendung bei der Behandlung eines Krebspatienten, wobei der Krebspatient gemäß dem Verfahren nach Anspruch 9, Schritt (e), ausgewählt wurde.

11. Wirkstoff, ausgewählt aus einer für die Stammzelltherapie geeigneten Stammzelle, einem Hormon und/oder einem gezielten Arzneimittelinhibitor, zur Verwendung bei der Behandlung eines Krebspatienten, wobei der Krebspatient gemäß dem Verfahren nach Anspruch 9, Schritt (d), ausgewählt wurde.

12. Verfahren zur Vorhersage des klinischen Ansprechens auf eine Immuntherapie bei einem Krebspatienten, wobei das Verfahren umfasst:
(a) Gewinnen einer Gesamtpopulation von T-Zellen aus einer Tumorprobe des Krebspatienten;
(b) Messen des Anteils der T-Zellen in der Gesamtpopulation, die (i) einen Phänotyp haben oder (ii) nicht haben, wobei der Phänotyp die Marker CD3⁺, CD8⁺, CD39⁻ und CD69⁻ umfasst; und
(c) Vergleichen des Anteils der T-Zellen, die (i) den Phänotyp aufweisen oder (ii) nicht aufweisen, mit einer Kontrolle; und
(d) Identifizierung des Patienten als wahrscheinlich für ein negatives klinisches Ansprechen auf die Immuntherapie, wenn (i) der Anteil der T-Zellen, die den Phänotyp aufweisen, niedriger ist als bei der Kontrolle oder (ii) der Anteil der T-Zellen, denen der Phänotyp fehlt, gleich oder höher ist als bei der Kontrolle; oder
(e) Identifizierung des Patienten als wahrscheinlich für ein positives klinisches Ansprechen auf die Immuntherapie, wenn (i) der Anteil der T-Zellen, die den Phänotyp aufweisen, gleich oder höher als bei der Kontrolle ist oder (ii) der Anteil der T-Zellen, denen der Phänotyp fehlt, niedriger als bei der Kontrolle ist,
wobei es sich bei der Immuntherapie gegebenenfalls um eine adoptive Zelltherapie (ACT), eine Immuncheckpoint-Blockade-Therapie, eine Immunsystem-Modulator-Therapie, eine T-Zell-Therapie und/oder eine chimäre Antigenrezeptor-Therapie (CAR) handelt.

13. Verfahren nach Anspruch 12, wobei das Verfahren durchgeführt wird, bevor dem Patienten eine Immuntherapie verabreicht wird.

14. Verfahren nach Anspruch 12, wobei das Verfahren nach der Verabreichung der Immuntherapie an den Patienten durchgeführt wird.

15. Verfahren nach Anspruch 12, wobei das Verfahren vor der Verabreichung der Immuntherapie an den Patienten und erneut nach der Verabreichung der Immuntherapie an den Patienten durchgeführt wird.

16. Verfahren nach einem der Ansprüche 1-15, wobei der Phänotyp ferner einen oder mehrere Marker umfasst:
(i) AHNAK⁺, AL592183.1⁺, ANXA1⁺, ANXA4⁺, AQP3⁺, ATM⁺, BIN1⁺, C10orf54⁺, C11orf21⁺, C16orf54⁺, CCDC109B⁺, CD27⁺, CD52⁺, CD55⁺, CDC25B⁺, CDC42SE1⁺, CLEC2B⁺, CLIC3⁺, CLUAP1⁺, CRBN⁺, CTD-3184A7.4⁺, DD12⁺, DND1⁺, EMP3⁺, EPB41⁺, ERN1⁺, FAIM3⁺, FAM65B⁺, FBXL8⁺, FGFBP2⁺, GADD45B⁺, GIMAP4⁺, GIMAP7⁺, GPR155⁺, GZMM⁺, HSD17B11⁺, IL10RA⁺, IL7R⁺, ISG20⁺, KANSL1-AS1⁺, KLF2⁺, KLHL24⁺, LDLRAP1⁺, LEF1⁺, LGALS3⁺, LINC00861⁺, LITAF⁺, LYAR⁺, MAPKAPK5-AS1⁺, MED15⁺, MIAT⁺, MIR142⁺, MXI1⁺, MYC⁺, NEAT1⁺, NOSIP⁺, ODF2L⁺, P2RY8⁺, PDE6G⁺, PIK3IP1⁺, PLEC⁺, PLP2⁺, PPP2R5C⁺, PXN⁺, R3HDM4⁺, RAMP1⁺, RASA3⁺, RASGRP2⁺, RCBTB2⁺, RNASET2⁺, RP11-395B7.4⁺, RP11-539L10.2⁺, RP11-640M9.1⁺, S100A10⁺, S100A4⁺, S100A6⁺, S1PR1⁺, S1PR4⁺, SAMD3⁺, SELL⁺, SH3BP5⁺, SIGIRR⁺, SLAMF6⁺, SLCO3A1⁺, SORL1⁺, STK38⁺, SYNJ2⁺, TCF7⁺, TIMP1⁺, TRADD⁺, TSC22D3⁺, TSPAN32⁺, TXNIP⁺, UBXN11⁺, VCL⁺, VNN2⁺, YPEL3⁺, ZFP36L2⁺, ZNF276⁺, und ZNF683⁺;
(ii) ACOT7⁻, ADAM19⁻, AGPAT9⁻, AGTRAP⁻, AIF1⁻, ASPM⁻, ATAD2⁻, AURKA⁻, AURKB⁻, BIRC3⁻, BIRC5⁻, BRCA1⁻, C15orf48⁻, CASC5⁻, CCL3⁻, CCNA2⁻, CCNB1⁻, CCNB2⁻, CCND2⁻, CD38⁻, CD40LG⁻, CDC20⁻, CDCA3⁻, CDCA8⁻, CDK1⁻, CDKN3⁻, CDT1⁻, CENPA⁻, CENPE⁻, CENPF⁻, CENPM⁻, CENPW⁻, CEP55⁻, CISH⁻, CKS1B⁻, CKS2⁻, CLSPN⁻, CRTAM⁻, CSF2⁻, DLGAP5⁻, DUSP5⁻, DUSP6⁻, DUT⁻, EGR1⁻, ENTPD1⁻, FEN1⁻, GINS2⁻, GTSE1⁻, H2AFX⁻, HIST1H4C⁻, HLA⁻DQA2⁻, HMMR⁻, IFI27⁻, IFNG⁻, IL2RA⁻, IL5⁻, KIAA0101⁻, KIF11⁻, KIF23⁻, KPNA2⁻, KRT7⁻, MAD2L1⁻, MCM7⁻, MKI67⁻, MX1⁻, MYBL2⁻, NCAPG⁻, NDC80⁻, NDFIP2⁻, NUDT1⁻, NUSAP1⁻, ORC6⁻, PBK⁻, PCNA⁻, PLK1⁻, RPL39L⁻, RRM2⁻, SGOL2⁻, SHCBP1⁻, SMC2⁻, SPC25⁻, STMN1⁻, TESC⁻, TK1⁻, TNF⁻, TNFRSF18⁻, TNFSF10⁻, TOP2A⁻, TPM4⁻, TPX2⁻, TUBA1B⁻, TUBA1C⁻, TUBB⁻, TYMS⁻, UBE2C⁻, UBE2S⁻, UBE2T⁻, XCL1⁻, und ZWINT⁻;
(iii) AHI1⁺, ALOX5AP⁺, ANXA5⁺, CD68⁺, CD74⁺, CD99⁺, CDC27⁺, CDCA7⁺, CISH⁺, COTL1⁺, CTSH⁺, CTSW⁺, DDX60⁺, GTSF1⁺, HIST1H2AG⁺, HLA-DPA1⁺, HLA-DRB1⁺, HLA-DRB5⁺, HMGN3⁺, IGFBP3⁺, IL32⁺, INTS4⁺, ITGAE⁺, ITGB1⁺, KLRC3⁺, LGALS1⁺, LIME1⁺, PDCD1⁺, PPM1M⁺, PRSS57⁺, RAB34⁺, RBPMS⁺, S100A11⁺, S100A4⁺, SNAP47⁺, TNFRSF10A⁺, VSIR⁺, ZBP1⁺, und ZNF683⁺; und/oder
(iv) AQP3⁻, ASXL2⁻, CD6⁻, CLDND1⁻, EEF1A1⁻, EPB41⁻, ERN1⁻, FCMR⁻, GIMAP4⁻, GIMAP7⁻, GNLY⁻, GZMK⁻, IL27RA⁻, LINC01943⁻, MRPL57⁻, MT⁻CO1⁻, MT⁻ CO2⁻, RGS10⁻, RPL13A⁻, RPL18⁻, RPL18A⁻, RPL37⁻, RPL41⁻, RPLP0⁻, SFMBT2⁻, TPT1⁻, und TRGV10⁻.

## Revendications

1. Procédé d'obtention d'une population cellulaire enrichie en lymphocytes T présentant un phénotype comprenant des marqueurs CD3⁺, CD8⁺, CD39⁻, et CD69⁻, le procédé comprenant :
(a) l'obtention d'une population en vrac de lymphocytes T à partir d'un échantillon tumoral prélevé chez un patient ;
(b) la sélection spécifique de lymphocytes T présentant un phénotype comprenant des marqueurs CD3⁺, CD8⁺, CD39⁻, et CD69⁻ à partir de la population en vrac ;
(c) la séparation des cellules sélectionnées en (b) de cellules dépourvues du phénotype ; et
(d) l'isolement de lymphocytes T réactifs à la tumeur par rapport aux lymphocytes T séparés présentant le phénotype pour obtenir une population cellulaire enrichie en lymphocytes T présentant le phénotype.

2. Procédé d'obtention d'une population cellulaire enrichie en lymphocytes T présentant un phénotype comprenant des marqueurs CD3⁺, CD8⁺, CD39⁻, et CD69⁻, le procédé comprenant :
(a) l'obtention d'une population en vrac de lymphocytes T à partir d'un échantillon tumoral prélevé chez un patient ;
(b) l'isolement de lymphocytes T réactifs à la tumeur par rapport à l'échantillon tumoral ;
(c) la sélection spécifique de lymphocytes T présentant un phénotype comprenant des marqueurs CD3⁺, CD8⁺, CD39⁻, et CD69⁻ à partir des lymphocytes T réactifs à la tumeur isolés ; et
(d) la séparation des cellules sélectionnées en (c) de cellules dépourvues du phénotype pour obtenir une population cellulaire enrichie en lymphocytes T présentant le phénotype.

3. Le procédé de la revendication 1 ou 2, sachant que la sélection des lymphocytes T isolés présentant le phénotype comprend :
(i) l'exécution d'une analyse par incrustation stochastique de voisinage t-distribuée (t-SNE, t-Distributed Stochastic Neighbor Embedding) ;
(ii) l'exécution d'une analyse par indexage cellulaire des transcriptomes et des épitopes par séquençage (CITE-seq, Cellular Indexing of Transcriptomes and Epitopes by Sequencing) ;
(iii) la détection de la présence ou de l'absence d'ADN codant pour un ou plusieurs marqueurs du phénotype dans les lymphocytes T ; et/ou
(iv) la détection de la présence ou de l'absence d'expression en surface cellulaire d'un ou de plusieurs marqueurs du phénotype par les lymphocytes T.

4. Procédé d'obtention d'une composition pharmaceutique comprenant une population cellulaire enrichie en lymphocytes T présentant un phénotype comprenant des marqueurs CD3⁺, CD8⁺, CD39⁻, et CD69⁻, le procédé comprenant :
l'obtention d'une population cellulaire selon le procédé de l'une quelconque des revendications 1 à 3 ; et
l'obtention de la population cellulaire enrichie en lymphocytes T présentant le phénotype avec un support pharmaceutiquement acceptable pour obtenir une composition pharmaceutique comprenant une population cellulaire enrichie en lymphocytes T présentant le phénotype.

5. Le procédé selon l'une quelconque des revendications 1 à 4, comprenant en outre :
(i) l'introduction d'un acide nucléique codant pour un TCR exogène dans les cellules dans la population enrichie en lymphocytes T présentant le phénotype dans des conditions pour exprimer le TCR exogène par les cellules ;
(ii) l'introduction d'un acide nucléique codant pour un récepteur antigénique chimérique (CAR) dans les cellules dans la population enrichie en lymphocytes T présentant le phénotype dans des conditions pour exprimer le CAR par les cellules ;
(iii) l'expansion du nombre de cellules dans la population enrichie en lymphocytes T présentant le phénotype obtenue par le procédé.

6. Population cellulaire isolée ou purifiée obtenue selon le procédé de l'une quelconque des revendications 1 à 3 ou 5.

7. Composition pharmaceutique comprenant la population cellulaire isolée ou purifiée de la revendication 6 et un support pharmaceutiquement acceptable.

8. Population cellulaire isolée ou purifiée obtenue selon le procédé de l'une quelconque des revendications 1 à 3 ou 5 pour utilisation dans le traitement ou la prévention d'un cancer chez un mammifère.

9. Procédé de sélection d'une thérapie pour un patient atteint d'un cancer, le procédé comprenant :
(a) l'obtention d'une population en vrac de lymphocytes T à partir d'un échantillon tumoral prélevé chez un patient ;
(b) la mesure de la proportion de lymphocytes T dans la population en vrac qui (i) présentent ou (ii) sont dépourvus d'un phénotype, sachant que le phénotype comprend des marqueurs CD3⁺, CD8⁺, CD39⁻, et CD69⁻ ;
(c) la comparaison de la proportion de lymphocytes T qui (i) présentent ou (ii) sont dépourvus du phénotype avec un témoin ; et
(d) la sélection du patient pour une thérapie qui n'est pas une immunothérapie lorsque (i) la proportion de lymphocytes T qui présentent le phénotype est inférieure au témoin ou (ii) la proportion de lymphocytes T qui sont dépourvus du phénotype est égale ou supérieure au témoin ; ou
(e) la sélection du patient pour une immunothérapie lorsque (i) la proportion de lymphocytes T qui présentent le phénotype est égale ou supérieure au témoin ou (ii) la proportion de lymphocytes T qui sont dépourvus du phénotype est inférieure au témoin.

10. Agent actif sélectionné parmi des cellules adaptées à une thérapie cellulaire adoptive (ACT), un inhibiteur de points de contrôle immunitaire, un modulateur du système immunitaire, un lymphocyte T et/ou un récepteur antigénique chimérique pour utilisation dans le traitement d'un patient atteint d'un cancer, sachant que le patient atteint d'un cancer a été sélectionné par le procédé de la revendication 9, étape (e).

11. Agent actif sélectionné parmi une cellule souche adaptée à une thérapie cellulaire par cellules souches, une hormone et/ou un inhibiteur de médicament ciblé pour utilisation dans le traitement d'un patient atteint d'un cancer, sachant que le patient atteint d'un cancer a été sélectionné par le procédé de la revendication 9, étape (d).

12. Procédé de prédiction de la réponse clinique à une immunothérapie chez un patient atteint d'un cancer, le procédé comprenant :
(a) l'obtention d'une population en vrac de lymphocytes T à partir d'un échantillon tumoral prélevé chez le patient atteint d'un cancer ;
(b) la mesure de la proportion de lymphocytes T dans la population en vrac qui (i) présentent ou (ii) sont dépourvus d'un phénotype, sachant que le phénotype comprend des marqueurs CD3⁺, CD8⁺, CD39⁻, et CD69⁻ ; et
(c) la comparaison de la proportion de lymphocytes T qui (i) présentent ou (ii) sont dépourvus du phénotype avec un témoin ; et
(d) l'identification du patient comme susceptible d'avoir une réponse clinique négative à l'immunothérapie lorsque (i) la proportion de lymphocytes T qui présentent le phénotype est inférieure au témoin ou (ii) la proportion de lymphocytes T qui sont dépourvus du phénotype est égale ou supérieure au témoin ; ou
(e) l'identification du patient comme susceptible d'avoir une réponse clinique positive à l'immunothérapie lorsque (i) la proportion de lymphocytes T qui présentent le phénotype est égale ou supérieure au témoin ou (ii) la proportion de lymphocytes T qui sont dépourvus du phénotype est inférieure au témoin,
facultativement sachant que l'immunothérapie est une thérapie cellulaire adoptive (ACT), une thérapie par blocage des points de contrôle immunitaire, une thérapie par modulateur du système immunitaire, une thérapie par lymphocytes T, et/ou une thérapie par récepteur antigénique chimérique (CAR).

13. Le procédé de la revendication 12, sachant que le procédé est effectué avant qu'une immunothérapie soit administrée au patient.

14. Le procédé de la revendication 12, sachant que le procédé est effectué après qu'une immunothérapie a été administrée au patient.

15. Le procédé de la revendication 12, sachant que le procédé est effectué avant qu'une immunothérapie soit administrée au patient et à nouveau après qu'une immunothérapie a été administrée au patient.

16. Le procédé de l'une quelconque des revendications 1 à 15, sachant que le phénotype comprend en outre l'un ou plusieurs des marqueurs :
(i) AHNAK⁺, AL592183.1⁺, ANXA1⁺, ANXA4⁺, AQP3⁺, ATM⁺, BIN1⁺, C10orf54⁺, C11orf21⁺, C16orf54⁺, CCDC109B⁺, CD27⁺, CD52⁺, CD55⁺, CDC25B⁺, CDC42SE1⁺, CLEC2B⁺, CLIC3⁺, CLUAP1⁺, CRBN⁺, CTD-3184A7.4⁺, DDI2⁺, DND1⁺, EMP3⁺, EPB41⁺, ERN1⁺, FAIM3⁺, FAM65B⁺, FBXL8⁺, FGFBP2⁺, GADD45B⁺, GIMAP4⁺, GIMAP7⁺, GPR155⁺, GZMM⁺, HSD17B11⁺, IL10RA⁺, IL7R⁺, ISG20⁺, KANSL1-AS1⁺, KLF2⁺, KLHL24⁺, LDLRAP1⁺, LEF1⁺, LGALS3⁺, LINC00861⁺, LITAF⁺, LYAR⁺, MAPKAPK5- AS1⁺, MED15⁺, MIAT⁺, MIR142⁺, MXI1⁺, MYC⁺, NEAT1⁺, NOSIP⁺, ODF2L⁺, P2RY8⁺, PDE6G⁺, PIK3IP1⁺, PLEC⁺, PLP2⁺, PPP2R5C⁺, PXN⁺, R3HDM4⁺, RAMP1⁺, RASA3⁺, RASGRP2⁺, RCBTB2⁺, RNASET2⁺, RP11-395B7.4⁺, RP11-539L10.2⁺, RP11-640M9.1⁺, S100A10⁺, S100A4⁺, S100A6⁺, S1PR1⁺, S1PR4⁺, SAMD3⁺, SELL⁺, SH3BP5⁺, SIGIRR⁺, SLAMF6⁺, SLC03A1⁺, SORL1⁺, STK38⁺, SYNJ2⁺, TCF7⁺, TIMP1⁺, TRADD⁺, TSC22D3⁺, TSPAN32⁺, TXNIP⁺, UBXN11⁺, VCL⁺, VNN2⁺, YPEL3⁺, ZFP36L2⁺, ZNF276⁺, et ZNF683⁺ ;
(ii) ACOT7⁻, ADAM19⁻, AGPAT9⁻, AGTRAP⁻, AIF1⁻, ASPM⁻, ATAD2⁻, AURKA⁻, AURKB⁻, BIRC3⁻, BIRC5⁻, BRCA1⁻, C15orf48⁻, CASC5⁻, CCL3⁻, CCNA2⁻, CCNB1⁻, CCNB2⁻, CCND2⁻, CD38⁻, CD40LG⁻, CDC20⁻, CDCA3⁻, CDCA8⁻, CDK1⁻, CDKN3⁻, CDT1⁻, CENPA⁻, CENPE⁻, CENPF⁻, CENPM⁻, CENPW⁻, CEP55⁻, CISH⁻, CKS1B⁻, CKS2⁻, CLSPN⁻, CRTAM⁻, CSF2⁻, DLGAP5⁻, DUSP5⁻, DUSP6⁻, DUT⁻, EGR1⁻, ENTPD1⁻, FEN1⁻, GINS2⁻, GTSE1⁻, H2AFX⁻, HIST1H4C⁻, HLA-DQA2⁻, HMMR⁻, IFI27⁻, IFNG⁻, IL2RA⁻, IL5⁻, KIAA0101⁻, KIF11⁻, KIF23⁻, KPNA2⁻, KRT7⁻, MAD2L1⁻, MCM7⁻, MKI67⁻, MX1⁻, MYBL2⁻, NCAPG⁻, NDC80⁻, NDFIP2⁻, NUDT1⁻, NUSAP1⁻, ORC6⁻, PBK⁻, PCNA⁻, PLK1⁻, RPL39L⁻, RRM2⁻, SGOL2⁻, SHCBP1⁻, SMC2⁻, SPC25⁻, STMN1⁻, TESC⁻, TK1⁻, TNF⁻, TNFRSF18⁻, TNFSF10⁻, TOP2A⁻, TPM4⁻, TPX2⁻, TUBA1B⁻, TUBA1C⁻, TUBB⁻, TYMS⁻, UBE2C⁻, UBE2S⁻, UBE2T⁻, XCL1⁻, et ZWINT⁻ ;
(iii) AHI1⁺, ALOX5AP⁺, ANXA5⁺, CD68⁺, CD74⁺, CD99⁺, CDC27⁺, CDCA7⁺, CISH⁺, COTL1⁺, CTSH⁺, CTSW⁺, DDX60⁺, GTSF1⁺, HIST1H2AG⁺, HLA-DPA1⁺, HLA-DRB1⁺, HLA-DRB5⁺, HMGN3⁺, IGFBP3⁺, IL32⁺, INTS4⁺, ITGAE⁺, ITGB1⁺, KLRC3⁺, LGALS1⁺, LIME1⁺, PDCD1⁺, PPM1M⁺, PRSS57⁺, RAB34⁺, RBPMS⁺, S100A11⁺, S100A4⁺, SNAP47⁺, TNFRSF10A⁺, VSIR⁺, ZBP1⁺, et ZNF683⁺ ; et/ou
(iv) AQP3⁻, ASXL2⁻, CD6⁻, CLDND1⁻, EEF1A1⁻, EPB41⁻, ERN1⁻, FCMR⁻, GIMAP4⁻, GIMAP7⁻, GNLY⁻, GZMK⁻, IL27RA⁻, LINC01943⁻, MRPL57⁻, MT⁻CO1⁻, MT-CO2⁻, RGS10⁻, RPL13A⁻, RPL18⁻, RPL18A⁻, RPL37⁻, RPL41⁻, RPLP0⁻, SFMBT2⁻, TPT1⁻, et TRGV10⁻.
